(19) Europäisches Patentamt
European Patent Office
Office européen des brevets

(11) EP 1 815 867 A1

(12) EUROPEAN PATENT APPLICATION
published in accordance with Art. 158(3) EPC

(43) Date of publication:
**08.08.2007 Bulletin 2007/32**

(21) Application number: **05805515.3**

(22) Date of filing: **02.11.2005**

(51) Int Cl.:
*A61K 45/06* (2006.01) *A61K 31/18* (2006.01)
*A61K 31/42* (2006.01) *A61K 31/426* (2006.01)
*A61K 31/519* (2006.01) *A61K 31/5377* (2006.01)
*A61K 31/635* (2006.01) *A61P 1/04* (2006.01)
*A61P 1/16* (2006.01) *A61P 3/00* (2006.01)
*A61P 3/08* (2006.01) *A61P 3/10* (2006.01)
*A61P 5/14* (2006.01) *A61P 7/00* (2006.01)
*A61P 7/04* (2006.01) *A61P 7/06* (2006.01)
*A61P 11/00* (2006.01) *A61P 13/02* (2006.01)
*A61P 15/08* (2006.01) *A61P 15/12* (2006.01)
*A61P 17/00* (2006.01)

(86) International application number:
**PCT/JP2005/020221**

(87) International publication number:
**WO 2006/049215 (11.05.2006 Gazette 2006/19)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **02.11.2004 JP 2004318771**

(71) Applicant: **Dainippon Sumitomo Pharma Co., Ltd.**
**Osaka 541-8524 (JP)**

(72) Inventors:
- **TAGASHIRA, Shuzo**
**Dainippon Sumitomo Pharma Co, Ltd**
**Osaka-shi, Osaka 5540022 (JP)**
- **FUKUSHIMA, A.**
**Dainippon Sumitomo Pharma Co., Ltd.**
**Osaka-shi, Osaka 5540022 (JP)**

(74) Representative: **Duckworth, Timothy John**
**J.A. Kemp & Co.,**
**14 South Square,**
**Gray's Inn**
**London WC1R 5JJ (GB)**

# (54) COMBINATION DRUG FOR TREATING AUTOIMMUNE DISEASE

(57) It is intended to provide a combination drug for preventing and/or treating an autoimmune disease. More specifically speaking, a combination drug for preventing and/or treating an autoimmune disease which contains a CaMKII inhibitor and a disease-modifying antirheumatic drug; a medicinal composition, a commercial package, a kit, etc. for the combination; an enhancer for the effect of preventing and/or treating an autoimmune disease of a disease-modifying antirheumatic drug which contains a CaMKII inhibitor as the active ingredient; a method of screening a CaMKII inhibitor appropriately usable in combination, and so on.



Printed by Jouve, 75001 PARIS (FR)

SEQUENCE LISTING

<110> Dainippon Sumitomo Pharma Co., Ltd.

<120> Agents for combination therapy in the treatment of autoimmune diseases

<130> 533923

<150> JP 2004-318771
<151> 2004-11-02

<160> 12

<170> PatentIn version 3.2

<210> 1
<211> 13
<212> PRT
<213> Artificial

<220>
<223> inhibitor peptide

<400> 1

Lys Lys Ala Leu Arg Arg Gln Glu Ala Phe Asp Ala Tyr
1 5 10

<210> 2
<211> 13
<212> PRT
<213> Artificial

<220>
<223> inhibitor peptide

<400> 2

Lys Lys Lys Leu Arg Arg Gln Glu Ala Phe Asp Ala Tyr
1 5 10

<210> 3
<211> 13
<212> PRT
<213> Artificial

<220>
<223> inhibitor peptide

<400> 3

Lys Lys Ala Leu His Arg Gln Glu Ala Val Asp Cys Leu
1 5 10

<210> 4
<211> 31
<212> DNA
<213> Artificial

<220>
<223> primer

<400> 4
tcggatccag catggccacc accgccacct g 31

<210> 5
<211> 29
<212> DNA

<213> Artificial

<220>
<223> primer

<400> 5
tagaattcac actgcagcgg tgcggcagg 29

<210> 6
<211> 21
<212> DNA
<213> Artificial

<220>
<223> siRNA/sense 1-19 are RNA, 20-21 are DNA

<400> 6
cgugaggcuc ggauauguct t 21

<210> 7
<211> 21
<212> DNA
<213> Artificial

<220>
<223> siRNA/antisense 1-19 are RNA, 20-21 are DNA

<400> 7
gacauauccg agccucacgt t 21

<210> 8
<211> 21
<212> DNA

<213> Artificial

<220>
<223> siRNA/sense 1-19 are RNA, 20-21 are DNA

<400> 8
cauccaaaca ucgugcgcct t 21

<210> 9
<211> 21
<212> DNA
<213> Artificial

<220>
<223> siRNA/antisense 1-19 are RNA, 20-21 are DNA

<400> 9
ggcgcacgau guuuggaugt t 21

<210> 10
<211> 21
<212> DNA
<213> Artificial

<220>
<223> siRNA/sense 1-19 are RNA, 20-21 are DNA

<400> 10
gcagaugcca gccacuguat t 21

<210> 11
<211> 21
<212> DNA

<213> Artificial

<220>
<223> siRNA/antisense 1-19 are RNA, 20-21 are DNA

<400> 11
uacaguggcu ggcaucugct t 21

<210> 12
<211> 13
<212> PRT
<213> Artificial

<220>
<223> PLP(139-151)

<400> 12

His Ser Leu Gly Lys Trp Leu Gly His Pro Asp Lys Phe
1 5 10

## Description

TECHNICAL FIELD

The present invention relates to a drug for a combination therapy for preventing and/or treating an autoimmune disease.

BACKGROUND ART

Normal living bodies do not exhibit immune response to their own constituents (autoantigens) and maintain immunological homeostasis. Such homeostasis is called innate immune tolerance. This tolerance state is broken in some cases, causing antibodies or lymphocytes reacting with autoantigens. Such a phenomenon is called autoimmunity, and diseases triggered by the autoimmunity are called autoimmune diseases. The autoimmune diseases are divided into systemic autoimmune diseases and organ-specific autoimmune diseases. Representative examples of the former include systemic lupus erythematosus and rheumatoid arthritis, while representative examples of the latter include multiple sclerosis, myasthenia gravis, autoimmune hemolytic anemia, and Hashimoto thyroiditis. The systemic autoimmune diseases were once categorized as collagen diseases characterized by the hyperplasia of connective tissues. However, studies have been conducted on their causes in recent years, suggesting at least partial involvement of autoimmunity in the causes. However, much remains to be elucidated. On the other hand, most of the organ-specific autoimmune diseases clearly show the pathogenic roles of autoantibodies. It has been deduced from previous studies that the organ-specific autoimmune diseases are caused by T-lymphocytes stimulated with antigens. A variety of views of the mechanisms of autoantibody production have been created. The first theory is that genes responsible for particular autoantigen production are involved therein. The second theory is that antigens usually present in only trace amounts in blood are released in large amounts for some reason or that antigens are modified for some reason. The third theory is that lymphocytes responsible for antibody production are abnormal and deviate from the tolerance state. Probably, these factors are combined to cause autoantibody production.

Rheumatoid arthritis, one of autoimmune diseases, is a systemic inflammatory disease of unknown etiology involving polyarthritis as the main symptom and simultaneously causing multiple organ failures. Rheumatoid arthritis chronically progresses, while showing repeated remission and exacerbation. This disease, when left untreated, leads to joint destruction and deformity and finally to motor dysfunction and can sometimes threaten the lives of patients. However, the current medicine is supposed to neither completely cure nor prevent rheumatoid arthritis. Thus, the treatment goal at the present time is to improve the physical, mental, and social QOL (quality of life) of patients by diagnosing rheumatoid arthritis at the early stage, initiating an aggressive therapy from the early stage, inhibiting rheumatic inflammation as quickly as possible and to the fullest extent, preventing the occurrence of irreversible changes, and blocking the progression thereof.

Main drugs used in the treatment of rheumatoid arthritis are nonsteroidal anti-inflammatory drugs (hereinafter, referred to as "NSAIDs"), disease modifying antirheumatic drugs (hereinafter, also referred to as "DMARDs"), and adrenocorticosteroid drugs.

NSAIDs are drugs that are used first for patients with rheumatoid arthritis. Although their analgesic effects are quickly produced, their anti-inflammatory effects are expressed 1 to 2 weeks later. NSAIDs have the effect of attenuating the extent of inflammation but have no effect on blocking the progression of rheumatic inflammation and preventing joint destruction. Most of the side effects of NSAIDs are closely linked to their pharmacological actions (cyclooxygenase-1 inhibitory effect), and gastrointestinal toxicity and so on often prevents the treatment.

DMARDs are generic names for drugs that are intended to suppress rheumatic inflammation by correcting the immune abnormality of rheumatoid arthritis and induce remission. DMARDs are generally slow-acting. However, once their effects are expressed, the effects last over a long period. DMARDs have no analgesic effect and are therefore used in combination with quick-acting NSAIDs or adrenocorticosteroid drugs at the start of administration. On the other hand, the known problems of DMARDs are that serious side effects such as myelosuppression, nephritis, and interstitial pneumonitis appear on rare occasion and that the optimum dose thereof differs among individuals. Furthermore, tolerance is often shown to conventional DMARDs administered continuously (escape phenomenon). Their effects are diminished in 2 to 3 years, and the administration thereof is discontinued in many cases.

The adrenocorticosteroid drugs inhibit rheumatic inflammation quickly and reliably, and significantly improve the QOL of patients with rheumatoid arthritis, albeit for a short time.
However, they have many harmful effects, for example, serious side effects such as the effects diminished by long-term continuous use, difficulty in withdrawal (rebound phenomenon and withdrawal syndrome), incapability of blocking the progression of joint destruction, induction of infectious diseases, adrenal gland dysfunction, gastrointestinal dysfunction, and osteoporosis.

It is known that various cells are involved in the pathogenesis of autoimmune diseases, particularly rheumatoid

arthritis. Representative examples thereof include T-lymphocytes, macrophage cells, osteoclasts (involved in joint destruction), fibroblasts (involved in ankylosis) (G. S. Firestein et al., "RHEUMATOID ARTHRITIS"; OXFORD UNIVERSITY PRESS, 2000) .

Although studies have been conducted on a combination therapy of DMARDs (for example, a combination therapy of leflunomide and methotrexate) for intractable rheumatoid arthritis, patients with intractable or drug resistant rheumatoid arthritis against the therapy have also been observed. Moreover, studies have heretofore been conducted on treatment methods in which several nonsteroidal anti-inflammatory drugs, DMARDs, and adrenocorticosteroid drugs are changed along with the states of patients. For example, pyramid, Sawtooth, and Step-down bridge approaches have been attempted.

1. Pyramid approach

In this approach, nonsteroidal anti-inflammatory drugs are used first. At a point in time when they became ineffective, DMARDs or adrenocorticosteroid drugs are added.

2. Sawtooth approach

In this approach, DMARDs are used from the beginning. At a point in time when they became ineffective (escape phenomenon), they are changed to next DMARDs. Multiple-agent use of DMARDs is also practiced along with the approach.

3. Step-down bridge approach

In this approach, multiple-agent use of adrenocorticosteroid drugs and DMARDs is performed from the beginning. At a stage in which inflammation could be inhibited, the drug use is discontinued in order from strong DMARDs to weak.
However, even the use of these approaches does not exert sufficiently effective prophylactic agents and/or therapeutic agents for autoimmune diseases, particularly rheumatoid arthritis. Thus, more excellent prophylactic agents and/or therapeutic agents for autoimmune diseases have been demanded.

Calcium/calmodulin-dependent protein kinase II (CaM kinase II: abbreviated herein to "CaMKII") is an enzyme found in 1980 as a new type of calmodulin-dependent protein kinase that activates tryptophan hydroxylase (rate-limiting enzyme for the biosynthesis of serotonin, one of neurotransmitters) in the rat brain. This enzyme has been known to be activated by complexes of $Ca^{2+}$ and calmodulin increased in number in cells by extracellular stimulation. Progress in recent studies has revealed that this enzyme plays an important role in control of higher nervous functions such as memory and in regulation of a variety of cellular functions. This enzyme has such distinctive characteristics that 1) the enzymes are present in large amounts in the brain, 2) they are classified into soluble and granule-bound types, 3) they have wide substrate specificity, 4) they include organ-specific isoforms, and 5) their activities are controlled by autophosphorylation. Particularly, it has been suggested that the enzyme is involved in neurotransmitter biosynthesis, neurotransmitter release, long-term potentiation action probably serving as the basic principle of memory, and memory, particularly spatial memory, in the cerebral nervous system. CaMKII isoforms (subtypes) known so far are CaMKIIα, β, β', γ, and δ. CaMKIIγ is known to further include γA, γB, and γC. Of them, the isoforms CaMKIIα, β, and β' are localized in the brain.
However, there has been no known report that the prophylaxis and/or treatment of an autoimmune disease can be achieved by regulating CaMKII expression or activity. Besides, it has not been known that CaMKII inhibitors and DMARDs are used in combination for the purpose of preventing and/or treating an autoimmune disease.

Specific examples of the CaMKII inhibitors include the followings:
KN-93: N-(2-[N-[4-chlorocinnamyl]-N-methylaminomethyl]phenyl)-N-(2-hydroxyethyl)-4-methoxybenzenesulfonamide (JP-A-6-293730 and Non-Patent Document 1)

[Formula 1]

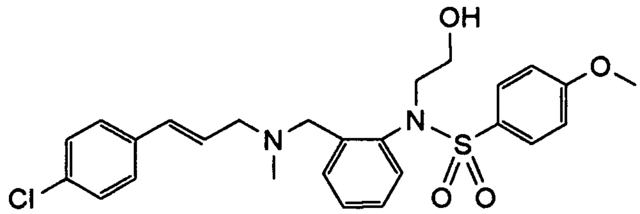

1-[1-oxo-11-(5,6-dihydro-3-iminobenzo[h]cinnolin-2(3H)-yl)-undecyl]-4-(2-pyrimidinyl)-piperazine (Non-Patent Document 2)

[Formula 2]

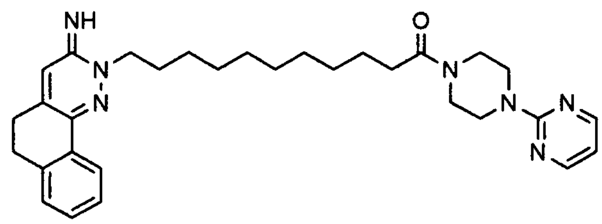

Peptide having an amino acid sequence represented by the sequence Lys-Lys-X-Leu-Arg-Arg-Gln-Glu-Ala-Phe-Asp-Ala-Tyr (wherein X represents Ala or Lys) (Patent Document 1)

Peptide having an amino acid sequence represented by the sequence Lys-Lys-Ala-Leu-His-Arg-Gln-Glu-Ala-Val-Asp-Cys-Leu (KKALHRQEAVDCL) (Patent Document 2 and JP-A-2001-509808; which have suggested antiarrhythmic applications thereof)

However, effects on an autoimmune disease have not been known about these specific CaMKII inhibitors.

On the other hand, it has been reported that 3-[(1S)-1-(2-fluorobiphenyl-4-yl)ethyl]-5-{[amino(morpholin-4-yl) methylene]amino}isoxazole (hereinafter, also referred to as a "compound (I)") represented by the following Formula 1a has anti-arthritis effects on adjuvant arthritis rats and collagen-induced arthritis rats used as representative rheumatoid arthritis model animals (see e.g., Patent Document 3 and Non-Patent Documents 3 to 5). Formula 1a:

[Formula 3]

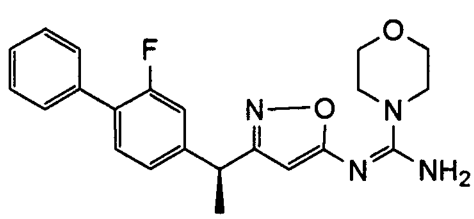

It has also been reported that 4-[1-(2-fluorobiphenyl-4-yl)ethyl]-2-(methylamino)thiazole (hereinafter, also referred to as a "compound (II)") represented by the following Formula 2 has anti-arthritis effects on adjuvant arthritis rats and has inhibitory effects on edema in mouse type III allergy reaction tests (see e.g., Patent Document 4).

Formula 2:

[Formula 4]

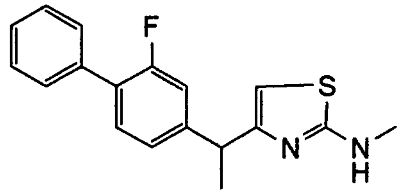

However, any effect of the compounds (I) and (II) on CaMKII has not been known so far. Moreover, any effect of combined use of these compounds and DMARDs has not been known so far.

Patent Document 1: JP-A-11-152298
Patent Document 2: Pamphlet of International Publication No. WO 98/33491
Patent Document 3: Japanese Patent No. 3237608
Patent Document 4: JP-B-4-80035
Non-Patent Document 1: Biochem Biophys Res Commun 1991; 181 (3): p. 968-75
Non-Patent Document 2: J. Med. Chem., 2002, 45, p. 563-566
Non-Patent Document 3: S. Tagashira, "Inflammation and Regeneration" Vol. 21, No. 4, p. 472 (2001)
Non-Patent Document 4: F. Nishikaku, "Annals of the Rheumatic Diseases", vol. 60 supplement 1, p. 159 (2001)
Non-Patent Document 5: F. Nishikaku, "Annals of the Rheumatic Diseases", vol. 61 supplement 1, p. 194 (2002)

DISCLOSURE OF THE INVENTION

An object of the present invention is to provide an excellent drug for a combination therapy for preventing and/or treating an autoimmune disease. A further object of the present invention is to provide a screening method useful for searching and obtaining candidate substances serving as an active ingredient in the drug for a combination therapy.

The present inventors have conducted diligent studies for attaining the objects and have consequently gained the following surprising findings I and II:

I. A CaMKII inhibitor is useful as a prophylactic agent and/or therapeutic agent for an autoimmune disease.

(1) Compounds (I) and (II) and derivatives thereof exhibit CaMKII inhibitory effects (Examples 1 and 2).
(2) CaMKII inhibitors such as the compound I and KN-93 act in inhibitory manners on various cells (T-lymphocytes, macrophage cells, osteoclasts, fibroblasts, synovial cells, and so on) involved in the pathogenesis of autoimmune diseases, particularly rheumatoid arthritis (Examples 3 to 7).
(3) KN-93 or the like known as a CaMKII inhibitor in the art exhibits usefulness for autoimmune disease animal models (Example 10).
(4) The proportion of CaMKII producing cells is increased in the affected parts of autoimmune disease animal models, that is, CaMKII is involved in the pathogenesis of autoimmune diseases (Example 12).
(5) The compound (I) and KN-93 have the following effects (a) to (c) on a variety of factors varying due to phosphorylation by CaMKII:

(a) The compound (I) and KN-93 inhibit IκBα degradation (Example 13).
(b) The compound (I) and KN-93 inhibit cyclin D1 expression enhanced by bFGF (Example 14).
(c) The compound (I) and KN-93 inhibit Akt phosphorylation (Example 14).

(6) CaMKII expression is induced in monocyte lineage cells by inflammatory stimulation, that is, CaMKII is involved in the pathogenesis of autoimmune diseases (Example 15).
(7) CaMKII sequence-specific siRNA inhibits the proliferation of synovial cells (Example 16), that is, the inhibition of CaMKIIγ expression or activity inhibits the abnormal growth of synovial cells and can thereby achieve improvement in the disease states of joints (e.g., inhibition of chronic inflammation, inhibition of joint destruction, inhibition of progression to joint deformity) in diseases such as rheumatoid arthritis

(8) The compound (I) exhibits an excellent inhibitory effect on joint destruction in rheumatoid arthritis animal models (Examples 20 and 21).

Many factors varying in relation to CaMKII are known such as IκBα, cytoplasmic phospholipase A2 (cPLA2), cell cycle factor cyclin D1, cell surface receptor CD44, EGF receptor, and Stat1 (J. Biol. Chem. 276, 36008-36013; J. Biol. Chem. 276, 39653-39660; Exp. Cell Res. 240, 218-227; Biochem. J. 357, 843-850; J. Biol. Chem. 274, 16168-16173; and Proc. Natl. Acad. Sci. USA 99, 5971-5976).
The present inventors have diligent studies by focusing on IκBα and cyclin D1 among them and have consequently gained the finding (5). These findings suggest that the CaMKII inhibitors such as the compound (I) inhibit T-lymphocyte activation through the inhibition of IκBα, degradation. Moreover, they suggest that the CaMKII inhibitors such as the compound (I) inhibit CaMKII in fibroblasts, thereby terminating the cell cycle, that is, inhibiting the cell growth through inhibiting downstream cyclin D1 expression via inhibiting Akt phosphorylation.
Based on the findings, the present inventors have found that these compounds (e.g., the compound (I) and KN-93) exert an improving effect on the state of an autoimmune disease via their CaMKII inhibitory effects, and have been convinced that a substance that inhibits CaMKII expression or activity can be utilized effectively as a prophylactic agent and/or therapeutic agent for an autoimmune disease.

II. The combined use of the CaMKII inhibitor and DMARDs achieves an excellent combined effect in the prophylaxis and/or treatment of an autoimmune disease.

The present inventors have used the compound I as a CaMKII inhibitor in combination with a variety of disease modifying antirheumatic drugs (DMARDs) and
have consequently found that this combined use has a remarkably excellent prophylactic and/or therapeutic effect on an autoimmune disease, which cannot be expected from an effect obtained by each single use of them, that is, the combined use of the CaMKII inhibitor and DMARDs exhibits remarkable usefulness in the prophylaxis and/or treatment of an autoimmune disease (Examples 17 to 23). Particularly, the present inventors have found that a combination therapy of the CaMKII inhibitor and DMARDs exhibits an excellent inhibitory effect on joint destruction that can hardly receive sufficient effects from a single-agent therapy of conventionally known antirheumatic drugs (Example 20) .
The present invention can achieve an excellent prophylactic and/or therapeutic effect on an autoimmune disease, which cannot be achieved by treatment using only either the CaMKII inhibitor or DMARDs. Moreover, the present invention can also achieve an excellent prophylactic and/or therapeutic effect on an autoimmune disease with reduced side effects by the combined use of the CaMKII inhibitor and DMARDs at each reduced dose, even when treatment using only either the CaMKII inhibitor or DMARDs presents side effect problems.
The present invention has been completed based on the findings I and II.
Specifically, the present invention is as follows:

[1] A prophylactic agent and/or therapeutic agent for an autoimmune disease comprising a CaMKII inhibitor and a disease modifying antirheumatic drug.
[2] A prophylactic agent and/or therapeutic agent for an autoimmune disease comprising a CaMKII inhibitor for use in combination with a disease modifying antirheumatic drug.
[3] The prophylactic agent and/or therapeutic agent according to item [2], wherein the prophylactic agent and/or therapeutic agent is administered simultaneously with, separately from, or subsequent to administration of the disease modifying antirheumatic drug.
[4] A prophylactic agent and/or therapeutic agent for an autoimmune disease comprising a CaMKII inhibitor, which is intended to be administered simultaneously with, separately from, or subsequent to administration of a disease modifying antirheumatic drug to a mammal with an autoimmune disease to which the disease modifying antirheumatic drug in an amount giving a sufficient prophylactic and/or therapeutic effect on the autoimmune disease cannot be administered due to a side effect.
[5] The prophylactic agent and/or therapeutic agent according to item [4], wherein the side effect is one or several side effects selected from hepatic disorder, renal disorder, and gastrointestinal disorder.

[6] The prophylactic agent and/or therapeutic agent according to any of items [1] to [5], wherein the CaMKII inhibitor is an isoxazole derivative represented by Formula 1 or a pharmaceutically acceptable salt thereof:
Formula 1:
[Formula 5]

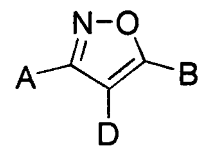

[wherein D represents a hydrogen atom, a halogen atom, a hydroxy group, a mercapto group, a nitro group, a cyano group, a carboxy group, an amino group which may be substituted, a hydroxylamino group which may be substituted, a carbamoyl group which may be substituted, a sulfamoyl group which may be substituted, a sulfo group, $-R^5$, $-OR^5$, $-CO_2R^6$, $-SR^7$, $-(CO)SR^7$, $-(CS)OR^7$,or $-CS_2R^7$ (wherein $R^5$ represents an alkyl group which may be substituted, an alkenyl group which may be substituted, an alkynyl group which may be substituted, a cycloalkyl group which may be substituted, a cycloalkenyl group which may be substituted, an aryl group which may be substituted, a heterocyclic group which may be substituted, or an acyl group; $R^6$ represents an alkyl group which may be substituted, an alkenyl group which may be substituted, an alkynyl group which may be substituted, an aryl group which may be substituted, or a heterocyclic group which may be substituted; $R^7$ represents an alkyl group which may be substituted or an aryl group which may be substituted);

one of A and B represents a group represented by Formula:

[Formula 6]

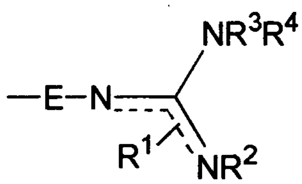

(E represents a single bond or an alkylene group;

one of two broken lines denotes a double bond together with a solid line, and the other denotes a single bond together with a solid line; $R^1$ binds to a nitrogen atom binding to the bond in which the broken line denotes the single bond together with the solid line;

$R^1$, $R^2$, $R^3$, and $R^4$ each independently represent a hydrogen atom, a halogen atom, a hydroxy group, a mercapto group, a nitro group, a cyano group, a carboxy group, an amino group which may be substituted, a hydroxylamino group which may be substituted, a carbamoyl group which may be substituted, a sulfamoyl group which may be substituted, a sulfo group, a protecting group for an NH group, $-R^5$, $-OR^5$, $-CO_2R^6$, $-SR^7$, $-(CO)SR^7$, $-(CS)OR^7$, or $-CS_2R^7$ (wherein $R^5$, $R^6$. and $R^7$ represent the same as above); or any two of $R^1$, $R^2$, $R^3$, and $R^4$ may bind together and form, together with a nitrogen atom, a heterocyclic ring which may be substituted; or Formula: $-NR^3R^4$ may represent a group represented by Formula: $-N=C(NH_2)NR^{43}R^{44}$;

$R^{93}$ and $R^{44}$ represent the following (1) or (2):

(1) $R^{93}$ and $R^{44}$ each independently represent a hydrogen atom, an alkyl group having 1 to 4 carbon atoms, $-(CH_2)_n-COCH_3$ (n represents an integer of 1 to 3), $-(CH_2)_n-CO_2R^{32}$ (n represents the same as above; $R^{32}$ represents an alkyl group having 1 to 3 carbon atoms), $-(CH_2)_n-CONR^{33}R^{34}$ (n represents the same as above; $R^{33}$ and $R^{34}$ each independently represent a hydrogen atom or an alkyl group having 1 to 3 carbon atoms), $-(CH_2)_m-OR^{35}$ (m represents 2 or 3; $R^{35}$ represents a hydrogen atom, an alkyl group having 1 to 3 carbon atoms, or $-(CH_2)_m-OR^{36}$ (m represents the same as above; $R^{36}$ represents a hydrogen atom or an alkyl group having 1 to 3 carbon atoms)), $-(CH_2)_m-NR^{37}R^{38}$ (m represents the same as above; $R^{37}$ and $R^{38}$ each independently represent a hydrogen atom or an alkyl group having 1 to 3 carbon atoms, or together represent pyrrolidine, piperidine, azepane, morpholine, or N-methylpiperazine (wherein the pyrrolidine, piperidine, azepane, morpholine, and N-methylpiperazine may be substituted by one or two methyl groups) together with a nitrogen atom), phenyl, pyridyl, pyrimidinyl, pyridazinyl, pyrazinyl, tetrazolyl, benzyl, pyridylmethyl, pyrimidinylmethyl, pyridazinylmethyl, pyrazinylmethyl, tetrazolylmethyl, a hydroxy group, an alkoxy group having 1 to 3 carbon atoms, or $-NR^{39}R^{40}$ ($R^{39}$ and $R^{40}$ each independently

represent a hydrogen atom, an alkyl group having 1 to 3 carbon atoms, phenyl, or pyridyl); and
(2) $R^{43}$ and $R^{44}$ together represent a 5- to 7-membered saturated nitrogen-containing heterocyclic group (the 5- to 7-membered saturated nitrogen-containing heterocyclic group may be substituted by one or two substituents optionally selected from the group consisting of an alkyl group, an amino group, a hydroxy group, an alkoxy group, and an oxo group) together with a nitrogen atom);
the other of A and B represents a group represents by Formula: -J-G
(wherein G represents an aryl group which may be substituted or a heterocyclic group which may be substituted; J represents $-C(R^8R^9)$ - or $-C(=CR^8R^9)$-; $R^8$ and $R^9$ each independently represent a hydrogen atom, a lower alkoxy group which may be substituted, or a lower alkyl group which may be substituted; or $R^8$ and $R^9$ may bind together and form, together with a carbon atom, a hydrocarbon ring which may be substituted, a 1,3-dioxane ring which may be substituted, or a 1,3-dioxolane ring which may be substituted)].

[7] The prophylactic agent and/or therapeutic agent according to item [6], wherein E is a single bond or a lower alkylene group.
[8] The prophylactic agent and/or therapeutic agent according to item [6] or [7], wherein D is a hydrogen atom, a nitro group, a cyano group, a carboxy group, an amino group which may be substituted, a hydroxylamino group which may be substituted, a carbamoyl group which may be substituted, $-R^5$, or $-CO_2R^6$ (wherein $R^5$ and $R^6$ represent the same as in item [6]). [9] The prophylactic agent and/or therapeutic agent according to item [8], wherein D is a hydrogen atom, a carboxy group, $-R^5$, or $-CO_2R^6$ (wherein $R^5$ and $R^6$ represent the same as in item [6]).
[10] The prophylactic agent and/or therapeutic agent according to any of items [6] to [9], wherein $R^1$, $R^2$, $R^3$, and $R^4$ each independently represent a hydrogen atom, a hydroxy group, an amino group which may be substituted, a hydroxylamino group which may be substituted, an alkoxy group which may be substituted, an alkyl group which may be substituted, an alkenyl group which may be substituted, an alkynyl group which may be substituted, a cycloalkyl group which may be substituted, a cycloalkenyl group which may be substituted, an aryl group which may be substituted, a heterocyclic group which may be substituted, or an acyl group; or Formula: $-NR^3R^4$ represents a group represented by Formula: $-N=C (NH_2) NR^{43}R^{44}$ ($R^{43}$ and $R^{44}$ represent the same as in item [6]) ; or any two of $R^1$ $R^2$, $R^3$, and $R^4$ bind together and form, together with a nitrogen atom, a heterocyclic ring which may be substituted.
[11] The prophylactic agent and/or therapeutic agent according to item [10], wherein $R^1$, $R^2$, $R^3$, and $R^4$ each independently represent a hydrogen atom, a hydroxy group, an amino group which may be substituted, a hydroxylamino group which may be substituted, an alkoxy group which may be substituted, an alkyl group which may be substituted, an alkenyl group which may be substituted, an alkynyl group which may be substituted, a cycloalkyl group which may be substituted, an aryl group which may be substituted, a heterocyclic group which may be substituted, or an acyl group; or Formula: $-NR^3R^4$ represents a group represented by Formula: $-N=C(NH_2)NR^{43}R^{44}$ ($R^{43}$ and $R^{49}$ represent the same as in item [6]) ; or any two of $R^1$, $R^2$, $R^3$, and $R^4$ bind together and form, together with a nitrogen atom, a heterocyclic ring which may be substituted.
[12] The prophylactic agent and/or therapeutic agent according to any of items [6] to [11], wherein G is phenyl which may be substituted, naphthyl which may be substituted, furyl which may be substituted, thienyl which may be substituted, indolyl which may be substituted, isothiazolyl which may be substituted, benzothienyl which may be substituted, isobenzofuranyl which may be substituted, pyrrolyl which may be substituted, benzofuryl which may be substituted, imidazolyl which may be substituted, pyrazolyl which may be substituted, isoxazolyl which may be substituted, isothiazolyl which may be substituted, thiazolyl which may be substituted, oxazolyl which may be substituted, benzimidazolyl which may be substituted, benzothiazolyl which may be substituted, benzoxazolyl which may be substituted, pyridyl which may be substituted, pyrazinyl which may be substituted, pyrimidinyl which may be substituted, pyridazinyl which may be substituted, triazinyl which may be substituted, quinolyl which may be substituted, isoquinolyl which may be substituted, quinazolinyl which may be substituted, quinoxalinyl which may be substituted, 2,3-dihydro-benzo[1,4]dioxinyl which may be substituted, or carbazolyl which may be substituted.

[13] The prophylactic agent and/or therapeutic agent according to item [6], wherein the CaMKII inhibitor is an isoxazole derivative represented by Formula A1 or A2 or a pharmaceutically acceptable salt thereof:
Formula A1:

[Formula 7]

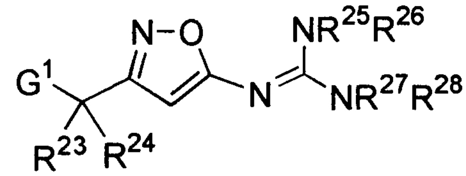

[wherein $G^1$ represents phenyl, biphenyl-4-yl, 3-benzoylphenyl, 4-benzoylphenyl, 1H-indol-2-yl, 1H-indol-3-yl, 1-methyl-1H-indol-2-yl, 1-methyl-1H-indol-3-yl, 2,3-dihydro-benzo[1,4]dioxin-6-yl, 1-benzofuran-5-yl, 1-benzofuran-6-yl, quinolyl, isoquinolyl, phenylpyridyl, phenylpyrimidinyl, phenylpyridazinyl, or phenylpyrazinyl (wherein the phenyl, biphenyl-4-yl, 3-benzoylphenyl, 4-benzoylphenyl, 1H-indol-2-yl, 1H-indol-3-yl, 1-methyl-1H-indol-2-yl, 1-methyl-1H-indol-3-yl, 2,3-dihydro-benzo[1,4]dioxin-6-yl, 1-benzofuran-5-yl, 1-benzofuran-6-yl, quinolyl, isoquinolyl, phenylpyridyl, phenylpyrimidinyl, phenylpyridazinyl, and phenylpyrazinyl may be substituted by one or two groups optionally selected from the group consisting of a fluorine atom, a chlorine atom, a bromine atom, acetyl, cyano, $-CO_2R^{29}$ ($R^{29}$ represents an alkyl group having 1 to 3 carbon atoms), and $-CONR^{30}R^{31}$ ($R^{30}$ and $R^{31}$ each independently represent a hydrogen atom or an alkyl group having 1 to 3 carbon atoms));
$R^{23}$ and $R^{24}$ each independently represent a hydrogen atom, an alkyl group having 1 to 4 carbon atoms, methoxy, or ethoxy, or together represent methylene;
Formula: $=C(NR^{25}R^{26})NR^{27}R^{28}$ represents the following (1), (2), or (3):

(1) $R^{25}$ and $R^{26}$ represent the following (a) or (b) and $R^{27}$ and $R^{28}$ represent the following (c) or (d):

(a) $R^{25}$ and $R^{26}$ each independently represent a hydrogen atom, an alkyl group having 1 to 4 carbon atoms, $-(CH_2)_n-COCH_3$ (n represents the same as in item [6]), $-(CH_2)_n-CO_2R^{32}$ (n and $R^{32}$ represent the same as in item (61), $-(CH_2)_n-CONR^{33}R^{34}$ (n, $R^{33}$, and $R^{34}$ represent the same as in item [6]), $-(CH_2)_m-OR^{35}$ (m and $R^{35}$ represent the same as in item [6]), $-(CH_2)_m-NR^{3}7R^{38}$ (m, $R^{37}$, and $R^{38}$ represent the same as in item [6]), phenyl, pyridyl, pyrimidinyl, pyridazinyl, pyrazinyl, tetrazolyl, benzyl, pyridylmethyl, pyrimidinylmethyl, pyridazinylmethyl, pyrazinylmethyl, tetrazolylmethyl, a hydroxy group, an alkoxy group having 1 to 3 carbon atoms, or $-NR^{39}R^{40}$ ($R^{39}$ and $R^{40}$ represent the same as in item [6]);
(b) $R^{25}$ and $R^{26}$ together represent a 5- to 7-membered saturated nitrogen-containing heterocyclic group (wherein the 5- to 7-membered saturated nitrogen-containing heterocyclic group may be substituted by one or two substituents optionally selected from the group consisting of an alkyl group, an amino group, a hydroxy group, an alkoxy group, and an oxo group) together with a nitrogen atom;
(c) $R^{27}$ and $R^{28}$ each independently represent a hydrogen atom, an alkyl group having 1 to 4 carbon atoms, $-(CH_2)_n-COCH_3$ (n represents the same as above), $-(CH_2)_n-CO_2R^{32}$ (n and $R^{32}$ represent the same as above), $-(CH_2)_n-CONR^{33}R^{34}$ (n, $R^{33}$, and $R^{34}$ represent the same as above), $-(CH_2)_m-OR^{35}$ (m and $R^{35}$ represent the same as above), $-(CH_2)_m-NR^{37}R^{38}$ (m, $R^{37}$, and $R^{38}$ represent the same as above), phenyl, pyridyl, pyrimidinyl, pyridazinyl, pyrazinyl, tetrazolyl, benzyl, pyridylmethyl, pyrimidinylmethyl, pyridazinylmethyl, pyrazinylmethyl, tetrazolylmethyl, a hydroxy group, an alkoxy group having 1 to 3 carbon atoms, or $-NR^{39}R^{40}$ ($R^{39}$ and $R^{40}$ represent the same as above); and
(d) $R^{27}$ and $R^{28}$ together represent a 5- to 7-membered saturated nitrogen-containing heterocyclic group (wherein the 5- to 7-membered saturated nitrogen-containing heterocyclic group may be substituted by one or two substituents optionally selected from the group consisting of an alkyl group, an amino group, a hydroxy group, an alkoxy group, and an oxo group) together with a nitrogen atom;

(2) $R^{26}$ and $R^{27}$ together represent a 5- to 7-membered saturated nitrogen-containing heterocyclic group (wherein the 5- to 7-membered saturated nitrogen-containing heterocyclic group may be substituted by one or two substituents optionally selected from the group consisting of an alkyl group, an amino group, a hydroxy group, an alkoxy group, and an oxo group) together with two nitrogen atoms and a carbon atom;
$R^{25}$ and $R^{28}$ each independently represent a hydrogen atom, an alkyl group having 1 to 3 carbon atoms, acetyl, or $-(CH_2)_m-OR^{36}$ (m represents the same as above; $R^{36}$ represents the same as in item [6]); and
(3) Formula: $=C(NR^{25}R^{26})NR^{27}R^{28}$ represents Formula: $=C(NR^{41}R^{42})\ N=C\ (NH_2)\ NR^{43}R^{44}$;

$R^{41}$ and $R^{42}$ represent the following ($a^1$) or ($b^1$) and $R^{43}$ and $R^{44}$ represent the following ($c^1$) or ($d^1$):

($a^1$) $R^{41}$ and $R^{42}$ each independently represent a hydrogen atom, an alkyl group having 1 to 4 carbon atoms, $-(CH_2)_n-COCH_3$ (n represents the same as above),$-(CH_2)_n-CO_2R^{32}$ (n and $R^{32}$ represent the same as above),$-(CH_2)_n-CONR^{33}R^{34}$ (n, $R^{33}$, and $R^{34}$ represent the same as above), $-(CH_2)_m-OR^{35}$ (m and $R^{35}$ represent the same as above), $-(CH_2)_m-NR^{37}R^{38}$ (m, $R^{37}$, and $R^{38}$ represent the same as above), phenyl, pyridyl, pyrimidinyl, pyridazinyl, pyrazinyl, tetrazolyl, benzyl, pyridylmethyl, pyrimidinylmethyl, pyridazinylmethyl, pyrazinylmethyl, tetrazolylmethyl, a hydroxy group, an alkoxy group having 1 to 3 carbon atoms, or $-NR^{39}R^{40}$ ($R^{39}$ and $R^{40}$ represent the same as above);
($b^1$) $R^{41}$ and $R^{42}$ together represent a 5- to 7-membered saturated nitrogen-containing heterocyclic group (wherein the 5- to 7-membered saturated nitrogen-containing heterocyclic group may be substituted by one or two substituents optionally selected from the group consisting of an alkyl group, an amino group, a hydroxy group, an alkoxy group, and an oxo group) together with a nitrogen atom;
($c^1$) $R^{43}$ and $R^{44}$ each independently represent a hydrogen atom, an alkyl group having 1 to 4 carbon atoms, $-(CH_2)_n-COCH_3$ (n represents the same as above), $-(CH_2)_n-CO_2R^{32}$ (n and $R^{32}$ represent the same as above), $-(CH_2)_n-CONR^{33}R^{34}$ (n, $R^{33}$, and $R^{34}$ represent the same as above), $-(CH_2)_m-OR^{35}$ (m and $R^{35}$ represent the same as above), $-(CH_2)_m-NR^{37}R^{38}$ (m, $R^{37}$, and $R^{38}$ represent the same as above), phenyl, pyridyl, pyrimidinyl, pyridazinyl, pyrazinyl, tetrazolyl, benzyl, pyridylmethyl, pyrimidinylmethyl, pyridazinylmethyl, pyrazinylmethyl, tetrazolylmethyl, a hydroxy group, an alkoxy group having 1 to 3 carbon atoms, or $-NR^{39}R^{40}$ ($R^{39}$ and $R^{40}$ represent the same as above); and
($d^1$) $R^{43}$ and $R^{44}$ together represent a 5- to 7-membered saturated nitrogen-containing heterocyclic group (wherein the 5- to 7-membered saturated nitrogen-containing heterocyclic group may be substituted by one or two substituents optionally selected from the group consisting of an alkyl group, an amino group, a hydroxy group, an alkoxy group, and an oxo group) together with a nitrogen atom];

Formula A2:
[Formula 8]

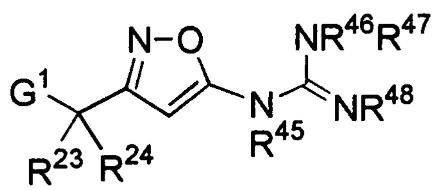

[wherein $G^1$, $R^{23}$, and $R^{24}$ represent the same as above;
Formula: $-N(R^{45})C(NR^{46}R^{47})=NR^{48}$ represents the following ($1^1$) or ($2^1$) :

($1^1$) $R^{45}$ represents an alkyl group having 1 to 3 carbon atoms or acetyl. $R^{48}$ represents a hydrogen atom, an alkyl group having 1 to 3 carbon atoms, or acetyl;
$R^{46}$ and $R^{47}$ represent the following ($a^2$) or ($b^2$) :

($a^2$) $R^{46}$ and $R^{47}$ each independently represent a hydrogen atom, an alkyl group having 1 to 4 carbon atoms, $-(CH_2)_n-COCH_3$ (n represents the same as above), $-(CH_2)_n-CO_2R^{32}$ (n and $R^{32}$ represent the same as above), $-(CH_2)_n-CONR^{33}R^{34}$ (n, $R^{33}$, and $R^{34}$ represent the same as above), $-(CH_2)_m-OR^{35}$ (m and $R^{35}$ represent the same as above), $-(CH_2)_m-NR^{37}R^{38}$ (m, $R^{37}$, and $R^{38}$ represent the same as above), phenyl, pyridyl, pyrimidinyl, pyridazinyl, pyrazinyl, tetrazolyl, benzyl, pyridylmethyl, pyrimidinylmethyl, pyridazinylmethyl, pyrazinylmethyl, tetrazolylmethyl, a hydroxy group, an alkoxy group having 1 to 3 carbon atoms, or $-NR^{39}R^{40}$ ($R^{39}$ and $R^{40}$ represent the same as above); and
($b^2$) $R^{46}$ and $R^{47}$ together represent a 5- to 7-membered saturated nitrogen-containing heterocyclic group (wherein the 5- to 7-membered saturated nitrogen-containing heterocyclic group may be substituted by one or two substituents optionally selected from the group consisting of an alkyl group, an amino group, a hydroxy group, an alkoxy group, and an oxo group) together with a nitrogen atom; and
($2^1$) $R^{45}$ and $R^{46}$ together represent a 5- to 7-membered saturated nitrogen-containing heterocyclic group

(wherein the 5- to 7-membered saturated nitrogen-containing heterocyclic group may be substituted by one or two substituents optionally selected from the group consisting of an alkyl group, an amino group, a hydroxy group, an alkoxy group, and an oxo group) together with a nitrogen atom;

$R^{47}$ and $R^{48}$ each independently represent a hydrogen atom, an alkyl group having 1 to 3 carbon atoms, acetyl, or $-(CH_2)m-OR^{36}$ (m and $R^{36}$ represent the same as above)].

[14] The prophylactic agent and/or therapeutic agent according to item [6], wherein the CaMKII inhibitor is an isoxazole derivative represented by Formula A3 or a pharmaceutically acceptable salt thereof:
Formula A3:

[Formula 9]

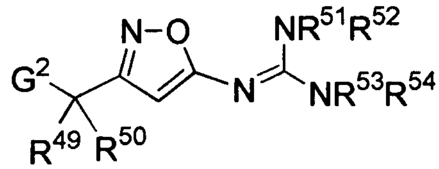

[wherein $G^2$ represents 2-fluoro-biphenyl-4-yl, 2'-fluoro-biphenyl-4-yl, or 3-benzoylphenyl; $R^{49}$ represents methyl; $R^{50}$ represents a hydrogen atom, methyl, methoxy, or ethoxy;
Formula: $=C(NR^{51}R^{52})NR^{53}R^{54}$ represents the following ($1^2$), ($2^2$), or ($3^2$) :

($1^2$) $R^{51}$ and $R^{52}$ represent the following ($a^3$), ($b^3$), or ($c^3$) and $R^{53}$ and $R^{54}$ represent the following ($d^3$), ($e^3$), or ($f^3$) :

($a^3$) $R^{51}$ and $R^{52}$ each independently represent a hydrogen atom or an alkyl group having 1 to 4 carbon atoms;
($b^3$) one of $R^{51}$ and $R^{52}$ represents a hydrogen atom, and the other represents $-(CH_2)_n-COCH_3$ (n represents the same as in item [6]), $-(CH_2)_n-CO_2R^{32}$ (n and $R^{32}$ represent the same as in item [6]),$-(CH_2)_n-CONR^{33}R^{34}$ (n, $R^{33}$, and $R^{34}$ represent the same as in item [6]),$-(CH_2)_m-OR^{35}$ (m and $R^{35}$ represent the same as in item [6]), or $-(CH_2)_m-NR^{37}R^{38}$ (m, $R^{37}$, and $R^{38}$ represent the same as in item [6]);
($c^3$) $R^{51}$ and $R^{52}$ together represent pyrrolidine, azepane, morpholine, thiazoline, piperidin-2-one, piperidin-4-one, thiamorpholine, piperadine which may be substituted at the 4th position by an alkyl group having 1 to 3 carbon atoms, piperidine which may be substituted at the 4th position by an alkoxy group having 1 to 3 carbon atoms, 4-hydroxypiperidine, or piperidine substituted at the 4th position by an amino group which may be substituted by one or two alkyl groups having 1 to 3 carbon atoms (wherein the pyrrolidine, azepane, morpholine, thiazoline, piperidin-2-one, piperidin-4-one, thiamorpholine, piperadine which may be substituted at the 4th position by an alkyl group having 1 to 3 carbon atoms, piperidine which may be substituted at the 4th position by an alkoxy group having 1 to 3 carbon atoms, 4-hydroxypiperidine, and piperidine substituted at the 4th position by an amino group which may be substituted by one or two alkyl groups having 1 to 3 carbon atoms may be substituted by one or two methyl groups) together with a nitrogen atom;
($d^3$) $R^{53}$ and $R^{54}$ each independently represent a hydrogen atom or an alkyl group having 1 to 4 carbon atoms;
($e^3$) one of $R^{53}$ and $R^{54}$ represents a hydrogen atom, and the other represents $-(CH_2)_n-COCH_3$ (n represents the same as above), $-(CH_2)_n-CO_2R^{32}$ (n and $R^{32}$ represent the same as above), $-(CH_2)_n-CONR^{33}R^{34}$ (n, $R^{33}$, and $R^{34}$ represent the same as above), $-(CH_2)_m-OR^{35}$ (m and $R^{35}$ represent the same as above), or $-(CH_2)_m-NR^{37}R^{38}$ (m, $R^{37}$, and $R^{38}$ represent the same as above); and
($f^3$) $R^{53}$ and $R^{54}$ together represent pyrrolidine, azepane, morpholine, thiazoline, piperidin-2-one, piperidin-4-one, thiamorpholine, piperadine which may be substituted at the 4th position by an alkyl group having 1 to 3 carbon atoms, piperidine which may be substituted at the 4th position by an alkoxy group having 1 to 3 carbon atoms, 4-hydroxypiperidine, or piperidine substituted at the 4th position by an amino group which may be substituted by one or two alkyl groups having 1 to 3 carbon atoms (wherein the pyrrolidine, azepane, morpholine, thiazoline, piperidin-2-one, piperidin-4-one, thiamorpholine, piperadine which may be substituted at the 4th position by an alkyl group having 1 to 3 carbon atoms, piperidine which may be substituted at the 4th position by an alkoxy group having 1 to 3 carbon atoms, 4-hydroxypiperidine, and piperidine substituted at the 4th position by an amino group which may be substituted by one or two alkyl groups having 1 to 3 carbon atoms

may be substituted by one or two methyl groups) together with a nitrogen atom;

($2^2$) Formula: $=C(NR^{51}N^{52})NR^{53}R^{54}$ represents a group represented by Formula:

[Formula 10]

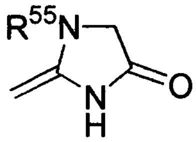

(wherein $R^{55}$ represents an alkyl group having 1 to 3 carbon atoms, acetyl, or - $(CH_2)_m$-$OR^{56}$ (m represents the same as above; $R^{56}$ represents a hydrogen atom or an alkyl group having 1 to 3 carbon atoms)); and

($3^2$) Formula: $=C(NR^{51}R^{52})NR^{53}R^{54}$ represents Formula: $=C\ (NR^{57}R^{58})\ N=C\ (NH_2)\ NR^{59}R^{60}$
$R^{57}$ and $R^{58}$ represent the following ($a^4$), ($b^4$), or ($C^4$), and $R^{59}$ and $R^{60}$ represent the following ($d^4$), ($e^4$), or ($f^4$) :

($a^4$) $R^{57}$ and $R^{58}$ each independently represent a hydrogen atom or an alkyl group having 1 to 4 carbon atoms;
($b^4$) one of $R^{57}$ and $R^{58}$ represents a hydrogen atom, and the other represents $-(CH_2)_n-COCH_3$ (n represents the same as above), $-(CH_2)_n-CO_2R^{32}$ (n and $R^{32}$ represent the same as above), - $(CH_2)_n-CONR^{33}R^{34}$ (n, $R^{33}$, and $R^{34}$ represent the same as above), $-(CH_2)_m-OR^{35}$ (m and $R^{35}$ represent the same as above), or $-(CH_2)_m-NR^{37}R^{38}$ (m, $R^{37}$, and $R^{38}$ represent the same as above);
($C^4$) $R^{57}$ and $R^{58}$ together represent pyrrolidine, azepane, morpholine, thiazoline, piperidin-2-one, piperidin-4-one, thiamorpholine, piperadine which may be substituted at the 4th position by an alkyl group having 1 to 3 carbon atoms, piperidine which may be substituted at the 4th position by an alkoxy group having 1 to 3 carbon atoms, 4-hydroxypiperidine, or piperidine substituted at the 4th position by an amino group which may be substituted by one or two alkyl groups having 1 to 3 carbon atoms (wherein the pyrrolidine, azepane, morpholine, thiazoline, piperidin-2-one, piperidin-4-one, thiamorpholine, piperadine which may be substituted at the 4th position by an alkyl group having 1 to 3 carbon atoms, piperidine which may be substituted at the 4th position by an alkoxy group having 1 to 3 carbon atoms, 4-hydroxypiperidine, and piperidine substituted at the 4th position by an amino group which may be substituted by one or two alkyl groups having 1 to 3 carbon atoms may be substituted by one or two methyl groups) together with a nitrogen atom;
($d^4$) $R^{59}$ and $R^{60}$ each independently represent a hydrogen atom or an alkyl group having 1 to 4 carbon atoms;
($e^4$) one of $R^{59}$ and $R^{60}$ represents a hydrogen atom, and the other represents $-(CH_2)_n-COCH_3$ (n represents the same as above), - $(CH2)_n-CO^2R^{32}$ (n and $R^{32}$ represent the same as above), - $(CH_2)_n-CONR^{33}R^{34}$ (n, $R^{33}$, and $R^{34}$ represent the same as above), - $(CH_2)_m-OR^{35}$ (m and $R^{35}$ represent the same as above), or - $(CH_2)_m-NR^{37}R^{38}$ (m, $R^{37}$ , and $R^{38}$ represent the same as above); and
($f^4$) $R^{59}$ and $R^{60}$ together represent pyrrolidine, azepane, morpholine, thiazoline, piperidin-2-one, piperidin-4-one, thiamorpholine, piperadine which may be substituted at the 4th position by an alkyl group having 1 to 3 carbon atoms, piperidine which may be substituted at the 4th position by an alkoxy group having 1 to 3 carbon atoms, 4-hydroxypiperidine, or piperidine substituted at the 4th position by an amino group which may be substituted by one or two alkyl groups having 1 to 3 carbon atoms (wherein the pyrrolidine, azepane, morpholine, thiazoline, piperidin-2-one, piperidin-4-one, thiamorpholine, piperadine which may be substituted at the 4th position by an alkyl group having 1 to 3 carbon atoms, piperidine which may be substituted at the 4th position by an alkoxy group having 1 to 3 carbon atoms, 4-hydroxypiperidine, and piperidine substituted at the 4th position by an amino group which may be substituted by one or two alkyl groups having 1 to 3 carbon atoms may be substituted by one or two methyl groups) together with a nitrogen atom].

[15] The prophylactic agent and/or therapeutic agent according to item [6], wherein the CaMKII inhibitor is an isoxazole derivative represented by Formula A4 or a pharmaceutically acceptable salt thereof:
Formula A4:

[Formula 11]

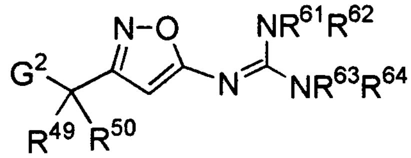

[wherein $G^2$, $R^{49}$, and $R^{50}$ represent the same as in item [14];
Formula: =C ($NR^{61}R^{62}$) $NR^{63}R^{64}$ represents the following ($1^3$), ($2^3$), or ($3^3$) :

($1^3$) $R^{63}$ and $R^{64}$ both represent a hydrogen atom;
$R^{61}$ and $R^{62}$ represent the following ($a^5$), ($b^5$), or ($c^5$) :

($a^5$) $R^{61}$ and $R^{62}$ each independently represent a hydrogen atom or an alkyl group having 1 to 3 carbon atoms;
($b^5$) one of $R^{61}$ and $R^{62}$ represents a hydrogen atom, and the other represents - $(CH_2)$-$CO_2R^{32}$ (n and $R^{32}$ represent the same as in item [6]), -$(CH_2)_m$-$OR^{65}$ (m represents 2 or 3. $R^{65}$ represents a hydrogen atom, an alkyl group having 1 to 3 carbon atoms, 2-hydroxyethyl, or 3-hydroxypropyl), or -$(CH_2)_m$-$NR^{66}R^{67}$ (m represents the same as above; $R^{66}$ and $R^{67}$ each independently represent a hydrogen atom or an alkyl group having 1 to 3 carbon atoms, or together represent pyrrolidine, piperidine, morpholine, or N-methylpiperazine (wherein the pyrrolidine, piperidine, morpholine, and N-methylpiperazine may be substituted by one or two methyl groups) together with a nitrogen atom); and
($C^5$) $R^{61}$ and $R^{62}$ together represent pyrrolidine, piperidine, morpholine, or N-methylpiperazine (wherein the pyrrolidine, piperidine, morpholine, and N-methylpiperazine may be substituted by one or two methyl groups) together with a nitrogen atom;

($2^3$) Formula: =C ($NR^{61}R^{62}$) $NR^{63}R^{64}$ represents a group represented by Formula:

[Formula 12]

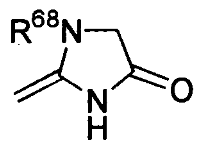

(wherein $R^{68}$ represents an alkyl group having 1 to 3 carbon atoms, 2-hydroxyethyl, or 3-hydroxypropyl); and

($3^3$) $R^{61}$ and $R^{62}$ together represent morpholine together with a nitrogen atom, and $R^{63}$ and $R^{64}$ together represent amino-morpholin-4-yl-methylene].

[16] The prophylactic agent and/or therapeutic agent according to item [15], wherein $G^2$ is 2-fluoro-biphenyl-4-yl or 2'-fluoro-biphenyl-4-yl, $R^{50}$ represents a hydrogen atom or methyl, $R^{63}$ and $R^{64}$ both are a hydrogen atom, and $R^{61}$ and $R^{62}$ represent the following ($a^6$) or ($b^6$) :

($a^6$) $R^{61}$ and $R^{62}$ each independently represent a hydrogen atom or an alkyl group having 1 to 3 carbon atoms; and
($b^6$) $R^{61}$ and $R^{62}$ together represent morpholine (the morpholine may be substituted by one or two methyl groups) together with a nitrogen atom.

[17] The prophylactic agent and/or therapeutic agent according to any of items [1] to [5], wherein the CaMKII inhibitor

is 3-[(1S)-1-(2-fluorobiphenyl-4-yl)ethyl]-5-{[amino(morpholin-4-yl)methylene]amino}isoxazole or a pharmaceutically acceptable salt thereof.

[18] The prophylactic agent and/or therapeutic agent according to any of items [1] to [17], wherein the disease modifying antirheumatic drug is one or several agents selected from the followings:

leflunomide, methotrexate, D-penicillamine, bucillamine, salazosulfapyridine, actarit, lobenzarit, cyclophosphamide, chlorambucil, mizoribine, azathioprine, auranofin, gold sodium thiomaleate, aurothiosulfate, aurothioglucose, cyclosporine, hydroxychloroquine, chlorouquine, tacrolimus, etanercept, infliximab, anakinra, keliximab, adalimumab, rituximab, and a humanized anti-IL-6R antibody.

[19] The prophylactic agent and/or therapeutic agent according to item [18], wherein the disease modifying antirheumatic drug is methotrexate, salazosulfapyridine, or leflunomide.
[20] The prophylactic agent and/or therapeutic agent according to any of items [1] to [19], wherein the autoimmune disease is any disease selected from the followings:

rheumatoid arthritis, systemic lupus erythematosus, discoid lupus erythematosus, polymyositis, scleroderma, mixed connective tissue disease, Hashimoto's disease, primary myxedema, thyrotoxicosis, pernicious anemia, Good-pasture's syndrome, rapidly progressive glomerulonephritis, myasthenia gravis, pemphigus vulgaris, bullous pemphigoid, insulin resistant diabetes, juvenile diabetes, type I diabetes mellitus, Addison's disease, atrophic gastritis, male sterility, climacterium precox, lens-induced uveitis, multiple sclerosis, ulcerative colitis, primary biliary cirrhosis, chronic active hepatitis, autoimmune blood disease, autoimmune hemolytic anemia, idiopathic thrombocytopenia, paroxysmal hemoglobinuria, idiopathic thrombocytopenic purpura, interstitial pulmonary fibrosis, and Sjogren's syndrome.

[21] The prophylactic agent and/or therapeutic agent according to item [20], wherein the autoimmune disease is rheumatoid arthritis.

[22] An enhancer for a prophylactic and/or therapeutic effect of a disease modifying antirheumatic drug on an autoimmune disease, comprising a CaMKII inhibitor as an active ingredient.

[23] The enhancer according to item [22], wherein the CaMKII inhibitor is a substance selected by a screening method comprising the following steps (a), (b), and (c):

(a) bringing a test substance into contact with a CaMKII enzyme and a substrate;
(b) measuring a phosphorylation level of the substrate by the CaMKII enzyme brought into contact with the test substance, and comparing the phosphorylation level with a phosphorylation level of the substrate by a control enzyme kept from contact with the test substance; and
(c) selecting, on the basis of the comparison result of the step (b), a test substance that inhibits CaMKII activity.

[24] The enhancer according to item [23], wherein the CaMKII inhibitor is a substance having a CaMKII inhibition rate of 30% or higher at a concentration of 10 μM.

[25] The enhancer according to item [22], wherein the CaMKII inhibitor is 3-[(1S)-1-(2-fluorobiphenyl-4-yl)ethyl]-5-{[amino(morpholin-4-yl)methylene]amino}isoxazole or a pharmaceutically acceptable salt thereof.
[26] The enhancer according to any of items [22] to [25], wherein the disease modifying antirheumatic drug is methotrexate, salazosulfapyridine, or leflunomide.
[27] The enhancer according to any of items [22] to [26], wherein the autoimmune disease is rheumatoid arthritis.
[28] A first pharmaceutical composition for use in combination with a second pharmaceutical composition to achieve a prophylactic and/or therapeutic effect on an autoimmune disease for a mammal with the autoimmune disease,

wherein the effect exceeds total prophylactic and/or therapeutic effects on the autoimmune disease achieved by separately administering the first and second pharmaceutical compositions, and the second pharmaceutical composition and the first pharmaceutical composition comprise a disease modifying antirheumatic drug and a CaMKII inhibitor, respectively.

[29] A first pharmaceutical composition for use in combination with a second pharmaceutical composition to achieve a prophylactic and/or therapeutic effect on an autoimmune disease for a mammal with the autoimmune disease, wherein the effect exceeds each of a prophylactic and/or therapeutic effect on the autoimmune disease achieved by separately administering the first and second pharmaceutical compositions, and the second pharmaceutical composition and the first pharmaceutical composition comprise a disease modifying antirheumatic drug and a CaMKII inhibitor, respectively.

[30] The composition according to item [28] or [29], wherein the CaMKII inhibitor is 3-[(1S)-1-(2-fluorobiphenyl-4-yl)ethyl]-5-{[amino(morpholin-4-yl)methylene]amino}isoxazole or a pharmaceutically acceptable salt thereof.

[31] The composition according to any of items [28] to [30], wherein the disease modifying antirheumatic drug is methotrexate, salazosulfapyridine, or leflunomide.

[32] The composition according to any of items [28] to [31], wherein the autoimmune disease is rheumatoid arthritis.

[33] A commercial package comprising the following (1) and (2):

(1) a pharmaceutical composition comprising a CaMKII inhibitor; and
(2) a document regarding the pharmaceutical composition indicating that the pharmaceutical composition can be used or should be used in a prophylactic and/or therapeutic application for an autoimmune disease in combination with a disease modifying antirheumatic drug.

[34] A commercial package comprising the following (1) and (2) :

(1) a pharmaceutical composition comprising a CaMKII inhibitor; and
(2) a document regarding the pharmaceutical composition indicating that the pharmaceutical composition can be used or should be used in an application for enhancing a prophylactic and/or therapeutic effect of a disease modifying antirheumatic drug on an autoimmune disease.

[35] The commercial package according to item [33] or [34], wherein the CaMKII inhibitor is 3-[(1S)-1-(2-fluorobiphenyl-4-yl)ethyl]-5-{[amino(morpholin-4-yl)methylene]amino}isoxazole .or a pharmaceutically acceptable salt thereof.

[36] The commercial package according to any of items [33] to [35], wherein the disease modifying antirheumatic drug is methotrexate, salazosulfapyridine, or leflunomide.

[37] The commercial package according to any of items [33] to [36], wherein the autoimmune disease is rheumatoid arthritis.

[38] A kit for prophylaxis and/or treatment of an autoimmune disease comprising a first composition comprising a CaMKII inhibitor and a second composition comprising a disease modifying antirheumatic drug in a package.

[39] Use of a CaMKII inhibitor and a disease modifying antirheumatic drug for manufacturing a pharmaceutical composition for prophylaxis and/or treatment of an autoimmune disease.

[40] Use of a CaMKII inhibitor for manufacturing a prophylactic agent and/or therapeutic agent for an autoimmune disease characterized by being used in combination with a disease modifying antirheumatic drug.

[41] Use of a CaMKII inhibitor for manufacting a pharmaceutical composition for enhancing a prophylactic and/or therapeutic effect of a disease modifying antirheumatic drug on an autoimmune disease.

[42] A method for preventing and/or treating an autoimmune disease, characterized by comprising administering a CaMKII inhibitor in combination with a disease modifying antirheumatic drug.

[43] A method for enhancing a prophylactic and/or therapeutic effect of a disease modifying antirheumatic drug on an autoimmune disease, comprising administering a CaMKII inhibitor.

[44] A method for preventing and/or treating an autoimmune disease, comprising administering a CaMKII inhibitor in an amount effective for enhancing a prophylactic and/or therapeutic effect of a disease modifying antirheumatic drug on the autoimmune disease to a mammal with the autoimmune disease on which the disease modifying antirheumatic drug cannot exert a sufficient prophylactic and/or therapeutic effect.

Alternative aspects of the present invention can include the followings:

[45] A screening method of an enhancer for a prophylactic and/or therapeutic effect of a disease modifying antirheumatic drug on an autoimmune disease, comprising the following steps (a), (b), and (c):

(a) bringing a test substance into contact with a CaMKII enzyme and a substrate;
(b) measuring a phosphorylation level of the substrate by the CaMKII enzyme brought into contact with the test substance, and comparing the phosphorylation level with a phosphorylation level of the substrate by a control enzyme kept from contact with the test substance; and
(c) selecting, on the basis of the comparison result of the step (b), a test substance that inhibits CaMKII activity.

[46] A screening method of a prophylactic agent and/or therapeutic agent for an autoimmune disease used in combination with a disease modifying antirheumatic drug, characterized by comprising the following steps (a), (b), and (c):

(a) bringing a test substance into contact with a CaMKII enzyme and a substrate;
(b) measuring a phosphorylation level of the substrate by the CaMKII enzyme brought into contact with the test substance, and comparing the phosphorylation level with a phosphorylation level of the substrate by a control enzyme kept from contact with the test substance; and
(c) selecting, on the basis of the comparison result of the step (b), a test substance that inhibits CaMKII activity.

Alternative aspects of the present invention can include the followings:

[47] A prophylactic agent and/or therapeutic agent for an autoimmune disease comprising a disease modifying antirheumatic drug, which is intended to be used in combination with a CaMKII inhibitor.
[48] A prophylactic agent and/or therapeutic agent for an autoimmune disease comprising a disease modifying antirheumatic drug, which is intended to be administered simultaneously with, separately from, or subsequent to administration of a CaMKII inhibitor to a mammal with the autoimmune disease to which the CaMKII inhibitor in an amount giving a sufficient preventive and/or therapeutic effect on the autoimmune disease cannot be administered due to a side effect.
[49] The prophylactic agent and/or therapeutic agent according to item [48], wherein the side effect is one or several side effects selected from hepatic disorder, renal disorder, and gastrointestinal disorder.
[50] An enhancer for a prophylactic and/or therapeutic effect of a CaMKII inhibitor on an autoimmune disease, comprising a disease modifying antirheumatic drug as an active ingredient.
[51] A first pharmaceutical composition for use in combination with a second pharmaceutical composition to achieve a prophylactic and/or therapeutic effect on an autoimmune disease for a mammal with the autoimmune disease, wherein the effect exceeds total prophylactic and/or therapeutic effects on the autoimmune disease achieved by separately administering the first and second pharmaceutical compositions, and the second pharmaceutical composition and the first pharmaceutical composition comprise a CaMKII inhibitor and a disease modifying antirheumatic drug, respectively.
[52] A first pharmaceutical composition for use in combination with a second pharmaceutical composition to achieve a prophylactic and/or therapeutic effect on an autoimmune disease for a mammal with the autoimmune disease, wherein the effect exceeds each of a prophylactic and/or therapeutic effect on the autoimmune disease achieved by separately administering the first and second pharmaceutical compositions, and the second pharmaceutical composition and the first pharmaceutical composition comprise a CaMKII inhibitor and a disease modifying antirheumatic drug, respectively.
[53] A commercial package comprising: a pharmaceutical composition comprising a disease modifying antirheumatic drug; and a document regarding the pharmaceutical composition indicating that the pharmaceutical composition can be used or should be used in a prophylactic and/or therapeutic application for an autoimmune disease in combination with a CaMKII inhibitor.
[54] A commercial package comprising: a pharmaceutical composition comprising a disease modifying antirheumatic drug; and a document regarding the pharmaceutical composition indicating that the pharmaceutical composition can be used or should be used in an application for enhancing a prophylactic and/or therapeutic effect of a CaMKII inhibitor on an autoimmune disease.
[55] Use of a disease modifying antirheumatic drug for manufacturing a prophylactic agent and/or therapeutic agent for an autoimmune disease characterized by being used in combination with a CaMKII inhibitor.
[56] Use of a disease modifying antirheumatic drug for manufacturing a pharmaceutical composition for enhancing a prophylactic and/or therapeutic effect of a CaMKII inhibitor on an autoimmune disease.

[57] A method for enhancing a prophylactic and/or therapeutic effect of a CaMKII inhibitor on an autoimmune disease, comprising administering a disease modifying antirheumatic drug.
[58] A method for preventing and/or treating an autoimmune disease, comprising administering a disease modifying antirheumatic drug in an amount effective for enhancing a prophylactic and/or therapeutic effect of a CaMKII inhibitor on the autoimmune disease, to a mammal with the autoimmune disease on which the CaMKII inhibitor cannot exert a sufficient prophylactic and/or therapeutic effect.

[59] The prophylactic agent and/or therapeutic agent, enhancer, composition, commercial package, kit, use, or method according to any of items [22] to [58], wherein the CaMKII inhibitor is an isoxazole derivative represented by Formula 1 according to item [6] or a pharmaceutically acceptable salt thereof.
[60] The prophylactic agent and/or therapeutic agent, enhancer, composition, commercial package, kit, use, or method according to any of items [22] to [59], wherein the CaMKII inhibitor is 3-[(1S)-1-(2-fluorobiphenyl-4-yl)ethyl]-5-{[amino(morpholin-4-yl)methylene]amino}isoxazole or a pharmaceutically acceptable salt thereof.
[61] The prophylactic agent and/or therapeutic agent, enhancer, composition, commercial package, kit, use, or method according to item [60], wherein a dose of the 3-[(1S)-1-(2-fluorobiphenyl-4-yl)ethyl]-5-{[amino(morpholin-4-yl)methylene]amino}isoxazole or the pharmaceutically acceptable salt thereof is approximately 40 to approximately 240 mg/person per day in terms of a free form of 3-[(1S)-1-(2-fluorobiphenyl-4-yl)ethyl]-5-{[amino(morpholin-4-yl)methylene]amino}isoxazole.
[62] The prophylactic agent and/or therapeutic agent, enhancer, composition, commercial package, kit, use, or method according to item [60], wherein a dose of the 3-[(1S)-1-(2-fluorobiphenyl-4-yl)ethyl]-5-{[amino(morpholin-4-yl)methylene]amino}isoxazole or the pharmaceutically acceptable salt thereof is approximately 140 to approximately 240 mg/person per day in terms of a free form of 3-[(1S)-1-(2-fluorobiphenyl-4-yl)ethyl]-5-{[amino(morpholin-4-yl)methylene]amino}isoxazole.
[63] The prophylactic agent and/or therapeutic agent, enhancer, composition, commercial package, kit, use, or method according to any of items [22] to [62], wherein the disease modifying antirheumatic drug is one or several agents selected from the followings:

leflunomide, methotrexate, D-penicillamine, bucillamine, salazosulfapyridine, actarit, lobenzarit, cyclophosphamide, chlorambucil, mizoribine, azathioprine, auranofin, gold sodium thiomaleate, aurothiosulfate, aurothioglucose, cyclosporine, hydroxychloroquine, chlorouquine, tacrolimus, etanercept, infliximab, anakinra, keliximab, adalimumab, rituximab, and a humanized anti-IL-6R antibody.

[64] The prophylactic agent and/or therapeutic agent, enhancer, composition, commercial package, kit, use, or method according to item [63], wherein the disease modifying antirheumatic drug is methotrexate, salazosulfapyridine, or leflunomide.
[65] The prophylactic agent and/or therapeutic agent, enhancer, pharmaceutical composition, composition, commercial package, kit, use, or method according to item [64], wherein a dose of the methotrexate is approximately 5 to approximately 30 mg/person per week.
[66] The prophylactic agent and/or therapeutic agent, enhancer, pharmaceutical composition, composition, commercial package, kit, use, or method according to item [64], wherein a dose of the salazosulfapyridine is approximately 0.5 to approximately 4 g/person per day.
[67] The prophylactic agent and/or therapeutic agent, enhancer, pharmaceutical composition, composition, commercial package, kit, use, or method according to item [64], wherein a dose of the leflunomide is approximately 5 to approximately 30 mg/person per day.
[68] The prophylactic agent and/or therapeutic agent, enhancer, composition, commercial package, kit, use, or method according to any of items [22] to [67], wherein the autoimmune disease is any disease selected from the followings:

rheumatoid arthritis, systemic lupus erythematosus, discoid lupus erythematosus, polymyositis, scleroderma, mixed connective tissue disease, Hashimoto's disease, primary myxedema, thyrotoxicosis, pernicious anemia, Good-pasture's syndrome, rapidly progressive glomerulonephritis, myasthenia gravis, pemphigus vulgaris, bullous pemphigoid, insulin resistant diabetes, juvenile diabetes, type I diabetes mellitus, Addison's disease, atrophic gastritis, male sterility, climacterium precox, lens-induced uveitis, multiple sclerosis, ulcerative colitis, primary biliary cirrhosis, chronic active hepatitis, autoimmune blood disease, autoimmune hemolytic anemia, idiopathic thrombocytopenia, paroxysmal hemoglobinuria, idiopathic thrombocytopenic purpura, interstitial pulmonary fibrosis, and Sjogren's syndrome.

[69] The prophylactic agent and/or therapeutic agent, enhancer, composition, commercial package, kit, use, or method according to item [68], wherein the autoimmune disease is rheumatoid arthritis.

Further aspects of the present invention can include the followings:

[70] A prophylactic agent and/or therapeutic agent for an autoimmune disease comprising a CaMKII inhibitor in combination with a disease modifying antirheumatic drug.
[71] A drug comprising a CaMKII inhibitor in combination with a disease modifying antirheumatic drug.

As described above, the CaMKII inhibitor of the present invention exhibits an excellent inhibitory effect on joint destruction (especially, an inhibitory effect on joint destruction in a mammal with an autoimmune disease) through a combination therapy with disease modifying antirheumatic drugs (DMARDs). Therefore, the aspects of the present invention can also include the followings:

[72] An agent for inhibiting joint destruction comprising a CaMKII inhibitor and a disease modifying antirheumatic drug.
[73] An agent for inhibiting joint destruction comprising a CaMKII inhibitor, which is intended to be used in combination with a disease modifying antirheumatic drug.
[74] The agent for inhibiting joint destruction according to item [73], wherein the agent for inhibiting joint destruction is administered simultaneously with, separately from, or subsequent to administration of the disease modifying antirheumatic drug.
[75] An enhancer for a joint destruction effect of a disease modifying antirheumatic drug, comprising a CaMKII inhibitor as an active ingredient.
[76] A kit for inhibiging joint destruction comprising a first composition comprising a CaMKII inhibitor and a second composition comprising a disease modifying antirheumatic drug in a package.
[77] Use of a CaMKII inhibitor and a disease modifying antirheumatic drug for manufacturing a pharmaceutical composition for inhibiting joint destruction.
[78] Use of a CaMKII inhibitor for manufacturing an agent for inhibiting joint destruction characterized by being used in combination with a disease modifying antirheumatic drug.
[79] Use of a CaMKII inhibitor for manufacturing a pharmaceutical composition for enhancing an inhibitory effect of a disease modifying antirheumatic drug on joint destruction.
[80] A method for inhibiting joint destruction, characterized by comprising administering a CaMKII inhibitor in combination with a disease modifying antirheumatic drug.
[81] A method for enhancing an inhibitory effect of a disease modifying antirheumatic drug on joint destruction, comprising administering a CaMKII inhibitor.
[82] A method for inhibiting joint destruction, comprising administering a CaMKII inhibitor in an amount effective for enhancing an inhibitory effect of a disease modifying antirheumatic drug on the joint destruction, to a mammal with an autoimmune disease on which the disease modifying antirheumatic drug cannot exert a sufficient prophylactic and/or therapeutic effect.

Furthermore, the aspects of the present invention also include aspects in which the "prophylactic agent and/or therapeutic agent for an autoimmune disease" according to items [1] to [71] is replaced with an "agent for inhibiting joint destruction", the "prophylactic and/or therapeutic effect on an autoimmune disease" according to items [1] to [71] is replaced with an "inhibitory effect on joint destruction", the "prophylactic and/or therapeutic application for an autoimmune disease" according to items [1] to [71] is replaced with an "inhibitory application for joint destruction", the phrase "for prophylaxis and/or treatment of an autoimmune disease" according to items [1] to [71] is replaced with a phrase "for inhibiting joint destruction", and the "method for preventing and/or treating an autoimmune disease" according to items [1] to [71] is replaced with a "method for inhibiting joint destruction".

As described above and shown in Examples 1 to 16, the CaMKII inhibitor of the present invention is useful as a prophylactic agent and/or therapeutic agent for an autoimmune disease, even when administered alone. Thus, the aspects of the present invention can include the followings:

[B1] A screening method of a prophylactic agent and/or therapeutic agent for an autoimmune disease, comprising the following steps (a), (b), and (c):

(a) bringing a test substance into contact with a CaMKII enzyme and a substrate;
(b) measuring a phosphorylation level of the substrate by the CaMKII enzyme brought into contact with the test substance, and comparing the phosphorylation level with a phosphorylation level of the substrate by a control enzyme kept from contact with the test substance; and

(c) selecting, on the basis of the comparison result of the step (b), a test substance that inhibits CaMKII activity.

[B2] The screening method according to [B1], wherein the prophylactic agent and/or therapeutic agent for an autoimmune disease is an improving agent for abnormally enhanced immunity.
[B3] The screening method according to [B1], wherein the prophylactic agent and/or therapeutic agent for an autoimmune disease is a chronic inflammation inhibitor, an agent for inhibiting joint destruction, a inhibitor of progression to joint deformity, or an improving agent for physical function for a patient with rheumatoid arthritis.
**[B4]** The screening method according to any of [B1] to [B3], wherein the screening method comprises the step of selecting a substance having a CaMKII inhibition rate of 30% or higher at a concentration of 10 μM .
[B5] The screening method according to any of [B1] to [B3], wherein the screening method comprises the step of selecting a substance having a CaMKII inhibition rate of 60% or higher at a concentration of 10 μM.
[B6] A prophylactic agent and/or therapeutic agent for an autoimmune disease comprising, as an active ingredient, a substance selected by use of a screening method according to any of [B1] to [B5].

[B7] A prophylactic agent and/or therapeutic agent for an autoimmune disease comprising, as an active ingredient, a substance having a CaMKII inhibitory effect.
[B8] The prophylactic agent and/or therapeutic agent for an autoimmune disease according to [B7], wherein the substance having a CaMKII inhibitory effect is a substance having a CaMKII inhibition rate of 30% or higher at a concentration of 10 μM.
[B9] The prophylactic agent and/or therapeutic agent for an autoimmune disease according to [B7], wherein the substance having a CaMKII inhibitory effect is a substance having a CaMKII inhibition rate of 60% or higher at a concentration of 10 μM.
[B10] The prophylactic agent and/or therapeutic agent for an autoimmune disease according to any of [B6] to [B9], wherein the prophylactic agent and/or therapeutic agent is a prophylactic agent and/or therapeutic agent for rheumatoid arthritis.
[B11] The prophylactic agent and/or therapeutic agent for an autoimmune disease according to any of [B6] to [B9], wherein the prophylactic agent and/or therapeutic agent is intended to inhibit chronic inflammation, inhibit joint destruction, inhibit progression to joint deformity, or improve physical function in a patient with rheumatoid arthritis.

[B12] A CaMKII inhibitor comprising, as an active ingredient, an isoxazole derivative represented by Formula 1 according to [6] or a pharmaceutically acceptable salt thereof.

[B13] The CaMKII inhibitor according to [B12], wherein E is a single bond or a lower alkylene group. **[B14]** The CaMKII inhibitor according to [B12] or
[B13], wherein D is a hydrogen atom, a nitro group, a cyano group, a carboxy group, an amino group which may be substituted, a hydroxylamino group which may be substituted, a carbamoyl group which may be substituted, $-R^5$, or $-CO_2R^6$ (wherein $R^5$ and $R^6$ represent the same as in [B12]).
[B15] The CaMKII inhibitor according to [B14], wherein D is a hydrogen atom, a carboxy group, $-R^5$, or $-CO_2R^6$ (wherein $R^5$ and $R^6$ represent the same as in [B12]).

[B16] The CaMKII inhibitor according to any of [B12] to [B15], wherein $R^1$, $R^2$, $R^3$, and $R^4$ each independently represent a hydrogen atom, a hydroxy group, an amino group which may be substituted, a hydroxylamino group which may be substituted, an alkoxy group which may be substituted, an alkyl group which may be substituted, an alkenyl group which may be substituted, an alkynyl group which may be substituted, a cycloalkyl group which may be substituted, a cycloalkenyl group which may be substituted, an aryl group which may be substituted, a heterocyclic group which may be substituted, or an acyl group; or Formula: $-NR^3R^4$ represents a group represented by Formula: $-N=C(NH_2)NR^{43}R^{44}$ ($R^{43}$ and $R^{44}$ represent the same as in [B12]); or any two of $R^1$, $R^2$, $R^3$, and $R^4$ bind together and form, together with a nitrogen atom, a heterocyclic ring which may be substituted.
[B17] The CaMKII inhibitor according to [B16], wherein $R^1$, $R^2$, $R^3$, and $R^4$ each independently represent a hydrogen atom, a hydroxy group, an amino group which may be substituted, a hydroxylamino group which may be substituted,

an alkoxy group which may be substituted, an alkyl group which may be substituted, an alkenyl group which may be substituted, an alkynyl group which may be substituted, a cycloalkyl group which may be substituted, an aryl group which may be substituted, a heterocyclic group which may be substituted, or an acyl group; or Formula: $-NR^3R^4$ represents a group represented by Formula: $-N=C(NH_2)NR^{43}R^{44}$ ($R^{43}$ and $R^{44}$ represent the same as in [B12]); or any two of $R^1$, $R^2$, $R^3$, and $R^4$ bind together and form, together with a nitrogen atom, a heterocyclic ring which may be substituted.

[B18] The CaMKII inhibitor according to any of [B12] to [B17], wherein G is phenyl which may be substituted, naphthyl which may be substituted, furyl which may be substituted, thienyl which may be substituted, indolyl which may be substituted, isothiazolyl which may be substituted, benzothienyl which may be substituted, isobenzofuranyl which may be substituted, pyrrolyl which may be substituted, benzofuryl which may be substituted, imidazolyl which may be substituted, pyrazolyl which may be substituted, isoxazolyl which may be substituted, isothiazolyl which may be substituted, thiazolyl which may be substituted, oxazolyl which may be substituted, benzimidazolyl which may be substituted, benzothiazolyl which may be substituted, benzoxazolyl which may be substituted, pyridyl which may be substituted, pyrazinyl which may be substituted, pyrimidinyl which may be substituted, pyridazinyl which may be substituted, triazinyl which may be substituted, quinolyl which may be substituted, isoquinolyl which may be substituted, quinazolinyl which may be substituted, quinoxalinyl which may be substituted, 2,3-dihydro-benzo[1,4]dioxinyl which may be substituted, or carbazolyl which may be substituted.

[B19] The CaMKII inhibitor according to [B12], wherein the CaMKII inhibitor comprises, as an active ingredient, an isoxazole derivative represented by Formula A1 or A2 according to [13] or a pharmaceutically acceptable salt thereof.

[B20] The CaMKII inhibitor according to [B12], wherein the CaMKII inhibitor comprises, as an active ingredient, an isoxazole derivative represented by Formula A3 according to [14] or a pharmaceutically acceptable salt thereof.

[B21] The CaMKII inhibitor according to [B12], wherein the CaMKII inhibitor comprises, as an active ingredient, an isoxazole derivative represented by Formula A4 according to [15] or a pharmaceutically acceptable salt thereof.

[B22] The CaMKII inhibitor according to [B21], wherein $G^2$ is 2-fluoro-biphenyl-4-yl or 2'-fluoro-biphenyl-4-yl, $R^{50}$ is a hydrogen atom or methyl, $R^{63}$ and $R^{64}$ both are a hydrogen atom, and $R^{61}$ and $R^{62}$ represent the following ($a^6$) or ($b^6$) :

($a^6$) $R^{61}$ and $R^{62}$ each independently represent a hydrogen atom or an alkyl group having 1 to 3 carbon atoms; and
($b^6$) $R^{61}$ and $R^{62}$ together represent morpholine (the morpholine may be substituted by one or two methyl groups) together with a nitrogen atom.

[B23] A CaMKII inhibitor comprising 3-[(1S)-1-(2-fluorobiphenyl-4-yl)ethyl]-5-{[amino(morpholin-4-yl)methylene] amino}isoxazole or a pharmaceutically acceptable salt thereof as an active ingredient.
[B24] A CaMKII inhibitor comprising 4-[1-(2-fluorobiphenyl-4-yl)ethyl]-2-(methylamino)thiazole or a pharmaceutically acceptable salt thereof as an active ingredient.
[B25] A prophylactic agent and/or therapeutic agent for an autoimmune disease comprising, as an active ingredient, a substance having an inhibitory effect on $I\kappa B\alpha$ degradation.
[B26] A prophylactic agent and/or therapeutic agent for an autoimmune disease comprising, as an active ingredient, a substance having an inhibitory effect on enhanced expression of cyclin D1.
[B27] A prophylactic agent and/or therapeutic agent for an autoimmune disease comprising, as an active ingredient, a substance having an inhibitory effect on Akt phosphorylation.

[B28] The prophylactic agent and/or therapeutic agent for an autoimmune disease according to any of [B7] to [B11], wherein the substance having a CaMKII inhibitory effect is a polynucleotide having at least 15 consecutive bases in a nucleotide sequence of a CaMKII gene and/or a polynucleotide complementary to the polynucleotide.
[B29] The prophylactic agent and/or therapeutic agent for an autoimmune disease according to any of [B7] to [B11], wherein the substance having a CaMKII inhibitory effect is siRNA against a CaMKII gene.
[B30] The prophylactic agent and/or therapeutic agent for an autoimmune disease according to [B29], wherein the siRNA is siRNA represented by the following sequence:

sense strand: 5'-CGU GAG GCU CGG AUA UGU CdTdT-3'; and
antisense strand: 5'-GAC AUA UCC GAG CCU CAC GdTdT-3'.

[B31] The prophylactic agent and/or therapeutic agent for an autoimmune disease according to any of [B7] to [B11], wherein the substance having a CaMKII inhibitory effect is an anti-CaMKII antibody.

[B32] A method for preventing and/or treating an autoimmune disease in a mammal, comprising inhibiting CaMKII expression or activity in the mammal to an extent effective for preventing and/or treating the autoimmune disease.
[B33] A method for preventing and/or treating an autoimmune disease in a mammal, comprising administering, to the mammal, a substance having a CaMKII inhibitory effect in an amount effective for preventing and/or treating the autoimmune disease. [B34] Use of a substance having a CaMKII inhibitory effect for manufacturing a prophylactic agent and/or therapeutic agent for an autoimmune disease.

Alternative aspects of the present invention can include the followings:

[B35] A screening method of a prophylactic agent and/or therapeutic agent for an autoimmune disease, comprising the following steps (a), (b), and (c):

(a) bringing a test substance into contact with a CaMKII expressing cell;
(b) measuring a CaMKII protein level in the CaMKII expressing cell brought into contact with the test substance, and comparing the protein level with a CaMKII protein level in a control cell kept from contact with the test substance; and
(c) selecting, on the basis of the comparison result of the step (b), a test substance that inhibits CaMKII production.

[B36] A screening method of a prophylactic agent and/or therapeutic agent for an autoimmune disease, comprising the following steps (a), (b), and (c):

(a) bringing a test substance into contact with a CaMKII expressing cell;
(b) measuring a CaMKII gene expression level in the CaMKII expressing cell brought into contact with the test substance, and comparing the gene expression level with a CaMKII gene expression level in a control cell kept from contact with the test substance; and
(c) selecting, on the basis of the comparison result of the step (b), a test substance that inhibits CaMKII gene expression.

[B37] A screening method of a prophylactic agent and/or therapeutic agent for an autoimmune disease, comprising the following steps (a), (b), and (c):

(a) bringing a test substance into contact with a dephosphorylated CaMKII enzyme;
(b) measuring a phosphorylation level of the CaMKII enzyme brought into contact with the test substance, and comparing the phosphorylation level with a phosphorylation level of a control enzyme kept from contact with the test substance; and
(c) selecting, on the basis of the comparison result of the step (b), a test substance that inhibits CaMKII activity.

Preferable aspects of the present invention can include the followings:

[B38] The screening method according to any of [B1] to [B5], wherein the CaMKII is CaMKIIγ or CaMKIIδ.

[B39] The screening method according to any of [B1] to [B5], wherein the CaMKII is CaMKIIγ.
[B40] The prophylactic agent and/or therapeutic agent for an autoimmune disease according to any of [B6] to [B11], wherein the CaMKII is CaMKIIγ or CaMKIIδ. [B41] The prophylactic agent and/or therapeutic agent for an autoimmune disease according to any of [B6] to [B11], wherein the CaMKII is CaMKIIγ.
[B42] The prophylaxis and/or treatment method according to [B32] or [B33], wherein the CaMKII is CaMKIIγ or CaMKIIδ.
[B43] The prophylaxis and/or treatment method according to [B32] or [B33], wherein the CaMKII is CaMKIIγ.
[B44] The use according to [B34], wherein the CaMKII is CaMKIIγ or CaMKIIδ.
[B45] The use according to [B34], wherein the CaMKII is CaMKIIγ.

Alternative aspects of the present invention can include the followings:

[B46] A prophylactic agent and/or therapeutic agent for an autoimmune disease comprising, as an active ingredient, a substance selected by use of a screening method according to any of [B1] to [B5] or [B35] to [B39] except for the following (1) and (2):

(1) an isoxazole derivative represented by Formula 1 according to [B12] or a pharmaceutically acceptable salt thereof; and
(2) 4-[1-(2-fluorobiphenyl-4-yl)ethyl]-2-(methylamino)thiazole or a pharmaceutically acceptable salt thereof.

[B47] A prophylactic agent and/or therapeutic agent for an autoimmune disease comprising, as an active ingredient, a substance having a CaMKII inhibitory effect except for the following (1) and (2):

(1) an isoxazole derivative represented by Formula 1 according to [B12] or a pharmaceutically acceptable salt thereof; and
(2) 4-[1-(2-fluorobiphenyl-4-yl)ethyl]-2-(methylamino)thiazole or a pharmaceutically acceptable salt thereof.

[B48] The prophylactic agent and/or therapeutic agent for an autoimmune disease according to [B47], wherein the prophylactic agent and/or therapeutic agent is a prophylactic agent and/or therapeutic agent for rheumatoid arthritis.
[B49] An improving agent for abnormally enhanced immunity comprising a CaMKII inhibitor according to any of [B12] to [B24] as an active ingredient.
[B50] A chronic inflammation inhibitor, an agent for inhibiting joint destruction, an inhibitor of progression to joint deformity, or an improving agent for physical function for a patient with rheumatoid arthritis comprising a CaMKII inhibitor according to any of [B12] to [B24] as an active ingredient.
[B51] The screening method according to [B2], wherein the autoimmune disease is rheumatoid arthritis, systemic lupus erythematosus, discoid lupus erythematosus, polymyositis, scleroderma, mixed connective tissue disease, Hashimoto thyroiditis, primary myxedema, thyrotoxicosis, pernicious anemia, Good-pasture's syndrome, rapidly progressive glomerulonephritis, myasthenia gravis, pemphigus vulgaris, bullous pemphigoid, insulin resistant diabetes, juvenile diabetes, Addison's disease, atrophic gastritis, male sterility, climacterium precox, lens-induced uveitis, polyarteritis nodosa, multiple sclerosis, ulcerative colitis, primary biliary cirrhosis, chronic active hepatitis, autoimmune hemolytic anemia, paroxysmal hemoglobinuria, idiopathic thrombocytopenic purpura, or Sjogren's syndrome.
[B52] The screening method according to [B2], wherein the autoimmune disease is rheumatoid arthritis or multiple sclerosis.

The present invention can provide an excellent drug, particularly an excellent drug for a combination therapy, for preventing and/or treating an autoimmune disease. The present invention can further provide a screening method useful for searching and obtaining a candidate substance serving as an active ingredient in the prophylactic drug, therapeutic drug, or drug for a combination therapy.

BRIEF DESCRIPTION OF THE DRAWINGS

Fig. 1 is a graph showing the amount of IL-2 production when EL-4 cells (mouse T-lymphocyte cell line) were stimulated with Phorbol 12-myristate 13-acetate (PMA) and Calcium Ionophore A23187. The results are shown by the average and the standard deviation of six wells for each group. The results of Compound (I) added group (1 to 30 μM), KN-93 added group (1 to 10 μM) and control group are shown.

Fig. 2 is a graph showing the amount of IL-6 production when J774A.1 cells (mouse macrophage cell line) were stimulated with lipopolysaccharide (LPS). The results are shown by the average and the standard deviation of six wells for each group. The results of Compound (I) added group (1 to 30 μM), KN-93 added group (1 to 3 μM) and control group are shown.

Fig. 3 is a graph showing the cell proliferation when L929 cells (mouse fibroblast cell line) were stimulated with recombinant human FGF basic (bFGF). Uptake of bromodeoxyuridine (Brd-U) as an index of cell proliferation was measured as absorbance at wavelength of 450 nm. The results are shown by the average and the standard deviation of six wells for each group. ▲ shows the results of Compound (I) added group (0.3 to 10 μM). ■ shows the results of KN-93 added group (1 to 30 μM). ○ shows the results of unstimulated control group. ● shows the results of stimulated control group.

Fig. 4 is a graph showing the proliferation of SW982 cells (human synovial membrane derived cells). The results are shown by the average and the standard deviation of six wells for each group. ▲ shows the results of Compound (I) added group (1 to 30 μM). ■ shows the results of KN-93 added group (1 to 30 μM). ● shows the results of control group.

Fig. 5 is a graph showing the number of the formation of osteoclasts when the cells derived from arthritic joint of collagen-induced arthritis mouse were cultured. The results are shown by the average and the standard deviation of six wells for each group. The results of Compound (I) added group (0.5 to 20 μM), KN-93 added group (0.3 to 5 μM) and control group are shown.

Fig. 6 is a graph showing the change of edema volume of the left hind paw in adjuvant-induced arthritis rats. The results are shown by the average and the standard deviation of 10 examples for each group. The results of Compound (I) administered group (2.5 to 80 mg/kg), Indomethacin administered group (0.5 mg/kg) and control group are shown.

Fig. 7 is a graph showing the change of edema volume of the right and left hind paws in adjuvant-induced arthritis rats. The results of Compound (II) administered group (10 to 50 mg/kg) (the average and standard deviation of 7 examples for each group) and control group (the average and standard deviation of 8 examples) are shown.

Fig. 8 is a graph showing the daily change of the volume of the right and left hind paws in adjuvant-induced arthritis rats. In the graph, ○ shows the results of Compound (I) (2.5 mg/kg) administered group. ■ shows the results of Compound (I) (5 mg/kg) administered group. □ shows the results of Compound (I) (10 mg/kg) administered group. ▲ shows the results of Compound (I) (20 mg/kg) administered group. Δ shows the results of Compound (I) (40 mg/kg) administered group. ● shows the results of Compound (I) (80 mg/kg) administered group. ◇ shows the results of Indomethacin (0.5 mg/kg) administered group. × shows the results of test compound non-administered group (control group). + the results of normal control group. Each group is shown by the average of 10 examples. Statistical analysis was performed using the AUC value. In Williams's multiple comparison of one-sided test, right hind paw volume ($p<0.01$) and left hind paw volume ($p<0.05$) are significant in Compound (I) (20 mg/kg) administered group, and both the right and left hind paw volumes are significant ($p<0.01$) in Compound (I)(40 mg/kg) administered group and Compound (I) (80 mg/kg) administered group. In Student's t-test of two-sided test, both the right and left hind paw volumes are significant ($p<0.01$) in Indomethacin (0.5 mg/kg) administered group.

Fig. 9 is a graph showing the daily change of the edema volume of the right and left hind paws in adjuvant-induced arthritis rats. In the graph, ● shows the results of Compound (II) (50 mg/kg) administered group. Δ shows the results of D-penicillamine (50 mg/kg) administered group. ○ shows the results of test compound non-administered group (control group). Each group is shown by the average of 10 to 12 examples.

Fig. 10 is a graph showing arthritis onset ratio when Compound (I) was tested using the collagen-induced arthritis mouse which was an animal model of rheumatoid arthritis. ○ shows the results of Compound (I) (5 mg/kg) administered group. Δ shows the results of Compound (I) (10 mg/kg) administered group. □ shows the results of Compound (I) (20 mg/kg) administered group. ▲ shows the results of Compound (I) (40 mg/kg) administered group. ■ shows the results of Compound (I) (80 mg/kg) administered group. × shows the results of prednisolone (3 mg/kg) administered group. ● shows the results of test compound non-administered group (control group). Each group is shown by the average of 10 examples. In the frequency test by Fischer's direct probability method with Bonferroni's correction, each of Compound (I) (5 mg/kg) administered group ($p<0.05$) and Compound (I) (10, 20, 40 and 80 mg/kg) administered groups ($p<0.01$) showed a significant difference. In addition, in frequency test by Fischer's direct probability method, prednisolone (3 mg/kg) administered group showed a significant difference ($p<0.01$).

Fig. 11 is a graph showing arthritis score when Compound (I) was tested using the collagen-induced arthritis mouse which was an animal model of rheumatoid arthritis. ○ shows the results of Compound (I) (5 mg/kg) administered

group. Δ shows the results of Compound (I) (10 mg/kg) administered group. □ shows the results of Compound (I) (20 mg/kg) administered group. ▲ shows the results of Compound (I) (40 mg/kg) administered group. ■ shows the results of Compound (I) (80 mg/kg) administered group. × shows the results of Prednisolone (3 mg/kg) administered group. ● shows the results of test compound non-administered group (control group). Each group is shown by the average of 10 examples. In Shirley-Williams multiple comparison test, each of Compound (I) (5 mg/kg) administered group ($p<0.05$) and Compound (I) (10, 20, 40 and 80 mg/kg) administered groups ($p<0.01$) showed a significant difference. In Wilcoxon's test, Prednisolone (3 mg/kg) administered group showed a significant difference ($p<0.01$).

Fig. 12 is a graph showing the disease score when KN-93 was tested using the experimental allergic encephalomyelitis mouse which was an animal model of multiple sclerosis. * shows the results of KN-93 (1 mg/kg) administered group. ● shows the results of test compound non-administered group (control group). Each group is shown by the average of 10 examples.

Fig. 13 is a graph showing the disease condition score when Compound (I) was tested using the experimental allergic encephalomyelitis mouse which was an animal model of multiple sclerosis. □ shows the results of Compound (I) (20 mg/kg) administered group. ▲ shows the results of Compound (I) (40 mg/kg) administered group. ■ shows the results of Compound (I) (80 mg/kg) administered group. × shows the results of Prednisolone (3 mg/kg) administered group. ● shows the results of test compound non-administered group (control group). + shows the results of normal control group. The control group is shown by the average of 9 examples, each of the other groups is shown by the average of 10 examples.

Fig. 14 is a graph showing the effect on the weight gain when Compound (I) was tested using the experimental allergic encephalomyelitis mouse which was an animal model of multiple sclerosis. □ shows the results of Compound (I) (20 mg/kg) administered group.▲ shows the results of Compound (I) (40 mg/kg) administered group. ■ shows the results of Compound (I) (80 mg/kg) administered group. × shows the results of prednisolone (3 mg/kg) administered group. ● shows the results of test compound non-administered group (control group). + shows the results of normal control group. The control group is shown by the average of 9 examples, each of the other groups is shown by the average of 10 examples.

Fig. 15 is a result of immunostaining of CaMKII at a synovial membrane site of a CIA mouse. Cells appearing black show CaMKII expressing cells.

Fig. 16 is a result of immunostaining of CaMKII in pannus (granulation tissue) of a CIA mouse. Cells appearing black show CaMKII expressing cells.

Fig. 17 is the result detected by Western blotting showing expression of IκBα in the cytoplasm when EL-4 cells (mouse T-lymphocyte cell line) were stimulated with Phorbol 12-myristate 13-acetate (PMA) and Calcium Ionophore A23187. The results of the addition of 30 μM of Compound (I), 10 μM of KN-93 and control are shown.

Fig. 18 is the result detected by Western blotting showing expression of cyclin D1 in the cell when L929 cells (mouse fibroblast cell line) were stimulated with recombinant human FGF basic (bFGF). The results of the addition of Compound (I) (1.25 to 20 μM), KN-93 (5 to 20 μM), no stimulation control and stimulation control are shown.

Fig. 19 is the result detected by Western blotting showing phosphorylated Akt and expression of Akt in the cell when L929 cells (mouse fibroblast cell line) were stimulated with recombinant human FGF basic (bFGF). The results of the addition of Compound (I) (20 μM), KN-93 (20 μM), no stimulation control and stimulation control are shown.

Fig. 20 is the result detected by Western blotting showing expressions of CaMKII γ and CaMKII δ in the cell when THP-1 cells (human monocyte cell line) were stimulated with LPS + IFN γ.

Fig. 21 shows the result of flow cytometeric analysis for the case without transfection as control when the optimal introduction condition was examined using fluorescently labeled siRNA. The vertical axis represents cell counts and the horizontal axis represents fluorescence intensity on a Log scale.

Fig. 22 shows the result of flow cytometer analysis for the optimal condition (siRNA 60 pmol) when the optimal introduction condition was examined using fluorescently labeled siRNA. The vertical axis represents cell counts and the horizontal axis represents fluorescence intensity on a Log scale.

Fig. 23 is the result detected by Western blotting showing expression of CaMKII γ when CaMKII γ siRNA (A), (B) and (C) were respectively introduced into SW982 cells (human synovial membrane derived cell). (A), (B) and (C) in the drawing show the result of the cases where CaMKII γ siRNA (A), (B) and (C) were respectively introduced.

SC shows the result of the case where Scramble II Duplex (SC) was introduced as control. Mock shows the result of the case where transfection was performed only with introduction reagents containing no siRNA. The band indicated by "CaMKII γ→" in the drawing is a band by CaMKII γ.

Fig. 24 is a graph showing the cell proliferation when CaMKII γ siRNA (A), (B) and (C) were respectively introduced into SW982 cells (human synovial membrane derived cell). As an index of cell proliferation, fluorescence intensity (excited at 544 nm, wavelength: 590 nm) was measured after Alamar Blue(R) was added. (A), (B) and (C) in the drawing show the result of the cases where CaMKII γ siRNA (A), (B) and (C) were respectively introduced. SC shows the result of the case where Scramble II Duplex (SC) was introduced as control. Mock shows the result of the case where transfection was performed only with introduction reagents containing no siRNA.

Fig. 25 is a graph showing the change of edema volume of the left hind leg in adjuvant-induced arthritis rat. In the graphs, A shows the result of the group in which Compound (I) (10 mg/kg) and leflunomide (5 mg/kg) were administered in combination. B shows the result of the group in which only Compound (I) (10 mg/kg) was administered. C shows the result of the group in which only leflunomide (5 mg/kg) was administered. D shows the results of test compound non-administered group (control group). Each group is shown by the average of 7 examples. The percentage in the graph shows edema suppression ratio.

Fig. 26 is a graph showing the change of edema volume of the left hind paw in adjuvant-induced arthritis rats. In the graphs, A shows the result of the group in which Compound (I) (10 mg/kg) and salazosulfapyridine (400 m/kg) were administered in combination. B shows the result of the group in which only Compound (I) (10 mg/kg) was administered. C shows the result of the group in which only salazosulfapyridine (400 mg/kg) was administered. D shows the result of test compound non-administered group (control group). Each group is shown by the average of 7 examples. The percentage in the graph shows edema suppression ratio.

Fig. 27 is a graph showing the daily change of the volume of the left hind paw in adjuvant-induced arthritis rats. In the graph, ◇ shows the result of the group in which Compound (I) (10 mg/kg) and methotrexate (0.060 mg/kg) were administered in combination. ○ shows the result of the group in which only Compound (I) (10 mg/kg) was administered. ▲ shows the result of the group in which only methotrexate (0.060 mg/kg) was administered. ■ show the result of test compound non-administered group (control group). □ shows the result of the group of normal animal (normal control group) in which adjuvant-induced arthritis was not caused. Each group is shown by the average of 8 examples.

Fig. 28 is a graph showing the daily change of the onset ratio in adjuvant-induced arthritis rats. In the graph, ◇ shows the result of the group in which Compound (I) (10 mg/kg) and methotrexate (0.060 mg/kg) were administered in combination. ○ shows the result of the group in which only Compound (I) (10 mg/kg) was administered. ▲ shows the result of the group in which only methotrexate (0.060 mg/kg) was administered. ■ shows the result of test compound non-administered group (control group). Each group is shown by the average of 8 examples.

Fig. 29 is a graph showing the daily change of the volume of the left hind paw in adjuvant-induced arthritis rats. In the graph, ◇ shows the result of the group in which Compound (I) (10 mg/kg) and methotrexate (0.045 mg/kg) were administered in combination. ○ shows the result of the group in which only Compound (I) (10 mg/kg) was administered. ▲ shows the result of the group in which only methotrexate (0.045 mg/kg) was administered. ■ shows the result of test compound non-administered group (control group). □ shows the result of normal control group. Each group is shown by the average of 8 examples.

Fig. 30 is a graph showing the daily change of the onset ratio in adjuvant-induced arthritis rats. In the graph, ◇ shows the result of the group in which Compound (I) (10 mg/kg) and methotrexate (0.045 mg/kg) were administered in combination. O shows the result of the group in which only Compound (I) (10 mg/kg) was administered. ▲ shows the result of the group in which only methotrexate (0.045 mg/kg) was administered. ■ shows the result of test compound non-administered group (control group). Each group is shown by the average of 8 examples.

Fig. 31 is a graph showing the daily change of the increased volume (difference between the volume at the time point and the volume at the time of grouping) of the left hind paw in adjuvant-induced arthritis rat. In the graph, ♦ shows the result of the group in which Compound (I) (20 mg/kg) and methotrexate (0.06 mg/kg) were administered in combination. ○ shows the result of the group in which only Compound (I) (20 mg/kg) was administered. Δ shows the result of the group in which only methotrexate (0.06 mg/kg) was administered. ■ shows the result of test

compound non-administered group (control group). □ shows the result of normal control group. Each group is shown by the average of 10 examples.

Fig. 32 is a graph showing the daily change of the onset ratio in adjuvant-induced arthritis rats. In the graph, ♦ shows the result of the group in which Compound (I) (20 mg/kg) and methotrexate (0.06 mg/kg) were administered in combination. ○ shows the result of the group in which only Compound (I) (20 mg/kg) was administered. Δ shows the result of the group in which only methotrexate (0.06 mg/kg) was administered. ■ shows the result of test compound non-administered group (control group). □ shows the result of normal control group. Each group is shown by the average of 10 examples.

Fig. 33 is a graph showing the daily change of the increased volume (difference between the volume at the time point and the volume at the time of grouping) of the left hind paw in adjuvant-induced arthritis rats. In the graph, ♦ shows the result of the group in which Compound (I) (40 mg/kg) and methotrexate (0.06 mg/kg) were administered in combination. ○ shows the result of the group in which only Compound (I) (40 mg/kg) was administered. Δ shows the result of the group in which only methotrexate (0.06 mg/kg) was administered. ■ shows the result of test compound non-administered group (control group). □ shows the result of normal control group. Each group is shown by the average of 10 examples.

Fig. 34 is a graph showing the daily change of the onset ratio in adjuvant-induced arthritis rats. In the graph, ♦ shows the result of the group in which Compound (I) (40 mg/kg) and methotrexate (0.06 mg/kg) were administered in combination. O shows the result of the group in which only Compound (I) (40 mg/kg) was administered. Δ shows the result of the group in which only methotrexate (0.06 mg/kg) was administered. ■ shows the result of test compound non-administered group (control group). □ shows the result of normal control group. Each group is shown by the average of 10 examples.

Fig. 35 is a graph showing the daily change of the increased volume (difference between the volume at the time point and the volume at the time of grouping) of the left hind paw in adjuvant-induced arthritis rats. In the graph, ♦ shows the result of the group in which Compound (I) (80 mg/kg) and methotrexate (0.06 mg/kg) were administered in combination. ○ shows the result of the group in which only Compound (I) (80 mg/kg) was administered. Δ shows the result of the group in which only methotrexate (0.06 mg/kg) was administered. ■ shows the result of test compound non-administered group (control group). □ shows the result of normal control group. Each group is shown by the average of 10 examples.

Fig. 36 is a graph showing the daily change of the onset ratio in adjuvant-induced arthritis rats. In the graph, ♦ shows the result of the group in which Compound (I) (80 mg/kg) and methotrexate (0.06 mg/kg) were administered in combination. O shows the result of the group in which only Compound (I) (80 mg/kg) was administered. Δ shows the result of the group in which only methotrexate (0.06 mg/kg) was administered. ■ shows the result of test compound non-administered group (control group). □ shows the result of normal control group. Each group is shown by the average of 10 examples.

Fig. 37 is a graph showing AUC (area under the curve formed by plotting the increased volume at each time point during the administration period against the time) of the left hind paw in adjuvant-induced arthritis rats. In the graph, ♦ shows the result of the groups in which Compound (I) (20 mg/kg, 40 mg/kg, 80 mg/kg) and methotrexate (0.06 mg/kg) were administered in combination. ○ shows the result of the group in which only Compound (I) was administered. Δ shows the result of the group in which only methotrexate (0.06 mg/kg) was administered. ■ shows the result of test compound non-administered group (control group). □ shows the result of normal control group.

Fig. 38 is a graph showing the joint destruction score of the right hind paw in adjuvant-induced arthritis rats. The results are shown by the average and the standard deviation of 10 examples for each group. The results of the normal control group, the test compound non-administered group (control group), the group in which only methotrexate (0.06 mg/kg) was administered, the groups in which Compound (I) (20 mg/kg, 40 mg/kg, 80 mg/kg) and methotrexate (0.06 mg/kg) were administered in combination, and the groups in which only Compound (I) (20 mg/kg, 40 mg/kg, 80 mg/kg) was administered are shown.

Fig. 39 is a soft X-ray photograph image of the right hind paw of an adjuvant-induced arthritis rat. The photograph is a representative example of the individual which showed an average score in the control group (score 10).

Fig. 40 is a soft X-ray photograph image of the right hind paw of an adjuvant-induced arthritis rat. The photograph is a representative example of the individual which showed an average score in the group in which only methotrexate (0.06 mg/kg) was administered (score 9).

Fig. 41 is a soft X-ray photograph image of the right hind paw of an adjuvant-induced arthritis rat. The photograph is a representative example of the individual which showed an average score in the group in which only Compound (I) (40 mg/kg) was administered (score 5).

Fig. 42 is a soft X-ray photograph image of the right hind paw of an adjuvant-induced arthritis rat. The photograph

is a representative example of the individual which showed an average score in the group in which Compound (I) (40 mg/kg) + methotrexate (0.06 mg/kg) were administered in combination (score 2).

Fig. 43 is a graph showing the joint destruction score of the left hind paw in adjuvant-induced arthritis rats. The results are shown by the average and the standard deviation of 10 examples for each group. The results of the normal control group, the test compound non-administered group (control group), the group in which only methotrexate (0.06 mg/kg) was administered, the groups in which Compound (I) (20 mg/kg, 40 mg/kg, 80 mg/kg) and methotrexate (0.06 mg/kg) were administered in combination, and the groups in which only Compound (I) (20 mg/kg, 40 mg/kg, 80 mg/kg) was administered are shown.
Fig. 44 is a soft X-ray photograph image of the left hind paw of an adjuvant-induced arthritis rat. The photograph is a representative example of the individual which showed an average score in the control group (score 3).
Fig. 45 is a soft X-ray photograph image of the left hind paw of an adjuvant-induced arthritis rat. The photograph is a representative example of the individual which showed an average score in the group in which only methotrexate (0.06 mg/kg) was administered (score 2).
Fig. 46 is a soft X-ray photograph image of the left hind paw of an adjuvant-induced arthritis rat. The photograph is a representative example of the individual which showed an average score in the group in which only Compound (I) (40 mg/kg) was administered (score 1).
Fig. 47 is a soft X-ray photograph image of the left hind paw of an adjuvant-induced arthritis rat. The photograph is a representative example of the individual which showed an average score in the group in which Compound (I) (40 mg/kg) + methotrexate (0.06 mg/kg) were administered in combination (score 0).

Fig. 48 shows the combination effect of Compound (I) and methotrexate on IL-2 production when EL-4 cells (mouse T-lymphocyte cell line) were stimulated with Phorbol 12-myristate 13-acetate (PMA) and Calcium Ionophore A23187. Fa in the drawing represents IL-2 production inhibition rates and CI represents Combination Index.

BEST MODE FOR CARRYING OUT THE INVENTION

Hereinbelow, the present invention is described in more detail.
The indication of amino acids, peptides and the like by abbreviations in this specification follows the rules by IUPAC, IUB, "Guideline for preparing specification and so on containing base sequence or amino acid sequence" (edited by Japan Patent Office) and commonly used symbols in the field.
Examples of the aryl group include aryl groups having 6 to 14 carbon atoms and specifically include phenyl, 1-naphthyl, 2-naphthyl, phenanthryl, anthryl. Preferably included are phenyl, 1-naphthyl, 2-naphthyl.
Examples of the heterocyclic group include monocyclic, bicyclic or tricyclic 5- to 7-membered saturated or unsaturated heterocyclic groups containing 1 to 6 nitrogen atoms, oxygen atoms and/or sulfur atoms.
Specifically, the saturated heterocyclic group includes monocyclic, bicyclic or tricyclic 5-membered saturated heterocyclic groups such as tetrahydrofuryl, pyrrolidinyl, pyrazolidinyl and imidazolidinyl, monocyclic, bicyclic or tricyclic 6-membered saturated heterocyclic groups such as piperidyl, morpholinyl, thiamorpholinyl, piperazinyl and hexahydro-pyrimidinyl, monocyclic, bicyclic or tricyclic 7-membered saturated heterocyclic groups such as azepanyl.
Specifically, the unsaturated heterocyclic group includes monocyclic, bicyclic or tricyclic 5-membered unsaturated heterocyclic groups such as furyl, thienyl, indolyl, isothiazolyl, benzothienyl, isobenzofuranyl, pyrrolyl, benzofuryl, imidazolyl, 4,5-dihydro-1H-imidazolyl, pyrazolyl, isooxazolyl, isothiazolyl, thiazolyl, oxazolyl, benzimidazolyl, benzothiazolyl, benzoxazolyl and carbazolyl, monocyclic, bicyclic or tricyclic 6-membered unsaturated heterocyclic groups such as pyridyl, pyrazinyl, pyrimidinyl, 1,4,5,6-tetrahydro-pyrimidinyl, 3,6-dihydro-2H-[1,3,5]oxadiazinyl, pyridazinyl, triazinyl, quinolyl, isoquinolyl, quinazolinyl, quinoxalinyl, chromenyl and 2,3-dihydrobenzo[1,4]dioxanyl, monocyclic, bicyclic or tricyclic 7-membered unsaturated heterocyclic groups such as 4,5,6,7- tetrahydro-1H-[1,3]diazepinyl.
Examples of the substituent group in the substituted aryl group and substituted heterocyclic group include any group included in each of the following groups a) to g), and one or more groups of these may be optionally contained as substituent groups.

a): A halogen atom, a nitro group, a cyano group, an azido group, a mercapto group, an amino group which may be substituted, a hydroxylamino group which may be substituted, a lower alkoxy-amino group which may be substituted, a hydroxyl group, an acyl group, an acyloxy group, a carboxyl group, a carbamoyl group which may be substituted, carbamoyloxy group which may be substituted, a sulfo group, a sulfamoyl group which may be substi-

tuted;

b) : $-R^{10}$, $-OR^{10}$, $-CO_2R^{10}$, $-SO_3R^{10}$, $-SR^{10}$, $-OCH_2R^{10}$, $-SCH_2R^{10}$, -C (=NOH) $R^{10}$

[In the formula, $R^{10}$ represents a phenyl group or a monocyclic heterocyclic group. The phenyl group or monocyclic heterocyclic group may be substituted by one or more group optionally selected from the group of, for example, a halogen atom, a lower alkyl group, a lower haloalkyl group, a cyano group, a nitro group, an azido group, a hydroxyl group, a lower alkoxy group, a lower haloalkoxy group, an amino group which may be substituted, a carbamoyl group, a carboxy group, a lower alkylcarbonyl group, a lower alkoxycarbonyl group, a lower alkylthio group, a lower alkylsulfinyl group which may be substituted and a lower alkylsulfonyl group.];

c): An alkyl group, an alkoxy group, an alkoxycarbonyl group, a alkoxy(thiocarbonyl) group, an alkylthio group, an (alkylthio)thiocarbonyl group, an (alkylthio)carbonyl group, an alkylcarbonyl group, an alkylthioyl group, an alkylsulfinyl group, an alkylsulfonyl group, an alkylcarbonyloxy group, an alkylthioyloxy group, an alkylsulfonyloxy group [wherein each group of this group may be substituted by one or more group optionally selected from the group of, for example, a halogen atom, a nitro group, a cyano group, a mercapto group, an oxo group, a thioxo group, an amino group which may be substituted, a hydroxyl group, an acyl group, an acyloxy group, a carboxy group which may be substituted, a carbamoyl group which may be substituted, a carbamoyloxy group, a sulfo group which may be substituted, a sulfamoyl group which may be substituted, $-R^{10}$, $-OR^{10}$, $-SR^{10}$, $-OCH_2R^{10}$, $-SCH_2R^{10}$ (In the formula, $R^{10}$ means the same as above.), a lower cycloalkyl group (wherein the lower cycloalkyl group may be substituted by one or more group optionally selected from the group of, for example, a halogen atom, a lower alkyl group, a lower haloalkyl group, an amino group, a hydroxyl group, a lower alkoxy group which may be substituted and a lower haloalkoxy group.), a lower alkoxy group, a lower alkoxycarbonyl group and lower alkylthio group (wherein the lower alkoxy group, lower alkoxycarbonyl group and lower alkylthio group may be substituted by one or more group optionally selected from the group of, for example, a halogen atom, a lower cycloalkyl group, a monocyclic heterocyclic group, a phenyl group, a cyano group, a nitro group, a hydroxyl group, a lower alkoxy group, a lower haloalkoxy group, an amino group which may be substituted, a carbamoyl group, a carboxy group, a lower alkylcarbonyl group, a lower alkoxycarbonyl group, a lower alkylthio group, a lower alkyl sulfinyl group which may be substituted and a lower alkylsulfonyl group.)];

d): An alkenyl group [wherein the alkenyl group may be substituted by one or more group optionally selected from the group of, for example, a halogen atom, a nitro group, a cyano group, a mercapto group, an oxo group, a thioxo group, an amino group which may be substituted, a hydroxyl group, a lower alkoxy group, a lower haloalkoxy group, a lower alkoxycarbonyl group, a lower alkylthio group, an acyl group, an acyloxy group, a carboxy group, a carbamoyl group which may be substituted, $-R^{10}$, $-OR^{10}$, $-SR^{10}$, $-OCH_2R^{10}$ and $-SCH_2R^{10}$ (In the formula, $R^{10}$ means the same as above.)];

e): An alkynyl group

[wherein the alkynyl group may be substituted by one or more group optionally selected from the group of, for example, a halogen atom, a nitro group, a cyano group, a mercapto group, an oxo group, a thioxo group, an amino group which may be substituted, a hydroxyl group, a lower alkoxy group, a lower haloalkoxy group, a lower alkoxycarbonyl group, a lower alkylthio group, an acyl group, an acyloxy group, a carboxy group, a carbamoyl group which may be substituted, $-R^{10}$, $-OR^{10}$, $-SR^{10}$, $-OCH_2R^{10}$ and $-SCH_2R^{10}$ (In the formula, $R^{10}$ means the same as above.)];

f): An alkenyloxy group, an alkenyloxy carbonyl group, an alkenylcarbonyl group, an alkenylcarbonyloxy group, an alkynyloxy group, an alkynyloxycarbonyl group

[wherein each group of this group may be substituted by one or more group optionally selected from the group of, for example, a halogen atom, an oxo group, an amino group which may be substituted, a hydroxyl group, a lower alkoxy group, a lower haloalkoxy group, an acyl group, an acyloxy group, a lower alkylthio group, a carboxy group, a carbamoyl group which may be substituted, a lower alkoxycarbonyl group and a phenyl group.];

g): A lower cycloalkyl group, a lower cycloalkyloxy group, a lower cycloalkylcarbonyl group, a lower cycloalkylcarbonyloxy group, a lower cycloalkyloxy carbonyl group, a lower cycloalkenyl group, a lower cycloalkenyloxy group, a lower cycloalkenylcarbonyl group, a lower cycloalkenylcarbonyloxy group, a lower cycloalkenyloxy carbonyl group [wherein each group of this group may be substituted by one or more group optionally selected from the group of, for example, a halogen atom, a nitro group, a cyano group, a mercapto group, an oxo group, a thioxo group, a lower alkyl group, a lower haloalkyl group, an amino group which may be substituted, a hydroxyl group, a lower alkoxy group, a lower haloalkoxy group, an acyl group, an acyloxy group, a lower alkylthio group, a carboxy group, a carbamoyl group which may be substituted and a lower alkoxycarbonyl group.].

The substituent group in the substituted aryl group and substituted heterocyclic group specifically includes methyl, 2-methyl-1-propyl, hexyl, 2-methyl-2-propyl, 2-propyl, phenyl, trifluoromethyl, 2,2,2-trifluoroethyl, 1,1,2,2,2-pentafluoroethyl, 6,6,6-trifluorohexyl, hydroxymethyl, hydroxyethyl, methoxymethyl, hexyloxymethyl, cyclopropylmethoxymethyl, acetoxymethyl, N,N-dimethylcarbamoyloxymethyl, methanesulphonyloxymethyl, N,N-dimethylsulfamoyloxymethyl, 2-(1-pyrrolidinyl)ethoxymethyl, 2-methoxyethyl, carboxymethyl, methoxycarbonylmethyl, carbamoylmethyl, amidinomethyl, methylthiomethyl, cyanomethyl, aminomethyl, aminoethyl, N-acetylaminomethyl, ethenyl, 2-propenyl, ethynyl, 2-propynyl, 2-methoxycarbonylethenyl, fluoro, chloro, bromo, nitro, cyano, hydroxy, amino, N,N-dimethylamino, mercapto, sulfo, carboxy, amidino, methoxy, cyclopropylmethoxy, 2-(1-pyrrolidinyl)ethoxy, methoxycarbonylmethoxy, 2-acetoxyethoxy, 2-hydroxyethoxy, 2-methoxyethoxy, 4,4,5,5,5- pentafluoropentoxy, 2-methanesulfinylethoxy, phenoxy, benzyloxy, 4-methoxybenzyloxy, methoxycarbonyloxy, 1-pyrrolidinyl, 3-hydroxy-1-pyrrolidinyl, acetylamino, N-acetyl-N-methylamino, N-methanesulphonylamino, N-methanesulphonyl-N-methylamino, methoxycarbonyl, 2-methyl-2-propyloxycarbonyl, 2,2,2-trifluoroethoxycarbonyl, carbamoyl, N,N-dimethylcarbamoyl, 2-thiazolidinyl, 2-oxazolidinyl, 5-tetrazolyl, methanesulfinyl, sulfamoyl, N,N-dimethylsulfamoyl, acetyl, benzoyl, pivaloyl, trifluoroacetyl, formyl, ethylenedioxymethyl, imino, methoxyimino.

Preferable substituent groups among these substituent groups specifically include methyl, 2-methyl-1-propyl, hexyl, 2-methyl-2-propyl, 2-propyl, phenyl, trifluoromethyl, 2,2,2-trifluoroethyl, hydroxymethyl, hydroxyethyl, methoxymethyl, cyclopropyl methoxymethyl, acetoxymethyl, N,N-dimethylcarbamoyloxymethyl, methanesulphonyloxymethyl, N,N-dimethylsulfamoyloxymethyl,2-(1-pyrrolidinyl)ethoxymethyl, 2-methoxyethyl, carboxymethyl, methoxycarbonylmethyl, carbamoylmethyl, amidino methyl, methylthiomethyl, cyanomethyl, aminomethyl, aminoethyl, N-acetylaminomethyl, fluoro, chloro, bromo, nitro, cyano, hydroxy, amino, N,N-dimethylamino, methoxy, 2-(1-pyrrolidinyl)ethoxy, methoxycarbonylmethoxy, 2-acetoxyethoxy, 2-hydroxyethoxy, 2-methoxyethoxy, 2-methanesulfinylethoxy, 1-pyrrolidinyl, 3-hydroxy-1-pyrrolidinyl, acetylamino, N-acetyl-N-methylamino, N-methanesulphonylamino, N-methanesulphonyl-N-methylamino, methoxycarbonyl, 2-methyl-2-propyloxycarbonyl, 2,2,2-trifluoroethoxycarbonyl, carbamoyl, N,N-dimethylcarbamoyl, methanesulfinyl, acetyl, benzoyl, pivaloyl, trifluoroacetyl.
The number of the substituent groups in the aryl group and the heterocyclic group is preferably one or two or three. The aryl group and the heterocyclic group are also preferable when they have no substituent groups.

Examples of the alkyl group include linear or branched alkyl groups having 1 to 10 carbon atoms, and specifically include methyl, ethyl, propyl, 1-methylethyl, butyl, 1-methylpropyl, 2-methyl-1-propyl, 1,1-dimethylethyl, pentyl, 1,1-dimethylpropyl, 2,2-dimethylpropyl, 1-methylbutyl, 3-methylbutyl, hexyl, 2-methyl pentyl, 3,3- dimethylbutyl, heptyl, 1-ethylpentyl, 5-methylhexyl, octyl, 1,5-dimethylhexyl, 2-ethylhexyl, nonyl and decyl. The lower alkyl groups include alkyl groups having 1 to 6 carbon atoms.

Examples of the substituent group in the substituted alkyl group include any group included in each of the following groups a) to d), and one or more groups of these may be optionally contained as substituent groups.

a): A halogen atom, a nitro group, a cyano group, a mercapto group, an oxo group, a thioxo group, an amino group which may be substituted, a hydroxylamino group which may be substituted, a lower alkoxy amino group, a hydroxyl group, an acyl group, an acyloxy group, a carboxy group which may be substituted, a carbamoyl group which may be substituted, a carbamoyloxy group which may be substituted, a sulfo group, a sulfamoyl group which may be substituted;

b): A lower cycloalkyl group, a lower cycloalkyloxy group, a lower cycloalkylcarbonyl group, a lower cycloalkylcarbonyloxy group, a lower cycloalkyloxycarbonyl group, a lower cycloalkenyl group, a lower cycloalkenyloxy group, a lower cycloalkenylcarbonyl group, a lower cycloalkenylcarbonyloxy group, a lower cycloalkenyloxy carbonyl group; [wherein each group of this group may be substituted by one or more group optionally selected from the group of, for example, a halogen atom, a nitro group, a cyano group, a mercapto group, an oxo group, a thioxo group, a lower alkyl group, a lower haloalkyl group, an amino group which may be substituted, a hydroxyl group, a lower alkoxy group, a lower haloalkoxy group, an acyl group, an acyloxy group, a lower alkylthio group, a carboxy group, a carbamoyl group which may be substituted and a lower alkoxycarbonyl group.];

c): An alkoxy group, an alkoxycarbonyl group, a alkoxy(thiocarbonyl) group, an alkylthio group, an (alkylthio)thiocarbonyl group, an (alkylthio)carbonyl group, an alkylcarbonyl group, an alkylthioyl group, an alkylsulfinyl group, an alkylsulfonyl, an alkylcarbonyloxy group, an alkylthioyloxy group, an alkylsulfonyloxy group
[wherein each group of this group may be substituted by one or more group optionally selected from the group of, for example, a halogen atom, a nitro group, a cyano group, a mercapto group, an oxo group, a thioxo group, an amino group which may be substituted, a hydroxyl group, an acyl group, an acyloxy group, a lower alkylthio group, a carboxy group, a carbamoyl group which may be substituted, a sulfo group, a sulfamoyl group which may be substituted, $-R^{10}$, $-OR^{10}$, $-SR^{10}$, $-OCH_2R^{10}$ and $-SCH_2R^{10}$ (In the formula, $R^{10}$ means the same as above.),
a lower cycloalkyl group (wherein the lower cycloalkyl group may be substituted by one or more group optionally selected from the group of, for example, a halogen atom, a lower alkyl group, a lower haloalkyl group, an amino group which may be substituted, a hydroxyl group, a lower alkoxy group and a lower haloalkoxy group.),
a lower alkoxy group, a lower alkoxycarbonyl group, a lower alkylthio group (wherein the lower alkoxy group, the lower alkoxycarbonyl group and the lower alkylthio group may be substituted by one or more group optionally selected from the group of, for example, a halogen atom, a lower cycloalkyl group, a monocyclic heterocyclic group, a phenyl group, a cyano group, a nitro group, a hydroxyl group, a lower alkoxy group, a lower haloalkoxy group, an amino group which may be substituted, a carbamoyl group which may be substituted, a carboxy group, a lower alkylcarbonyl group, a lower alkoxycarbonyl group, a lower alkylthio group, a lower alkylsulfinyl group and a lower alkylsulfonyl group.)];
d) : $-R^{10}$, $-OR^{10}$, $-SR^{10}$, $-OCH_2R^{10}$ and $-SCH_2R^{10}$ (In the formula, $R^{10}$ means the same as above.).

The substituted alkyl group specifically includes trifluoromethyl, 2-nitroethyl, 2-cyanopropyl, 4-mercaptobutyl, 3-oxobutyl, 2-piperidinoethyl, 2-hydroxyethyl, 3-methoxy propyl, ethoxycarbonylmethyl, cyclopropylmethyl, cyclohexylmethyl, 6-cyclohexyhexyl, 3-cyclohexenylbutyl, 2-phenylbutyl, benzyl, 2-naphthylmethyl, phenethyl, 2-pyridylmethyl, 3-pyridylmethyl, 4-pyridylmethyl, 2-quinolylmethyl, 3-quinolylmethyl, 3-thienylpropyl, hydroxymethyl, hydroxyethyl, aminomethyl, aminoethyl, carboxymethyl, ethoxycarbonylmethyl, carbamoylmethyl.
The lower haloalkyl group refers to a lower alkyl group which is substituted by 1 to 5 halogen atoms.
The alkyloxy group refers to an oxy group to which an alkyl group is linked. Specifically included are methoxy, ethoxy, propoxy, 2-propoxy, butoxy, 1,1-dimethylethoxy, pentoxy, hexoxy. The substituent groups in the substituted alkoxy group include the same substituent groups as in the substituted alkyl group. The substituted alkoxy group specifically includes cyclopropylmethoxy, trifluoromethoxy, 2-pyrrolidinoethoxy, benzyloxy, 2-pyridylmethoxy.
The haloalkoxy group refers to an alkoxy group which is substituted by 1 to 5 halogen atoms.
The alkoxycarbonyl group refers to a carbonyl group to which an alkoxy group is linked. Specifically included are methoxycarbonyl, ethoxycarbonyl, propoxycarbonyl, 2-propoxy. The substituent groups in the substituted alkoxycarbonyl group include the same substituent groups as in the substituted alkyl group.
The alkenyl group includes linear or branched alkenyl groups having 2 to 10 carbon atoms and 1 to 3 double bonds. Specifically included are ethenyl, 1-propenyl, 2-propenyl, 1-methylethenyl, 1-butenyl, 2-butenyl, 3-butenyl, 2-methyl-2-propenyl, 1-pentenyl, 2-pentenyl, 4-pentenyl, 3-methyl-2-butenyl, 1-hexenyl, 2-hexenyl, 1-heptenyl, 2-heptenyl, 1-octenyl, 2-octenyl, 1,3-octadienyl, 2-nonenyl, 1,3-nonadienyl, 2-decenyl. Examples of preferable alkenyl group include ethenyl, 1-propenyl, 1-butenyl. The lower alkenyl group includes alkenyls group having 2 to 6 carbon atoms.
Examples of the substituent groups in the substituted alkenyl group include a halogen atom, a nitro group, a cyano group, a mercapto group, an oxo group, a thioxo group, an amino group which may be substituted, a hydroxyl group, a lower alkoxy group, a lower haloalkoxy group, a lower alkoxycarbonyl group, a lower alkylthio group, an acyl group, an acyloxy group, a carboxy group, a carbamoyl group which may be substituted, $-R^{10}$, $-OR^{10}$, $-SR^{10}$, $-OCH_2R^{10}$ and $-SCH_2R^{10}$ (In the formula, $R^{10}$ means the same as above.).
The alkenyloxy group refers to an oxy group to which an alkenyl group is linked.
The alkynyl group includes linear or branched alkynyl groups having 2 to 10 carbon atoms and 1 to 3 triple bonds. Specifically included are ethynyl, 1-propynyl, 2-propynyl, 1-butynyl, 2-butynyl, 3-butynyl, 1-pentynyl, 2-pentynyl, 4-pentynyl, 1-octynyl, 6-methyl-1-heptynyl, 2-decynyl. Examples of preferable alkynyl groups include 1-propynyl and 1-butynyl group. The lower alkynyl group includes alkynyl groups having 2 to 6 carbon atoms.
Examples of the substituent group in the substituted alkynyl group include a halogen atom, a nitro group, a cyano group, a mercapto group, an oxo group, a thioxo group, an amino group which may be substituted, a hydroxyl group, a lower alkoxy group, a lower haloalkoxy group, an acyl group, an acyloxy group, a lower alkylthio group, a carboxy group, a carbamoyl group which may be substituted, a lower alkoxycarbonyl group, $-R^{10}$, $-OR^{10}$, $-SR^{10}$, $-OCH_2R^{10}$ and $-SCH_2R^{10}$ (In the formula, $R^{10}$ means the same as above.).
The alkynyloxy group refers to an oxy group to which an alkynyl group is linked.
Examples of the cycloalkyl group include cycloalkyl groups having 3 to 10 carbon atoms, and specifically include cyclopropyl, cyclobutyl, cyclopentyl, cyclohexyl and cycloheptyl. The lower cycloalkyl group includes cycloalkyl groups having 3 to 6 carbon atoms. The cycloalkyloxy group refers to an oxy group to which a cycloalkyl group is linked.

Examples of the cycloalkenyl group include those having 3 to 10 carbon atoms, and specifically include cyclohexenyl. The lower cycloalkenyl group includes cycloalkenyl groups having 3 to 6 carbon atoms. The cycloalkenyloxy group refers to an oxy group to which a cycloalkenyl group is linked.
Examples of the substituent groups in the substituted cycloalkyl group and the substituted cycloalkenyl groups include a halogen atom, a nitro group, a cyano group, a mercapto group, an oxo group, a thioxo group, a lower alkyl group, a lower haloalkyl group, an amino group which may be substituted, a hydroxyl group, a lower alkoxy group, a lower haloalkoxy group, an acyl group, an acyloxy group, a lower alkylthio group, a carboxy group, a carbamoyl group which may be substituted and a lower alkoxycarbonyl group.

Examples of the acyl group include an acyl group represented by a formula -Z-$R^{11}$ (In the formula, Z represents -CO-, -CS-, -SO- or -$SO_2$-, and $R^{11}$ represents an alkyl group which may be substituted, an aryl which may be substituted or a heterocyclic group which may be substituted.). The acyl group specifically includes formyl, acetyl, propanoyl, 2-propanoyl, pivaloyl, valeryl, pivaloyl, trifluoroacetyl, benzoyl, naphthoyl, nicotinoyl, methanesulphonyl, trifluoromethane sulfonyl and p-toluenesulfonyl. Preferable acetyl group includes an acyl group. The acyloxy group refers to an oxy group to which an acyl group is linked.

Examples of the substituent groups in the substituted carbamoyl group include an alkyl group which may be substituted by an aryl group or a heterocyclic group, and an aryl group and a heterocyclic group, and the same or different plural groups may be independently contained as substituent groups. The substituted carbamoyl group specifically includes ethylcarbamoyl, dimethylcarbamoyl, phenylcarbamoyl, 2-pyridylcarbamoyl, benzylcarbamoyl(3-pyridylmethyl) carbamoyl.
Examples of the substituent group in the substituted sulfamoyl group include an alkyl group, an aryl group, a heterocyclic group, and the same or different plural groups may be independently contained as substituent groups. The substituted sulfamoyl group specifically includes ethylsulfamoyl, dimethylsulfamoyl, phenylsulfamoyl, 2-pyridylsulfamoyl.
Examples of the substituent group in the substituted amino group include an acyl group, an alkyl group, and the same or different plural groups may be independently contained as substituent groups. The substituted amino group includes acetamide, propionamide, butylamide, 2-butylamide, methylamino, 2-methyl-1-propylamino, diethylamino.
The substituent group in the substituted hydroxylamino group may be present either on the nitrogen atom or the oxygen atom, and the substituent group includes the same as in the substituted amino group.

Examples of the halogen atom include a fluorine atom, a chlorine atom, a bromine atom, an iodine atom.

Examples of the alkylene group include a linear or branched alkylene group having 1 to 10 carbon atoms. Specifically included are methylene, ethylene, trimethylene, tetramethylene, pentamethylene, hexamethylene, heptamethylene, octamethylene, nonamethylene, decamethylene, methylmethylene, ethylmethylene, dimethylmethylene, 1,1-dimethylethylene, 1,2-dimethylethylene, 1-methyltrimethylene, 2-methyltrimethylene, 1,1-dimethyltrimethylene, 1,2-dimethyltrimethylene, 1,3-dimethyltrimethylene, 2,2-dimethyltrimethylene, 1-ethyltrimethylene, 2-ethyltrimethylene, 1,1-diethyltrimethylene, 1,2-diethyltrimethylene, 1,3-diethyltrimethylene, 2,2-diethyltrimethylene.
Examples of the lower alkylene group include a linear or branched alkylene group having 1 to 6 carbon atoms.

Various protecting groups ordinarily used can be used as the protecting group for NH group and, preferably include, for example, carbamate type protecting groups such as methoxycarbonyl, ethoxycarbonyl, tert-butoxycarbonyl and benzyloxycarbonyl, amide type protecting groups such as acetyl and benzoyl, benzyl, nitro, p-toluenesulfonyl and methanesulphonyl.

Examples of the heterocyclic ring in the heterocyclic ring which may be substituted and is formed by linking of any two of $R^1$, $R^2$, $R^3$ and $R^4$ together with an nitrogen atom include 5- to 7-membered monocyclic or bicyclic saturated or unsaturated heterocyclic rings containing 1 to 6 nitrogen atoms, oxygen atoms and/or sulfur atoms (containing at least one nitrogen atom). Specifically included are pyrrolidine, imidazolidine, 4,5-dihydro-1H-imidazole, piperidine, piperidin-4-one, piperazine, morpholine, thiamorpholine, 1,4,5,6- tetrahydro-pyrimidine, hexahydro-pyrimidine, and 3,6-dihydro-2H-[1,3,5]oxadiazine. Examples of substituent groups in the substituted heterocyclic rings include the same as in the substituted heterocyclic groups.
Examples of the hydrocarbyl ring which may be substituted and is formed by linking of $R^8$ and $R^9$ together with a carbon atom include a cycloalkane ring having 3 to 8 carbon atoms which may be substituted or a cycloalkane ring having 3 to 8 carbon atoms which may be substituted. The cycloalkane ring or cycloalkene ring specifically includes cyclopropane, cyclobutane, cyclopentane, cyclohexane, cycloheptane, cyclopropene, cyclobutene, cyclopentene, cyclohexene, cycloheptene. Examples of substituent groups in the substituted hydrocarbyl rings include the same as in the substituted cycloalkyl groups.

Among the isoxazole compounds of Formula 1, those in which a polar functional group is introduced to the isoxazole ring have improved disposition and fewer side effects, and can be administered for a long term.

Pharmaceutically acceptable salts of the isoxazole derivative of Formula 1 include acid addition salt and base addition salts. Examples of the acid addition salts include inorganic salts such as hydrochlorides, hydrobromides, sulfates, hydroiodides, nitrates and phosphates, and organic salts such as citrates, oxalates, acetates, formates, propionates, benzoates, trifluoroacetates, fumarates, maleates, tartrates, aspartates, glutamates, methanesulfonates, benzensuplhonates, camphorsulphonates. Examples of the base addition salts include inorganic base salts such as sodium salts, potassium salts, calcium salts, magnesium salts and ammonium salts, and organic base salts such as triethylammonium salts, triethanolammonium salts, pyridinium salts, diisopropylammonium salts.

The CaMKII inhibitor of the present invention includes all stereoisomers, optically active substances, tautomers, prodrugs, derivatives and the like of the specific compounds exemplified in instant specification (for example, isoxazole derivative of Formula 1, Compound (I), Compound (Ib), Compound (Ic), Compound (II), etc.). In addition, the present invention encompasses solvates thereof such as hydrates and all crystalline forms.

The isoxazole derivatives of Formula 1 can be produced, for example, by a method described in Patent Document 3.

Hereinbelow, the prophylactic and/or therapeutic agent for an autoimmune disease of the present invention and a process for screening a CaMKII inhibitor of the present invention are described in detail.

## I. Prophylactic and/or therapeutic agent for an autoimmune disease

Generally, the "autoimmune disease" is diseases in which antibodies or lymphocytes which react to a component constituting an organ of oneself are persistently produced in the body and cause disorders of the organ and they can be roughly classified into the following two categories of organ-specific autoimmune diseases and organ-nonspecific autoimmune diseases (systemic autoimmune diseases).

(1) Organ-specific autoimmune diseases: Hashimoto’s disease, primary myxedema, thyrotoxicosis, pernicious anemia, Good-pasture’s syndrome, rapidly progressive glomerulonephritis, myasthenia gravis, pemphigus vulgaris, bullous pemphigoid, insulin resistant diabetes, juvenile diabetes, type I diabetes mellitus, Addison’s disease, atrophic gastritis, male sterility, climacterium precox, crystalline lens-induced uveitis, multiple sclerosis, ulcerative colitis, primary biliary cirrhosis, chronic active hepatitis, autoimmune blood disease (for example, autoimmune hemolytic anemia, cataplectic thrombocytopenia), paroxysmal hemoglobinuria, idiopathic thrombocytopenic purpura, interstitial pulmonary fibrosis and Sjogren’s syndrome.

(2) Organ-nonspecific autoimmune diseases (systemic autoimmune diseases): rheumatoid arthritis, systemic lupus erythematosus, discoid erythematosus, polymyositis, scleroderma and mixed connective tissue disease.

The present invention is directed to any of these "autoimmune diseases". Preferably exemplified are rheumatoid arthritis and multiple sclerosis. More preferably, rheumatoid arthritis is exemplified. The prophylactic agent and/or therapeutic agent, and enhancer, composition, commercial package, kit, use or method of the present invention can be applied to any mammal suffering from these autoimmune diseases, and more preferably patients suffering from autoimmune diseases.

The "prophylactic agent and/or therapeutic agent for an autoimmune disease" of the present invention means those having one or more actions selected from the actions shown by the following (1) and (2) for the various autoimmune diseases mentioned above.

(1) Prophylactic action: action to suppress the onset of disease (onset suppressing action) and action to delay the onset of disease (onset delaying action)

(2) Therapeutic action: action to ameliorate or improve the condition after the onset of disease (condition ameliorating action, condition improving action), action to suppress the progress of the condition (progress suppressing action), action to suppress the development of the condition (development suppressing action) or action to cure the disease (curing action)

In this sense, the "prophylactic agent and/or therapeutic agent for an autoimmune disease" of the present invention encompasses prophylactic agents for autoimmune diseases (the prophylactic agents for autoimmune diseases include agents for suppressing the onset of autoimmune diseases and agents for delaying the onset of autoimmune diseases), therapeutic agents for autoimmune diseases (the therapeutic agents for autoimmune diseases include agents for ameliorating the condition of autoimmune diseases, agents for improving the condition of autoimmune diseases, agents for suppressing the progress of autoimmune diseases, agents for suppressing the development of autoimmune diseases) and agents having the both prophylactic action and the therapeutic action of autoimmune diseases. The prophylactic action can be rephrased as prophylactic effect and the therapeutic action can be rephrased as therapeutic effect.

The "CaMKII inhibitory action" in the present invention widely means inhibitory actions to inhibit the expression

of the functions of CaMKII regardless of the mechanism of the action. Here, the "action to inhibit the expression of the function of CaMKII" encompasses action to inhibit synthesis of protein (including gene expression of CaMKII) or secretion of CaMKII, action to inhibit activation (phosphorylation) of expressed CaMKII, action to deactivate (dephosphorylate) activated CaMKII and action to inhibit the enzymatic activity of activated CaMKII. In this specification, such inhibition of the expression of the function of CaMKII is also referred to simply as CaMKII inhibitory effect or inhibition of CaMKII.

Note that it is sufficient if the "CaMKII inhibitor (substance having CaMKII inhibitory action)" of the present invention is a substance having at least one of the actions of the inhibitory actions mentioned above regardless of the form, nature or manner thereof. In addition, t is sufficient that the "CaMKII inhibitor" of the present invention has inhibitory action at least on human CaMKII and the presence or absence of inhibitory action on CaMKII derived from mammals other than human and other species is not limited in particular.

Proteins, peptides and low molecular weight compounds are also included in the CaMKII inhibitor of the present invention. Compounds having CaMKII inhibitory effect, for example, low molecular weight compounds can be obtained by performing the screening process of the present invention described below on a low molecular weight compound library (for example, low molecular weight compound libraries such as those available from CamBridge Research Laboratories Company).

When the CaMKII inhibitor of the present invention is an anti-CaMKII antibody, it may be any kind of antibody as long as the antibody can specifically recognize CaMKII, and such an antibody can be prepared by known methods using CaMKII protein. The anti-CaMKII antibody of the present invention encompasses polyclonal antibodies, monoclonal antibodies, chimeric antibodies, single strand antibodies and parts of the above antibodies having antigen-binding properties such as Fab fragments an fragments generated by Fab expression library or the like.

As the CaMKII inhibitor of the present invention, polynucleotides having at least 15 consecutive bases in the base sequence of a CaMKII gene and/or polynucleotides complementary thereto can be exemplified. The CaMKII gene may be any isoform such as α, β, β', γ and δ, but preferably includes CaMKII γ gene or CaMKII δ gene, and more preferably includes CaMII γ gene.

For example, gene sequences registered in GenBank under the following Accession Nos. can be used.

| | |
|---|---|
| Human CaMKII α | Accession No. AF145710 |
| Human CaMKII β | Accession No. NM_172084 |
| Human CaMKII γ | Accession No. L07044 |
| Human CaMKII δ | Accession No. AF071569 |

Gene sequences of various kinds of variants for each CaMKII gene isoform are also registered in GenBank, and the sequence information of these can be also used.

Here, the complementary polynucleotides (complementary strand, opposite strand) mean polynucleotides having complementary relation in terms of bases, which is based on the base pair relationship of A:T and G:C, with the full length sequence of polynucleotide comprising the base sequence of CaMKII gene or partial sequences thereof having at least 15 consecutive bases in the said base sequence (for convenience, these are referred to as "normal strands" here). It should be noted, however, that the complementary strands are not limited to those which form completely complementary sequences to the object normal strands but it is sufficient that they have complementary relationship enough to hybridize with the object normal strands under stringent conditions. The stringent conditions as used herein can be determined based on the melting temperature (Tm) of the complex or the nucleic acid which binds to the probe as shown in Berger and Kimmel (1987, Guide to Molecular Clonirlg Techniques Methods in Enzymology, Vol. 152, Academic Press, San Diego CA). For example, the washing condition after hybridization ordinarily includes conditions such as "1xSSC, 0.1% SDS, at 37°C". It is preferable that the complementary strand should maintain hybridization with the object normal strand even after washed under such a condition. More stringent hybridization conditions are not limited in particular, but include washing conditions such as "0.5×SSC, 0.1% SDS, at 42°C" or "0.1×SSC, 0.1% SDS, at 65°C" as more stringent hybridization conditions. As such a complementary strand, specifically exemplified are a strand comprising a base sequence which is completely complementary to the base sequence of the object normal strand and strands comprising base sequences having at least 90%, preferably 95% homology with the original strand.

The polynucleotide on the normal strand can include not only the strand comprising the base sequence of the above CaMKII gene or a partial sequence thereof but also the strands comprising the base sequences having complementary relation with base sequences of the above complementary strands.

Each of the polynucleotides of the above normal strand and the polynucleotides of the complementary strands (reverse strands) may be used as a disease marker in the form of a single strand or may be used as a disease marker in the form of a double strand.

In addition, the CaMKII inhibitor of the present invention includes siRNA (small interfering RNA) for the CaMKII gene as one embodiment of the polynucleotides having at least 15 consecutive bases in the base sequence of the

CaMKII gene and/or the complementary polynucleotides thereto. The siRNA is the general term of a double-stranded RNA having 21 to 23 bases to be used in RNAi (RNA interference). RNAi is a phenomenon that a double-stranded RNA introduced into a cell suppresses expression of a gene (proteosynthesis) containing the same sequence, and in late years this method attracts attention as a method which is effective for analyzing gene function since it destroys the target gene (mRNA) and thereby suppresses the expression thereof.

siRNA for the CaMKII gene can be designed and prepared by known methods in various ways based on the information of the above base sequence (GenBank) about the CaMKII gene (any isoform of α, β, β', γ and δ can be used). For example, as a design method of siRNA, the method described in Chalk et al. "Improved and automated prediction of effective siRNA", Biochem Biophys Res Commun. 2004; 319(1): 264-74 or Reynolds et al., "Rational siRNA design for RNA interference", Nature Biotechnology 2004; 22, 326-330. It is also possible to design an appropriate siRNA using siRNA designing programs available on Internet (for example, Cold Spring Harbor Laboratory, "siRNA sequence finder", http://www.sinc.sunysb.edu/Stu/shilin/rnai.html). It can be confirmed whether the thus designed and prepared siRNA really has an action to suppress the expression of the CaMKII gene by the testing method exemplified, for example, in Example 16.

The CaMKII inhibitor described above is useful as a prophylactic and/or therapeutic agent for an autoimmune disease, particularly as a prophylactic and/or therapeutic agent for an autoimmune disease used in combination with DMARDs. In addition, the CaMKII inhibitor is not particularly limited to the substances specifically mentioned above and can be obtained by a screening process of the present invention described below.

Therefore, the prophylactic and/or therapeutic agent for an autoimmune disease, particularly the prophylactic and/or therapeutic agent for an autoimmune disease to which the present invention is directed includes either of:

(1) Preparations which contain an effective amount of the CaMKII inhibitor mentioned above; and
(2) Preparations which contain an effective amount of the CaMKII inhibitor obtained by a screening process described below.

Here, the effective amount of the CaMKII inhibitor is not limited in particular as long as the amount of the contained CaMKII inhibitor is such an amount that the prophylactic and/or therapeutic agent for an autoimmune disease containing the CaMKII inhibitor exhibits the CaMKII inhibitory effect as a result. The prophylactic and/or therapeutic agent for an autoimmune disease may further contain pharmaceutically acceptable carrier and various additives besides the substance having a CaMKII inhibitory effect.

Such carriers and kinds of additives, form of the prophylactic and/or therapeutic agent for an autoimmune disease and dosage of the prophylactic and/or therapeutic agent for an autoimmune disease (dosage, administration method and dose) will be described in section (III) below in detail.

## II. Screening process of the active ingredient of the prophylactic and/or therapeutic agent for an autoimmune disease (particularly the prophylactic and/or therapeutic agent for an autoimmune disease useful in combination with DMARDs)

The present inventors have newly found that the onset of an autoimmune disease is suppressed and/or the condition thereof can be improved by inhibiting the functional expression of CaMKII as shown in the Examples below. It is considered from this that the CaMKII inhibitor is useful as an active ingredient of a prophylactic and/or therapeutic agent for an autoimmune disease. Furthermore, the present inventors have newly found that combined administration of a CaMKII inhibitor and DMARD have a conspicuously excellent prophylactic and/or therapeutic effect on autoimmune diseases which cannot be expected from the effect of each independent as shown in the Examples. It is considered from this that the CaMKII inhibitor is useful as an agent used in combination for prophylaxis and/or treatment of autoimmune diseases used in combination with DMARDs and as a prophylactic agent and/or therapeutic enhancer for autoimmune diseases by disease modifying antirheumatic drug.

The screening process of the present invention is developed based on these findings, and the object of the process is to acquire an active ingredient of (i) a prophylactic and/or therapeutic agent for an autoimmune disease which is effective even when used alone or (ii) an agent used in combination for prophylaxis and/or treatment of autoimmune diseases used in combination with DMARDs by searching for a CaMKII inhibitor from the test substances.

It is sufficient that the "CaMKII inhibitor", the object of the screening process of the present invention, is a substance capable of inhibiting functional expression (function display, action) of CaMKII, and the mechanism of the action is not limited in particular. The CaMKII inhibitory action specifically includes 1) action to inhibit the enzymatic activity of activated CaMKII, 2) action to inhibit the activation of CaMKII, 3) action to deactivate (dephosphorylate) activated CaMKII and 4) action to inhibit expression or secretion of CaMKII. CaMKII inhibitor should have at least 1 of CaMKII inhibitory effect to take. It is sufficient if the CaMKII inhibitor has at least one of the CaMKII inhibitory actions.

Candidate substances which can be CaMKII inhibitors include nucleic acids, peptides, proteins, organic compounds (including low molecular compounds), and inorganic compounds. The screening process of the present invention

can be performed on these candidate substances and samples containing these substances (generally referred to as "test substance"). Furthermore, as a sample (test substance) containing a candidate substance, cell extract, expression product of gene library, microbe culture supernatant and cell ingredient can be used.

The "CaMKII inhibitor" of the present invention may have any kind of structure as long as it is pharmaceutically acceptable, and it is not limited to those mentioned below, but specific examples of preferable substances include the following. Isoxazole derivative of Formula 1

Formula 1:

[Formula 13]

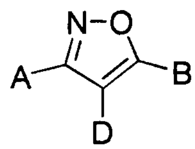

[In the formula, A, B and D means the same as in the above item [6].]

· Of the isoxazole derivatives of Formula 1, more preferable compounds include those mentioned in the above items [7], [8], [9], [10], [11], [12], [13], [14], [15] and [16] .
· 3-[(1S)-1-(2-fluorobiphenyl-4-yl)ethyl]-5-{[amino(morpholin-4-yl)methylene]amino}isoxazole (Compound (I))
· N'-{3-[1-(2-fluorobiphenyl-4-yl)-ethyl]-isoxazol-5-yl}-N,N-dimethyl-guanidine hydrochloride (Compound (Ib))
N-ethyl-N'-{3-[1-(2-fluorobiphenyl-4-yl)-ethyl]-isoxazol-5-yl}-guanidine hydrochloride (Compound (Ic))
· 4-[1-(2-fluorobiphenyl-4-yl)ethyl]-2-(methylamino)-ethylthiazole (Compound (II)))
· KN-93: N-(2-[N-[4-chlorocinnamyl]-N-methylaminomethyl)phenyl)-N-(2-hydroxyethyl)-4-methoxybenzene sulfonamide
· 1-[1-oxo-11-(5,6-dihydro-3-iminobenzo[h]cinnolin-2(3H)-yl)undecyl]-4-(2-pyrimidinyl)-piperazine
Peptide having amino acid sequence shown as sequence Lys-Lys-X-Leu-Arg-Arg-Gln-Glu-Ala-Phe-Asp-Ala-Tyr (In the formula, X is Ala or Lys.)
· Peptide having amino acid sequence shown as sequence Lys-Lys-Ala-Leu-His-Arg-Gln-Glu-Ala-Val-Asp-Cys-Leu

The screening process of the present invention is carried out by searching for a substance having at least one of the above CaMKII inhibitory effects from the test substances using as an index the above CaMKII inhibitory effects. The thus selected and obtained test substance is useful as an active ingredient of a prophylactic and/or therapeutic agent for an autoimmune disease, and it is also useful as an active ingredient of a prophylactic and/or therapeutic agent for an autoimmune disease to be used in combination with DMARDs.

Specific embodiment of the screening process of the present invention is not limited, but examples thereof include the methods shown below.

Process 1

Screening process of CaMKII inhibitor comprising the following steps (a), (b) and (c):

(a) Step of bringing a test substance into contact with a CaMKII enzyme and a substrate;
(b) Step of measuring a phosphorylation level of the substrate by the CaMKII enzyme brought into contact with the test substance, and comparing the phosphorylation level with a phosphorylation level of the substrate by a control enzyme kept from contact the test substance; and
(c) Step of selecting, on the basis of comparison result of the above (b) a test substance that inhibits CaMKII activity.

Process 2

Screening process of the substance having inhibitory effect on the production of CaMKII comprising the following steps (a), (b) and (c):

(a) Step of bringing a test substance into contact with CaMKII expression cells (Jurkat cells, THP-1 cells, etc.) with;

(b) Step of measuring an amount of CaMKII protein in the CaMKII expression cells brought into contact with the test substance (by Western blotting method, etc.), and comparing the amount of the protein with an amount of the protein in control cells kept from contact the test substance; and
(c) Step of selecting a test substance that inhibits the production of CaMKII based on the comparison result in the above (b).

Process 3

Screening process of the substance having inhibitory effect on the gene expression of CaMKII comprising the following steps (a), (b) and (c):

(a) Step of bringing a test substance into contact with CaMKII expression cells (Jurkat cells, THP-1 cells, etc.);
(b) Step of measuring an amount of expression of CaMKII gene in the CaMKII expression cells brought into contact with the test substance (by Northern blotting, RT-PCR, etc.) and comparing the amount of the gene expression with an amount of the gene expression in the control cells kept from contact the test substance; and
(c) Step of selecting a test substance that inhibits the gene expression of CaMKII based on the comparison result in the above (b).

Process 4

Screening process of the substance having inhibitory effect on the activation of CaMKII comprising the following steps (a), (b) and (c):

(a) Step of contacting dephosphorylated CaMKII enzyme with the test substance;
(b) Step of measuring a phosphorylation level of the CaMKII enzyme brought into contact with the test substance and comparing the phosphorylation level with a phosphorylation level of a control enzyme kept from contact the test substance; and
(c) Step of selecting a test substance that inhibits the activation of CaMKII based on the comparison result in the above (b).

The concentration of the test substance for performing screening and criteria for judging that the substance "has CaMKII inhibitory effect" in the processes 1 to 4 above can be set appropriately. For example, for the concentration of the test substance is not limited to these, but it includes 100 μM, 10 μM, 1 μM, 0.1 μM, 0.01 μM, 0.001 μM and intermediate concentrations of these (5 μM, 3 μM, 2 μM, 0.5 μM, 0.3 μM and 0.2 μM). The criteria for judging that the substance "has CaMKII inhibitory effect" is not limited to these, but those in which the inhibition rate of CaMKII compared with the control is, for example, the following:

(1) The inhibition rate of CaMKII is 30% or more when the concentration of the test substance is 10 μM;
(2) The inhibition rate of CaMKII is 40% or more when the concentration of the test substance is 10 μM;
(3) The inhibition rate of CaMKII is 60% or more when the concentration of the test substance is 10 μM;
(4) The inhibition rate of CaMKII is 40% or more when the concentration of the test substance is 1 μM;
(5) The inhibition rate of CaMKII is 60% or more when the concentration of the test substance is 1 μM;
(6) The inhibition rate of CaMKII is 40% or more when the concentration of the test substance is 0.1 μM; and
(7) The inhibition rate of CaMKII is 60% or more when the concentration of the test substance is 0.1 μM.
Preferable criteria include, for example, (1) (2), (3), (4) or (5) in the above criteria. More preferably, (1) (3) or (4) is included. Still more preferably, (3) or (4) is included.

The CaMKII used in the screening process mentioned above may be any of isoforms α, (β, β', γ and δ, but preferably included is CaMKII γ or CaMKII δ, and more preferably included is CaMKII γ.
The screening process of CaMKII inhibitor in the present invention is not limited to the above embodiment and can be designed appropriately based on the technology common among those skilled in the art.
Candidate substances selected from the test substances by the screening process of CaMKII inhibitor described above may be further subjected to an efficacy test (particularly an efficacy test in combination with DMARDs) using, for example, disease animal models which imitate various human autoimmune diseases and the safety test to obtain as an active ingredient of a more practical prophylactic and/or therapeutic agent for an autoimmune disease (prophylactic and/or therapeutic agent for an autoimmune disease particularly suitable for combined use with DMARDs).
More specifically, in order to examine whether the candidate substances selected by the screening process of the present invention are useful as a therapeutic agent for an autoimmune disease, particularly for chronic inflammation

such as rheumatoid arthritis, they can be evaluated by performing an efficacy test using a animal model of rheumatoid arthritis (for example, any of mice or rats suffering from collagen induced arthritis [Trentham DE, Townes AS, Kang AH: Autoimmunity to type II collagens: An experimental model of arthritis J Exp Med146: 857-868,1977], antigen induced arthritis or adjuvant-induced arthritis [Pearson CM: Development of arthritis, periarthritis and periotitis in rats given adjuvant, Proc Soc Expe Biolo Med 91:95-101,1956, etc.].

In particular, suppressive effect (preventive effect, therapeutic effect) on rheumatoid arthritis of the candidate substance can be evaluated in detail by using an adjuvant arthritis model in rat. The adjuvant arthritis in rat is an experimental arthritis in which T-lymphocytes participate in the onset mechanism and which progresses mainly via deterioration and fibrosis of cartilage/bone of joints, and it is widely used as a disease model in the fundamental study of chronic inflammation and study on efficacy and pharmacology of therapeutic agents. The condition of adjuvant arthritis progresses like (1) non-specific primary inflammation period in which only the injected paw makes swelling (the peak comes in 4 to 5 days), (2) induction phase in which activated lymphocytes increase in the local lymph nodes (the peak comes in 5 to 9 days), (3) exacerbation period of the adjuvant arthritis in which polyarthritis develops (the peak comes in 15 to 17 days) and (4) spontaneous curing period in which inflammation subsides leaving deformation/destruction of joints (period after 25th day). While non-specific primary inflammation by adjuvant sensitization also participates in the progress of the condition in the injected paw, the onset and the progress of the condition are caused by participation of T-lymphocytes in non-injected paws, and therefore it is considered that phenomenon more close to rheumatoid arthritis can be observed in the latter. As for the drug action, prophylactic effect by administration on the inoculated day and therapeutic effect by administration started after the arthritis develops are examined. As for the index for efficacy evaluation, (1) respective volume of right and left hind paws, (2) change in paw edema obtained by measuring respective volume of right and left hind paws before and after administration of a drug and calculating the change in the volume before and after the administration, (3) morphological evaluation of bones by soft X-ray and (4) pathological evaluation using tissue sections are generally used.

In addition, suppressive effect (preventive effect, therapeutic effect) on multiple sclerosis of the candidate substance can be evaluated by performing an efficacy test using a animal model of multiple sclerosis (for example, EAE mice).

Prophylactic and/or therapeutic effect of the candidate substance when used in combination with DMARDs can be evaluated by performing a test to administer in combination with DMARDs in the efficacy test using a animal model of an autoimmune disease (rheumatoid arthritis, multiple sclerosis) described above.

The thus selected and obtained substances can be further produced industrially by chemical synthesis, biological synthesis (fermentation) or gene operation based on the results of structural analysis thereof.

The CaMKII inhibitor selected by a screening process of the present invention described as above is useful as an active ingredient of a prophylactic and/or therapeutic agent for an autoimmune disease described as above, particularly as an agent for combined use with DMARDs. The autoimmune disease specifically includes various diseases described in Section I of this specification, but particularly the CaMKII inhibitor of the present invention can be used effectively for rheumatoid arthritis, multiple sclerosis and mixed connective tissue disease, preferably for rheumatoid arthritis and multiple sclerosis, and more preferably for rheumatoid arthritis.

The present invention provides a prophylactic and/or therapeutic agent for an autoimmune disease used in combination with DMARDs containing CaMKII inhibitor obtained by the screening process described above. The present invention also includes other embodiments such as combination drug, enhancer, kit, use and method, too, and these other embodiments will be described in detail later in Section (III).

III. Form and dosage of prophylactic and/or therapeutic agent for an autoimmune disease

The production process of a combination drug, dosage form, administration method and administration amount of prophylactic and/or therapeutic agent for an autoimmune disease are described below.

The combination agent for prophylaxis and/or treatment of autoimmune diseases of the present invention is a preparation comprising a combination of at least one kind of CaMKII inhibitors and at least one kind of disease modifying antirheumatic drugs (DMARDs) which is prepared by combining each of them alone or mixing with a pharmaceutically acceptable carrier or excipient and then combining the both and can be used orally or parenterally. The agent used in combination comprising a CaMKII inhibitor and a DMARD as active ingredients may have any of the forms, for example, (1) prepared into a single preparation optionally with a pharmaceutically acceptable excipient and the like following conventional manufacturing process of pharmaceutical preparations, (2) prepared into respective preparation optionally using a pharmaceutically acceptable excipient and the like respectively and used (combined) at the same time or with a time lag, or (3) prepared into a set (kit agent, etc.) or the like comprising preparations respectively prepared appropriately with a pharmaceutically acceptable excipient and the like. In the case that the respective compounds are separately prepared into preparations, the preparations may be used separately, at the same time or with a time lag, to the same subject and the route of administration and the number of times of administration may be different for the respective

preparations. In the agent used in combination for prophylaxis and/or treatment of autoimmune diseases of the present invention, all of the active ingredients may be included in one preparation, or each of or part of the active ingredients may be formed into preparations separately.

That is, embodiments of combination for prophylaxis and/or treatment of autoimmune diseases of the present invention are not limited to these but include the following embodiments.

(1) A prophylactic and/or therapeutic agent containing a CaMKII inhibitor and a DMARD;
(2) A prophylactic and/or therapeutic agent containing a CaMKII inhibitor for use in combination with a DMARD;
(3) A enhancer of prophylactic and/or therapeutic effect by a DMARD containing a CaMKII inhibitor as an active ingredient;
(4) A kit comprising a first composition containing a CaMKII inhibitor and a second composition containing a disease modifying antirheumatic drug;
(5) A prophylactic and/or therapeutic agent containing a DMARD for use in combination with a CaMKII inhibitor; and
(6) A enhancer of prophylactic and/or therapeutic effect by a CaMKII inhibitor containing a DMARD as an active ingredient.
In these embodiments, the prophylactic agent may be rephrased as a "pharmaceutical composition for prophylaxis", the therapeutic agent as a "pharmaceutical composition for treatment" and the enhancer as a "pharmaceutical composition for enhance" respectively.

In addition, use of either one of CaMKII inhibitors or disease modifying antirheumatic drugs, or combined use of the both for preparing the various prophylactic and/or therapeutic agents, enhancers, kits and the like mentioned above are also included in the embodiments of the present invention.
Furthermore, a method for preventing and/or treating an autoimmune disease characterized in that a CaMKII inhibitor is administered in combination with a disease modifying antirheumatic drug is also included in the embodiments of the present invention. In addition, a method for enhancing a prophylactic and/or therapeutic effect for autoimmune diseases by a disease modifying antirheumatic drug comprising administering a CaMKII inhibitor are also included in the embodiments of the present invention.

When the CaMKII inhibitor is a low molecular weight compound, a peptide or a protein such as an antibody, it can be prepared into a form of a common pharmaceutical composition (pharmaceutical preparation) which is ordinarily used for the substance, and can be administered orally or parenterally depending on the form thereof. The following dosages and administration methods are generally included.

The dosages include solid preparations such as tablets, pills, powdered drugs, powder, granules and capsules, and liquid preparations such as solution, suspension, emulsion, syrup and elixir. These various forms can be classified into oral agents as well as parenteral agents such as transnasal agent, transdermal agent, transrectal agent (suppository), sublingual agent, transvaginal agent and injection agent (intravenous, intra-arterial, intramuscular, subcutaneous, intradermal injection) and intravenous feeding agent. For example, oral agents can have forms such as tablets, pills, powdered drugs, powders, granules, capsules, solution, suspension, emulsion and syrup and transrectal agents and transvaginal agents can have forms such as tablets, pills and capsules. Transdermal agents can be, for example, liquid drugs such as lotions and besides they can have semi-solid forms such as creams or ointments.

Injection agents can have forms such as solution, suspension or emulsion and specific examples thereof include sterilized water, water-propylene glycol solution, buffered liquid, normal saline solution having a concentration of 0.4% by weight. Furthermore, injection agents may be subjected to freezing treatment or lyophilizing treatment after being prepared into liquid agents and can be store in these forms. Freeze-dried preparation prepared by the latter lyophilizing treatment can be used by adding thereto distilled water for injection and the like to be dissolved again at the time of use.

The forms of the pharmaceutical composition (pharmaceutical preparation) described above can be prepared by combining a CaMKII inhibitor and a pharmaceutically acceptable carrier by ordinary methods performed in the art. Examples of the pharmaceutically acceptable carrier include excipient, diluent, filler, extending agent, binding agent, disintegrating agent, humectant, lubricant and dispersant. Additives ordinarily used in the field can be also added. Such additives can be used depending on the form of the pharmaceutical composition to be prepared, appropriately selected, for example, from stabilizing agent, sterilizer, buffer, thickener, pH adjusting agent, emulsifier, suspending agent, preservative, flavor, coloring agent, tonicity adjusting agent, chelating reagent, surfactant, etc.

When the CaMKII inhibitor is an isoxazole derivative represented by Formula 1 or a pharmaceutically acceptable salt thereof, examples of the preparation containing the CaMKII inhibitor include preparations prepared by the methods described in JP-A-11-240873, WO98/47880, JP-A-2000-186077. The preparation containing Compound (I) can be prepared, for example, by the methods described in WO02/092094 pamphlet (corresponding EP patent: EP1374871A1).

The preparation containing a CaMKII inhibitor mentioned above can be used together with a preparation containing a DMARD, but, for example, a DMARD can be added to the above preparation to form a preparation. In addition, the both can be mixed according to a prescription of a doctor to produce a medicinal drug. When they are used

in combination, the CaMKII inhibitor and the DMARD can be administered at the same time or separately via various different routes or, for example, at different time on the same day or at a certain interval over several days to several weeks or even over several months.

The dose and the administration number of times of CaMKII inhibitors in these pharmaceutical compositions can be decided in consideration of the dosage, disease of the patient and the condition thereof, age, weight, general state of health, sex and meal of the patient, administration time, excretion rate, combination of drugs or the other factors. Typically, the respective dose per day for combined use of a CaMKII inhibitor and a DMARD is within the range not less than about 1/50 of the least recommended clinical dose and not more than the greatest recommended clinical dose of the actual condition (recommended clinical dose) for the case that they are individually administered. For example, as for the dose of a CaMKII inhibitor, it can be ordinarily administered, as the amount of the active ingredient per day for an adult, in the range of about 0.0001 mg to about 1000 mg, preferably about 0.001 mg to about 300 mg, more preferably about 0.1 mg to about 300 mg once per day or divided into several times a day. Preferably it is performed by oral administration. These doses can be decreased appropriately while observing the combination effect with a DMARD used together.

When the CaMKII inhibitor is Compound (I), it can be ordinarily administered as the amount of the dose per day for an adult preferably in the range of about 20 mg to about 300 mg, more preferably about 30 mg to about 300 mg, still more preferably about 40 mg to about 240 mg, particularly preferably about 80 mg to about 240 mg once per day or divided into several times a day. The number of times of administration is preferably once a day. More specifically. for example, 80 mg once a day, 120 mg once a day or 240 mg once a day can be orally administered. These doses can be decreased appropriately while observing the combination effect with a DMARD used together.

The disease modifying antirheumatic drugs (DMARDs) used together with a CaMKII inhibitor include drugs which subside rheumatic inflammation and induce remission by correcting immunological abnormality of rheumatoid arthritis. Specifically included are the following drugs.

Leflunomide: Lancet. 1999 Jan 23, 353(9149): 259-66
Methotrexate: US2512572
D-penicillamine: Nature, 151,107 (1943)
Bucillamine: US4305958
Salazosulfapyridine: Br Med J. 1980 Feb 16, 280 (6212): 442-4, also referred to as sulphasalazine
Actarit: US4720506
Lobenzarit: J Rheumatol. 1984 Oct, 11(5): 615-23)
Cyclophosphamide: Naturwiss, 45,64 (1957)
Chlorambucil: US2944079
Mizoribine: US3888843
Azathioprine: US3056785
Auranofin: US3635945
Gold sodium thiomalate: Rheumatol Rehabil, 1975 May, 14 (2) : 101-14
Aurothiosulfate
Aurothioglucose: Agents Actions 1976 Feb; 6(1-3): 355-63)
Cyclosporine: US4117118
Hydroxychloroquine: US2546658
Chloroquine: US2233970
Tacrolimus, FK506: EP184162
Etanercept: Ann Pharmacother 1997 Nov; 31(11): 1335-8 Infliximab: Lancet 1994 Oct 22; 344(8930): 1105-10
Anakinra: Expert Opin Investig Drugs. 2000 Jan; 9(1): 113-27
Keliximab: J Rheumatol 2002 Feb, 29(2): 220-9
Adalimumab, D2E7: Ann Rheum Dis. 1999 Nov, 58 Suppl 1: 170-2)
Rituximab, IDEC -102: Blood 1994 Jan, 83(2): 435-45, Blood 1997 Sep, 90(6): 2188-95
Human type anti-IL-6R antibody (MRA): Ann Rheum Dis. 2000 Nov, 59 Suppl 1: i21-7)

Among these DMARDs, preferably included are leflunomide, methotrexate and salazosulfapyridine. More preferably included is methotrexate.

In use of these DMARDs, side effect often becomes a problem. Examples of the side effect include liver disorder, kidney disorder, gastrointestinal disorder, blood disorder or marrow disorder. More specifically, when methotrexate or leflunomide is selected as a DMARD, methotrexate or leflunomide is difficult to be dosed to a patient suffering from an autoimmune disease (for example, rheumatoid arthritis) who has liver disorder, blood disorder or marrow disorder due to the side effect. Even when there are no these symptoms observed, these side effects may develop while the administration is continued which makes it difficult to continuously administer these DMARDs (methotrexate or leflunomide), forces to reduce the dose or makes it difficult to further increase the dose of DMARDs. On the other hand, the CaMKII inhibitor (for example, isoxazole derivatives of Formula 1 typically represented by Compound (I)) of the present invention

has no or little side effects of this kind, it is particularly effective for patients of autoimmune diseases mentioned above for whom the treatment with DMARDs such as methotrexate or leflunomide is not possible sufficiently. In addition, since the CaMKII inhibitors and DMARDs, when used together, supplement the actions each other, effectiveness (prophylactic and/or therapeutic effect) can be enhanced by combined use of the CaMKII inhibitors and DMARDs even with less administration amount of DMARDs than the amount when administered singly for patients of autoimmune diseases to whom DMARDs cannot be administered in an amount sufficient to attain prophylactic and/or therapeutic effect on the autoimmune diseases due to the side effects mentioned above. Therefore, according to the present invention, totally excellent prophylactic and/or therapeutic agents for autoimmune diseases having fewer side effects and higher effectiveness can be obtained.

When the DMARD is a low molecular weight compound, a peptide or a protein such as an antibody, it can be prepared into a form of a common pharmaceutical composition (pharmaceutical preparation) which is ordinarily used for the substance, and can be administered orally or parenterally depending on the form thereof. The following dosages and administration methods are generally included.

The dosages include solid preparations such as tablets, pills, powdered drugs, powder, granules and capsules, and liquid preparations such as solution, suspension, emulsion, syrup and elixir. These various forms can be classified into oral agents as well as parenteral agents such as transnasal agent, transdermal agent, transrectal agent (suppository), sublingual agent, transvaginal agent and injection agent (intravenous, intra-arterial, intramuscular, subcutaneous, intradermal injection) and intravenous feeding agent. For example, oral agents can have forms such as tablets, pills, powdered drugs, powders, granules, capsules, solution, suspension, emulsion and syrup and transrectal agents and transvaginal agents can have forms such as tablets, pills and capsules. Transdermal agents can be, for example, liquid drugs such as lotions and besides they can have semi-solid forms such as creams or ointments.

Injection agents can have forms such as solution, suspension or emulsion and specific examples thereof include sterilized water, water-propylene glycol solution, buffered liquid, normal saline solution having a concentration of 0.4% by weight. Furthermore, injection agents may be subjected to freezing treatment or lyophilizing treatment after being prepared into liquid agents and can be store in these forms. Freeze-dried preparation prepared by the latter lyophilizing treatment can be used by adding thereto distilled water for injection and the like to be dissolved again at the time of use.

The forms of the pharmaceutical composition (pharmaceutical preparation) described above can be prepared by combining a DMARD and a pharmaceutically acceptable carrier by ordinary methods performed in the field. Examples of the pharmaceutically acceptable carrier include excipient, diluent, filler, extending agent, binding agent, disintegrating agent, humectant, lubricant and dispersant. Additives ordinarily used in the field can be also added. Such additives can be used depending on the form of the pharmaceutical composition to be prepared, appropriately selected, for example, from stabilizing agent, sterilizer, buffer, thickener, pH adjusting agent, emulsifier, suspending agent, preservative, flavor, coloring agent, tonicity adjusting agent, chelating reagent, surfactant, etc.

The pharmaceutical composition having these forms can be administered via a suitable administration route in accordance with the object disease, target internal organs, etc. For example, it can be orally administered, or intravenously, intra-arterially, subcutaneously, intradermally or intramuscularly administered, or directly and locally administered to the tissue itself where lesion caused by the disease can be recognized, or alternatively transdermal administration or rectal administration is also possible.

The dose and the administration number of times of DMARDs in these pharmaceutical compositions can be decided in consideration of the dosage, disease of the patient and the condition thereof, age, weight, general state of health, sex and meal of the patient, administration time, excretion rate, combination of drugs or the other factors. Typically, the respective dose per day for combined use of a DMARD and a CaMKII inhibitor is within the range not less than about 1/50 of the least recommended clinical dose and not more than the greatest recommended clinical dose of the actual condition (recommended clinical dose) for the case that they are individually administered. For example, as for the dose of a DMARD, it can be ordinarily administered as the amount of the active ingredient per day for an adult, in the range of about 0.0001 mg to about 4000 mg, preferably about 0.001 mg to about 3000 mg, more preferably about 0.1 mg to about 300 mg once per day or divided into several times a day. It can be also administered once a week or divided into a couple of times per week assuming one week as an administration cycle.

Examples of the specific clinical dose and dosage of each DMARD includes the following. 5 to 30 mg per day, preferably 10 to 20 mg per day can administered in the case of leflunomide. 5 to 30 mg per week, preferably 6 to 25 mg at once or divided to a couple of times can be administered in the case of methotrexate. 0.5 to 4 mg per day, preferably 1 to 3 mg per day at once per day or divided to a couple of times can be administered in the case of salazosulfapyridine. Oral administration is preferable for any of these agents. These doses can be decreased appropriately while observing the combination effect with a CaMKII inhibitor used together.

The combination drug of the present invention can be used further in combination with a nonsteroidal anti-inflammatory drug or an adrenocorticosteroid drug. The nonsteroidal anti-inflammatory drugs specifically include salicylic acid drugs (aspirin, etc.), anthranil drugs (mefenamic acid, flufenamic acid, etc.), indoleacetic acid drugs (indomethacin, sulindac, acemetacin, etc.), phenylacetic acid drugs (diclofenac, fenbufen, etc.), propionic acid drugs (ibuprofen, ketoprofen, loxoprofen, naproxen, tiaprofen, etc.), oxycam drugs (piroxicam, tenoxicam, ampiroxicam, etc.), pyrazolone drugs (ketofen, butazone, etc.). The adrenocortical steroid drugs specifically include dexamethasone, hydrocortisone, cortisone, prednisolone, methylprednisolone, betamethasone, triamcinolone, triamcinolone acetonide, beclomethasone, fluocinonide, fluocinolone acetonide, halopredone. These compounds may be esterified, may be in the form of salts such as sodium salts, or solvates such as hydrates.

The "therapeutic effect" in the present invention refers to treatment action on autoimmune diseases. As mentioned above, examples of the therapeutic action on autoimmune diseases include action to ameliorate or improve the condition after the onset of disease (condition ameliorating action, condition improving action), action to suppress the progress of the condition (progress suppressing action), action to suppress the development of the condition (development suppressing action) or action to cure the disease (curing action).

When the autoimmune disease is rheumatoid arthritis, the "therapeutic effect" of the present invention can be judged using suppressing effect on chronic inflammation, suppressing effect on joint destruction, progress suppressing effect on joint deformation, improvement effect on physical function, etc. as indices of the effect.

The "improvement of physical function" in a patient with rheumatoid arthritis refers to improvement in the ability of daily living activity which is resulted from improvement in the arthritis conditions such as arthropathy and swelling of joints caused by chronic inflammation of synovial membrane as well as suppression of destruction and deformation of joints. The ability of daily living activity can be evaluated, for example, using as indices putting on and taking off the clothes and dressing, rising, eating a meal, walking, hygienic behavior, extension, grip and activity. Specifically, it can be evaluated, for example, using HAQ (Health Assessment Questionnaire) by a patient (Fries JF, et al., Arthritis Rheumatism, 23, p.137-145,1980).

Improvement criteria by American College of Rheumatology for evaluation of "therapeutic effect" in rheumatoid arthritis (ACR20: Felson Dt et al. The American College of Rheumatology preliminary definition of improvement in rheumatoid arthritis. Arthritis Rheum. 1995,38: p.727-735, 1996, 39: p.34-40) can be also used.

<u>Improvement criteria by American College of</u>

<u>Rheumatology (ACR20):</u> When 20% or more improvement is recognized in the following 1) and 2); and 20% or more improvement is recognized in at least three items of 3) to 7), it is assumed as "20% improvement according to the criteria by American College of Rheumatology" (20% improvement by ACR criteria).

1) Decrease in tender joint count
2) Decrease in swollen joint count
3) Global assessment of disease activity by the patient
4) Global assessment of disease activity by the physician
5) Assessment of pain by the patient
6) Assessment of physical functions by the patient
7) CRP or erythrocyte sedimentation

Likewise, when 50% or more improvement is recognized in the following 1) and 2); and 50% or more improvement is recognized in at least three items of 3) to 7), it is assumed as "50% improvement according to the criteria by American College of Rheumatology" (50% improvement by ACR criteria). In addition, when 70% or more improvement is recognized in the following 1) and 2); and 70% or more improvement is recognized in at least three items of 3) to 7), it is assumed as "70% improvement according to the criteria by American College of Rheumatology" (70% improvement by ACR criteria).

Furthermore, suppressing effect on joint destruction can be evaluated by evaluating progress of hand/foot joint destruction by using X-ray image and therapeutic effect can be judged. As for the evaluation method, for example, Larsen score or Sharp score can be used (Acta Radiologica Diagnosis 18: 481-491. 1977; J Rheumatol 22(10): 1974-1975. 1995; Bailliere's Clinical Rheumatology: 10(3): 435-453, 1996) .

The combination effect of the combined use of a CaMKII inhibitor and a DMARD can be evaluated by using an index optionally selected from the proportion of patients satisfying 20% improvement by ACR criteria, the proportion of patients satisfying 50% improvement by ACR criteria, the proportion of patients satisfying 70% improvement by ACR criteria, or evaluation index such as joint destruction suppressing effects (X-ray) and comparing the therapeutic effect in the group to which either one of a CaMKII inhibitor or a DMARD has been administered (or the both groups) with the therapeutic effect in the combined use group.

In the present invention, the "patient with autoimmune diseases to whom DMARDs cannot be administered in an amount sufficient to attain prophylactic and/or therapeutic effect on the autoimmune diseases due to the side effects" is not limited below, but, for example, includes the patient shown below.

(1) The patient to whom the maximum dose of the DMARD normally used (hereinbelow referred to as "the maximum recommended clinical dose") is already administered without attaining sufficient effect as the prophylactic and/or therapeutic effect on autoimmune diseases;
(2) The patient to whom the administration of the DMARD has been started at a small amount and the dose thereof has been increased step by step while observing the effect, but the dose thereof cannot be increased more than the present dose due to the development of side effects;
(3) The patient to whom the administration of the DMARD has been continued in a certain dose (both of the case that the dose is equal to the maximum recommended clinical dose and the case that the dose is less than that can be supposed) but sufficient prophylactic and/or therapeutic effect on autoimmune diseases cannot be attained any more owing to the above-mentioned escape phenomenon (a state in which tolerance due to the continuation of administration develops and the attenuation in the effect is shown) and the dose thereof cannot be increased any more due to side effects;
(4) The patient having a factor susceptible to the side effect which is easy to be caused by the administration of the DMARD and for whom it is considered to be dangerous to administer the recommended dose enough to attain the effect due to side effects when the DMARD is administered as the single agent; etc.
The side effects of DMARDs specifically include those mentioned above.

Excellent prophylactic and/or therapeutic effect on autoimmune diseases attributable to combined use can be achieved without increasing the dose of DMARDs by performing combined administration with a CaMKII inhibitor of the present invention to these patients of autoimmune diseases. Thus, it is enabled to achieve the excellent prophylactic and/or therapeutic effect on autoimmune diseases while reducing side effects.
The "sufficient therapeutic effect" can be evaluated by an index selected among the various kinds of evaluation indices mentioned above, and can vary depending on various kinds of factors such as the disease of the patient or the condition thereof, but, for example, the above-mentioned "20% improvement according to the criteria by American College of Rheumatology" or "50% improvement according to the criteria by American College of Rheumatology" can be exemplified as specific criteria for rheumatoid arthritis.
The meaning of "exceed the total therapeutic effect" and that of "exceed each of the therapeutic effect" are specifically explained in the following embodiments (1) and (2) of the present invention. (Embodiment 1) A first pharmaceutical composition to be used with a second pharmaceutical composition for achieving prophylactic and/or therapeutic effect on an autoimmune disease in a mammal suffering from the autoimmune disease, wherein said effect exceeds the total prophylactic and/or therapeutic effect on the autoimmune disease achieved by administering the first pharmaceutical composition and the second pharmaceutical composition separately and the second pharmaceutical composition comprises a disease modifying antirheumatic drug and the first pharmaceutical composition comprises a CaMKII inhibitor. (Embodiment 2) A first pharmaceutical composition to be used with a second pharmaceutical composition for achieving prophylactic and/or therapeutic effect on an autoimmune disease in a mammal suffering from the autoimmune disease, wherein said effect exceeds each of the prophylactic and/or therapeutic effect on the autoimmune disease achieved by administering the first pharmaceutical composition and the second pharmaceutical composition separately and the second pharmaceutical composition comprises a disease modifying antirheumatic drug and the first pharmaceutical composition comprises a CaMKII inhibitor.
For example, a test comparing the following four groups is assumed. It is assumed that the effective dose (dose of active ingredients) of the first pharmaceutical composition is identical between Group A and Group C, and the effective dose (dose of active ingredients) of the second pharmaceutical composition is identical between Group B and group C. Group P: group to which no drug has been administered (or placebo administered group)

Group A: group to which only the first pharmaceutical composition has been administered
Group B: group to which only the second pharmaceutical composition has been administered
Group C: group to which the first pharmaceutical composition and the second pharmaceutical composition have been administered in combination

In this case, when the effects in Groups P, A, B and C with regard to the index (assumed as E) of a focused specific therapeutic are E(P), E(A), E(B) and E(C) respectively, the phrase "exceeds the total prophylactic and/or therapeutic effect" in the above "Embodiment 1" means that the effect in Group C exceeds the total of the effect in Group A and the effect in Group B, namely $E(C) > E(A) + E(B)$. For comparison, as E is usually used the standardized value so that the value compared with the group to which no drug has been administered (or placebo administered group) $E(P)=0$.
Meanwhile, the phrase "exceeds each of the prophylactic and/or therapeutic effect" in the above "Embodiment 2" means that the effect in Group C exceeds the effect in Group A and exceeds the effect in Group B, namely "$E(C) > E(A)$ and $E(C) > E(B)$." The index E may be a qualitative index or a quantitative index. As a quantitative index, for example, the proportion of patients satisfying 20% improvement by ACR criteria, the proportion of patients satisfying 50% improvement

by ACR criteria and the like are included whereas as a qualitative index, for example, suppressing effect on hand/foot joint destruction by using X-ray image (evaluation by score) is included.

Examples

Hereinbelow, the present invention is specifically described by way of Examples, but the present invention is not limited by these Examples in any sense.
The following compounds were used in the Examples.

Compound (I): 3-[(1S)-1-(2-fluorobiphenyl-4-yl)ethyl]-5-{[amino(morpholin-4-yl)methylene]amino}isoxazole (This compound can be synthesized, for example, following a method described in the specification of Japanese Patent No. 3,237,608.)

Formula 1a:

[Formula 14]

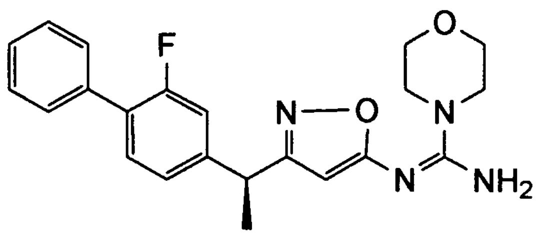

Compound (Ib): N'-{3-[1-(2-fluorobiphenyl-4-yl)-ethyl]-isoxazol-5-yl}-N,N-dimethyl-guanidine hydrochloride (This compound can be synthesized, for example, following a method described in the specification of Japanese Patent No. 3,237,608.)

Formula 1b:

[Formula 15]

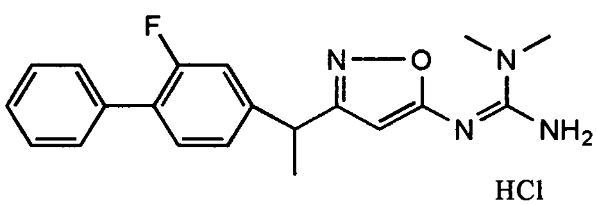

Compound (Ic): N-ethyl-N'-{3-[1-(2-fluorobiphenyl-4-yl)-ethyl]-isoxazol-5-yl}-guanidine hydrochloride (This compound can be synthesized, for example, following a method described in the specification of Japanese Patent No. 3,237,608.)

Formula 1c:

[Formula 16]

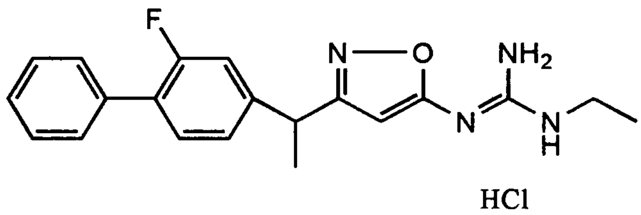

Compound (II): 4-[1-(2-fluorobiphenyl-4-yl)ethyl]-2-(methylamino)-ethylthiazole (This compound can be synthesized, for example, following a method described in JP-B-4-80035.)

Formula 2:

[Formula 17]

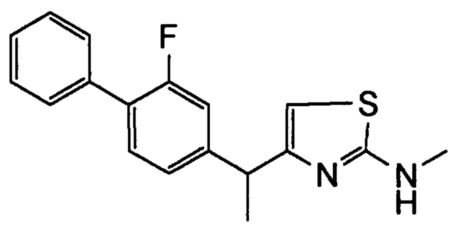

KN-93: N-(2-[N-[4-chlorocinnamyl]-N-methylaminomethyl]phenyl)-N-(2-hydroxyethyl)-4-methoxybenzene sulfonamide (This compound can be synthesized, for example, following a method described in JP-A-6-293730.)

Formula 3:

[Formula 18]

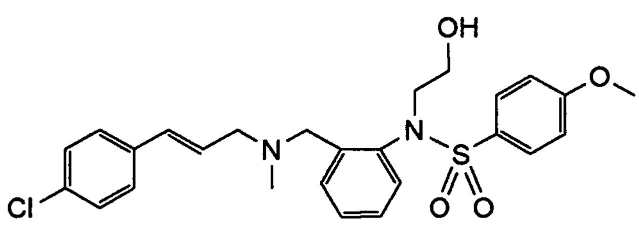

Example 1

Example 1 Examination of CaMKII inhibitory activity (1)

Test substances were dissolved in DMSO and diluted to various concentrations with a buffer solution (50 mM Tris-HCl: pH 7.5, 10 mM $MgCl_2$, 1 mM Dithiothreitol (DTT)) (all of them are available from Nacalai Tesque, Inc.) for

CaMKII reaction. CaMKIIα (final concentration 0.75 μg/ml) (Santa Cruz, Inc.) derived from rat was mixed with Autocamtide-2(R) (40 μM) (CALBIOCHEM Company) dissolved in CaMKII reaction buffer solution in the presence of the test substance and with CaMKII activation buffer solution (1 $mMCaCl_2$, 1 μM calmodulin) (Nacalai Tesque, Inc.) and the reaction was started by further adding ATP (50 μM) (Nacalai Tesque, Inc.). The reaction was performed at 30˚C for five minutes and then SDS-PAGE sample buffer solution was added and the reaction was terminated by boiling for five minutes. Proteins in the reaction mixture were separated by 15% SDS-PAGE and blotted onto a PVDF membrane. Phosphorus taken in by Autocamtide-2 was reacted with an anti-phosphorylated serine/threonine antibody (Santa Cruz, Inc.) and quantified by ECL system (Amersham, Inc.) and NIH Image (downloadable from http://rsb.info.nih.gov/nih-image/).
CaMKII inhibitory activity of each test substance is shown in Table 1. It has been revealed that each test substance has an effect of inhibiting the activity of CaMKII at a concentration of 1 μM or more or 10 μM or more.
Table 1. CaMKII inhibitory effect by test substances
[Table 1]

| Test Substance | Concentration (μm) | Inhibition rate (%) |
|---|---|---|
| Compound (I) | 10 | 85 |
| | 1 | 47 |
| | 0.1 | 39 |
| Compound (II) | 10 | 92 |
| | 1 | 62 |
| | 0.1 | 45 |
| Compound (Ib) | 10 | 71 |
| | 1 | 42 |
| | 0.1 | 13 |
| | 10 | 68 |
| Compound (Ic) | 1 | 36 |
| | 0.1 | 11 |
| KN-93 | 10 | 77 |
| | 1 | 58 |
| | 0.1 | 25 |

Example 2

Example 2 Examination of CaMKII inhibitory activity (2)

CaMKII γ gene was prepared by PCR method using two kinds of primers (5'-TC GGA TCC AGC ATG GCC ACC ACC GCC ACC TG-3' [SEQ ID NO 4] and 5'-TA GAA TTC ACA CTG CAG CGG TGC GGC AGG [SEQ ID NO 5]) synthesized based on the sequence of GenBank Accession No. L07044 and cDNA derived from cultured cells (JurketT cells) prepared by a conventional method as a template. The CaMKII γ gene was excised by BamHI and EcoRI and introduced into the MCS of pFastBacl transfer vector. Expression virus was prepared and amplified based on this vector. The details followed the manual of Bac-to-Bac baculovirus expression system (Invitrogen, Inc.). Expressed CaMKII γ was purified in accordance with the method described in literature (Biochemical & Biophysical Research Communications, 173(2): 578 - 84, 1990). That is, expression cells were pulverized by supersonic wave and preparation was performed by affinity refinement with calmodulin sepharose (Amasham Biosciences, Inc.) from cell soluble fraction obtained by centrifugal operation via ammonium sulfate fraction of final concentration of 60%. Confirmation of expression was performed by Western blotting method using anti-CaMKII γ specific antibody, sc1541 (Santa Cruz, Inc.).
CaMKII γ inhibitory activity of test substances were measured in the same conditions as in Example 1 except human CaMKII γ prepared by the above procedure was used in place of CaMKII α derived from rat.
CaMKII inhibitory activity of each test substance is shown in Table 2. It has been revealed that KN-93 at a concentration of 1 μM or more and Compound (I) at a concentration of 10 μM or more have an effect of inhibiting the activity of CaMKII.
Table 2. CaMKII inhibitory effect by test substances
[Table 2]

| Test Substance | Concentration (μm) | Inhibition rate (%) |
|---|---|---|
| Compound (I) | 30 | 57 |
| | 10 | 33 |
| | 1 | 7 |
| KN-93 | 30 | 107 |
| | 10 | 101 |
| | 1 | 27 |

Example 3

Example 3 Effect on T-lymphocytes

Effects of Compound (I) and KN-93 on IL-2 production when T-lymphocytes were stimulated with Phorbol 12-myristate 13-acetate (PMA) and Calcium Ionophore A23187 were evaluated.
EL-4 cells (mouse T-lymphocyte cell line) were cultured in Dulbecco's Modified Eagle Medium (DMEM: GIBCO, Inc.) containing 10% fetal bovine serum and subjected to the experiment. The cells were disseminated in 96-well plate in a ratio of $5\times10^4$ cells/well and a DMEM medium containing the test substance and a DMEM medium containing PMA and A23187 as a stimulant were added and incubated for 20 hours. The concentration was adjusted so that the final concentrations of the test substances and stimulants were 1 to 30 μM for Compound (I), 1 to 10 μM for KN-93, 25 ng/ml for PMA and 250 ng/ml for A23187. The plate was centrifuged after incubation and the supernatant was collected with micropipette. The concentration of IL-2 in the supernatant was quantified by Enzyme-linked Immunosorbent Assay (ELISA) method. The quantitative method was carried out in accordance with the instructions attached to the kit (MOUSE IL-2 Immunoassay, R & D Systems).
Compound (I) and KN-93 suppressed production of IL-2 dose-dependently as shown in Fig. 1.

Example 4

Example 4 Effect on macrophages

Effects of Compound (I) and KN-93 on IL-6 production when macrophages were stimulated with lipopolysaccharide (LPS) were evaluated.
J774A.1 cells (mouse macrophage cell line) were cultured in Dulbecco's Modified Eagle Medium (DMEM: GIBCO, Inc.) containing 10% fetal bovine serum and subjected to the experiment. The cells were disseminated in 96-well plate in a ratio of $5\times10^4$ cells/well and a DMEM medium containing the test substance and a DMEM medium containing LPS as a stimulant were added and incubated for 24 hours. The concentration was adjusted so that the final concentrations of the test substances and stimulants were 1 to 30 μM for Compound (I), 1 to 3 μM for KN-93 and 1 μg/ml for LPS. The plate was centrifuged after incubation and the supernatant was collected with micropipette. The concentration of IL-6 in the supernatant was quantified by Enzyme-linked Immunosorbent Assay (ELISA) method. The quantitative method was carried out in accordance with the instructions attached to the kit (MOUSE IL-6 Immunoassay, R & D Systems).
Compound (I) and KN-93 suppressed production of IL-6 dose-dependently as shown in Fig. 2. Example 5

Example 5 Effect on the proliferation of fibroblasts

Effects of Compound (I) and KN-93 on the proliferation of fibroblasts when the fibroblasts were stimulated with basic fibroblast growth factor (bFGF) were evaluated.
L929 cells (mouse fibroblast cell line) were cultured in MEM-Earl medium (E-MEM: GIBCO, Inc.) containing 10% fetal bovine serum and subjected to the experiment. The cells were suspended in a serum-free E-MEM and disseminated in 96-well plate in a ratio of $2x10^4$ cells/well and incubated for 72 hours. An E-MEM medium containing the test substance was added and incubated for 1 hour, and an E-MEM medium containing recombinant human FGF basic (bFGF) as a stimulant was added and incubated for 20 hours. 5-Bromo2'-deoxyuridine (Brd-U) solution was added to each well and incubated at 37°C for further four hours. The cells were fixed and uptake of Brd-U was detected by Cell proliferation ELISA system (Amersham) and absorbance at 450 nm was measured with a microplate reader. This value of absorbance was used as an index of cell proliferation.
Compound (I) and KN-93 dose-dependently suppressed the proliferation of fibroblasts as shown in Fig. 3. Example 6

Example 6 Effect on the proliferation of human synovial cells

Effects of Compound (I) and KN-93 on the proliferation of SW982 cells (human synovial cell line) were evaluated. SW982 cells (human synovial cell line; obtained from ATCC) was cultured in a FCS (product of SIGMA, Inc.) containing DMEM medium (product of GIBCO, Inc.) and subjected to the experiment. SW982 was disseminated in 96-well flat bottom plate in the absence of serum in a ratio of $4\times10^4$ cells/well. Compounds were added at the same time of the dissemination of the cells and incubated at 200 μl cells/well. 20 μl of alamar blue(R) was added to each well (BIOSOURCE, Inc.) after 18 hours and incubated for further six hours and the absorbance of each well was measured with a microplate reader. The absorbance was measured at two wavelengths of 570 nm and 600 nm. Assuming the absorbance of the well with cells at 570 nm as A1, the absorbance of the well with cells at 600 nm as A2, the absorbance of the well only containing the medium at 570 nm as B1 and the absorbance of the well only containing the medium at 600 nm as B2, the value of (A1-A2) - (B1-B2) was calculated and displayed as index of cell counts.
Compound (I) and KN-93 which were CaMKII inhibitors dose-dependently suppressed the proliferation of human synovial cells, SW982, as shown in Fig. 4. These results show that the CaMKII activity participates in maintaining the proliferation of SW982 and strongly suggest that Compound (I) and KN-93 induce cell death of SW982 by inhibiting CaMKII. Example 7

Example 7 Effect on the formation of osteoclasts

Effects of Compound (I) and KN-93 on the formation of osteoclasts were evaluated.
A mouse leg affected with developed collagen arthritis was removed and treated in an α-MEM culture medium (GIBCO, Inc.) containing 10% fetal bovine serum in the presence of dispase (Godo Shuzo) and collagenase S1 (Nitta Gelatin Inc.) at 37˚C for five to six hours and monodisperse cell suspended liquid was collected. This sample was centrifuged, rinsed by resuspension and subjected to the experiment. The cells were disseminated in 96-well plate in a ratio of $2x10^4$ cells/well, an α-MEM medium containing the test compound was added thereto and incubated for nine days. After the incubation, osteoclasts were stained with a kit (Sigma, Inc.) for TRAP staining and the number of the osteoclasts per well was measured by microscopic observation. The TRAP staining was carried out in accordance with instructions attached to the kit.
Compound (I) and KN-93 dose-dependently suppressed the formation of osteoclasts as shown in Fig. 5. Example 8

Example 8 Effect on adjuvant-induced arthritis

The adjuvant-induced arthritis model is a representative chronic inflammation model caused in rats, and it is widely used for pharmacological evaluation of antiinflammatory drugs and antirheumatic drugs. Accordingly, effects of Compound (I) and Compound (II) were examined in both therapeutic and prophylactic model. In the therapeutic model, the difference of the volume of a hind leg before and after the dosed period was calculated to examine edema suppression effect by the drug administration and the change of edema volume was examined. In the prophylactic model, the volume of a hind paw was measured over time during the dosed period and the effect of the drug for a long term period was examined. Although the hind paw volume or the edema volume was used as the index of efficacy evaluation, the edema volume means the increment from the normal volume of the hind paw which was not sensitized with the adjuvant, and therefore, the both indices can be used as approximately the same meaning.

(Method)

A liquid paraffin suspension (1 mg/200 μl) of Mycobacterium butyricum (adjuvant) was subcutaneously injected at the tarsal part of the right hind paw of SD or Lewis rats (both male, six-week old) to cause arthritis. In the therapeutic model, 0.5% methylcellulose suspension of Compound (I) or Compound (II) which was prepared so that the dose was 1 ml per 100 g body weight was orally administered once a day for consecutive five days from the 17th day after the injection of the adjuvant. An indomethacin suspension prepared in the similar way was administered as the positive control. The volume of the hind paws was measured respectively for the right and the left by water replacement method just before the administration and in five hours after the last administration and the difference in the values just before the administration and in five hours after the last administration was calculated and assumed as the change in the edema volume. In the prophylatic model, the compounds were administered once a day for consecutive 21 days after the day when the adjuvant was injected. The volume of the right and left hind paws was measured by water replacement method every other day. In the prophylactic model of Compound (II), a suspension of D-penicillamine, an antirheumatic drug, was administered in the same way as a control.
0.5% methylcellulose alone which did not contain the test compound was also administered as a control in the same way as above.

(Results)

Arthritis was developed in the left hind paw (non-injected paw) at about 10 days after adjuvant injection, and the paw volume increased to reach the maximum at about 17 days after adjuvant injection. Therefore, changes in the edema volume in the left hind paw when Compound (I) (2.5 to 80 mg/kg) was orally administered once a day for five days was shown in Fig. 6. Changes in the edema volume in the right and left hind paws when Compound (II) (10 to 50 mg/kg) was orally administered once a day for five days was shown in Fig. 7. In addition, the volume of the right and left hind paws when Compound (I) (2.5 to 80 mg/kg) was orally administered for 21 days after the day when the adjuvant was injected was shown in Fig. 8. The edema volume when Compound (II) (50 mg/kg) was orally administered for 21 days after the day when the adjuvant was injected was shown in Fig. 9. The results of the positive control indomethacin (0.5 mg/kg), D-penicillamine (50 mg/kg) and the control group were also shown in each of the drawings for the purpose of comparison.
As shown in Figs. 6 and 7, remarkable edema suppressing effect was observed in the Compound (I) administered group and Compound (II) administered group in the therapeutic model in which the administration was started after the edema in the left hind paw caused by adjuvant injection reached the maximum. As shown in Figs. 8 and 9, remarkable edema suppressing effect was also observed in the Compound (I) administered group and Compound (II) administered group in the prophylactic model in which the administration was started before the edema in the left hind paw emerged.

Example 9

Example 9 Effect on collagen-induced arthritis in mice

Efficacy evaluation of Compound (I) was performed using a collagen-induced arthritis in mice as an animal model of rheumatoid arthritis. The collagen-induced arthritis was caused following an ordinary method. That is, 0.1 ml of emulsion was injected to a mouse (DBA/1J, male, Charles River Laboratories Japan) at the base of the tail under etherization as primary immunization. The emulsion was prepared by mixing the same volume of a bovine type II collagen solution (2 mg/ml, Collagen Kenshukai) and Freund Complete Adjuvant (Difco). 0.1 ml of emulsion of the same composition was injected at the base of the tail as booster immunization after three weeks and arthritis was caused. The severity of arthritis was indicated as a score according to the following criteria.

0: No symptom
1: Swelling at two or less joints
2: Swelling at three or more joints/Slight redding and edema at limbs
3: Swelling at three or more joints/Moderate redding and edema at limbs
4: Severe redding and edema at limbs/Ankylosis

Evaluation is performed for each limb with 0 to 4 points and therefore the possible maximum score in one individual is 16.
Compound (I) and prednisolone (positive control) were suspended in 0.5% methylcellulose solution and 0.1 ml per 10 g mouse weight was orally administered. In a control group, only 0.5% methylcellulose solution was orally administered.
The administration was started with the primary immunization and carried out once a day for consecutive 35 days.
The results are shown in Figs. 10 and 11. In the control group, 9 of 10 mice developed arthritis, and the degree was severe. On the other hand, the onset was dose-dependently suppressed among the mice to which Compound (I) was administered, and the degree of arthritis was slight. Example 10

Example 10 Effect on the experimental allergic encephalomyelitis

The efficacy evaluation of KN-93 was performed with the experimental allergic encephalomyelitis mice as an animal model of multiple sclerosis. The experimental allergic encephalomyelitis was caused following the method of Bradley et al. (J. Clinical Investigation, 107: 995-1006, 2001). That is, 150 μg of PLP partial peptide ($PLP_{139-151}$: $NH_2$-HSLGKWLGHPDKF-COOH, SEQ ID NO 12) mixed in a Freund complete adjuvant was injected subcutaneously to a seven-week old female SJL/J mouse (purchased from Charles River Laboratories Japan) at the rear back.
The severity of the condition was indicated as a score (disease score) according to the following criteria.

0: No symptom
0.5: Slight paralysis of tail
1: Paralysis of tail
2: Light paralysis of hind leg
3: Medium to intense paralysis of hind leg or light forelimb paralysis

4: Complete paralysis of hind paw or medium to intense paralysis of forelimb
5: Paralysis of four limbs or toward death period
6: Death

KN-93 was suspended in 0.5% methylcellulose solution (0.1 mg/ml) and 0.1 ml per 10 g mouse weight was subcutaneously administered. In a control group, only 0.5% methylcellulose solution was subcutaneously administered. The administration was started on the 7 days after sensitization and carried out once a day for consecutive 24 days.
The results are shown in Fig. 12. The mice in the control group developed severe experimental allergic encephalomyelitis, and 7 of 10 died by neuroparalysis in the test period. On the other hand, the mice administered with KN-93 (1 mg/kg) also developed experimental allergic encephalomyelitis, the condition was much lighter as compared with the control group, and death was 2 of 10.

Example 11

Example 11 Effect on the experimental allergic encephalomyelitis

The efficacy evaluation of Compound (I) was performed with the experimental allergic encephalomyelitis mice as an animal model of multiple sclerosis. The experimental allergic encephalomyelitis was caused following the method of Bradley et al. (J. Clinical Investigation, 107: 995-1006, 2001). That is, 150 μg of PLP partial peptide ($PLP_{139-151}$) mixed in Freund Complete Adjuvant was injected subcutaneously to a seven-week old female SJL/J mouse (purchased from Charles River Laboratories Japan) at the rear back.
The severity of the condition was indicated as a score (disease score) according to the following criteria.

0: No symptom
0.5: Slight paralysis of tail
1: Paralysis of tail
2: Light paralysis of hind paw
3: Medium to intense paralysis of hind paw or light forelimb paralysis
4: Complete paralysis of hind paw or medium to intense paralysis of forelimb
5: Paralysis of four limbs or toward death period
6: Death

Compound (I) was suspended in 0.5% methylcellulose solution to prepare liquids of 8 mg/ml (80 mg/kg administered group), 4 mg/ml (40 mg/kg administered group) and 2 mg/ml (20 mg/kg administered group) for administration respectively and 0.1 ml per 10 g mouse weight was orally administered. In the prednisolone administered group as a positive control, prednisolone was suspended in 0.5% methylcellulose solution to prepare a liquid of 0.3 mg/ml for administration and 0.1 ml per 10 g mouse weight was orally administered. In a control group, only 0.5% methylcellulose solution was subcutaneously administered. The administration was started on the sensitization day and carried out once a day for consecutive 40 days. In normal group, neither of $PLP_{139-151}$ sensitization or administration of the test substance was performed.
The results are shown in Table 3, Fig. 13 and Fig. 14. Compound (I) suppressed the disease score at any dose, and the effect was dose-dependent. In addition, there was no death in the Compound (I) administered group except 1 of 10 died in 80 mg/kg administered group whereas in the control group, all (9 of 9) developed the disease and 4 of 9 of them died by neuroparalysis in the test period. Prednisolone strongly suppressed the onset (only 2 of 10 developed the disease) but different from the control group and the Compound (I) administered group, it strongly suppressed natural accruement of weight by growth.
This result is an effect accompanying continuous administration of prednisolone, and it becomes a problem when long term administration is to be performed to a chronic disease condition. Thus, it has been demonstrated that Compound (I) which was a CaMKII inhibitor dose-dependently suppresses aggravation of the condition of experimental allergic encephalomyelitis without being accompanied with strong suppression of body weight accruement which has been observed in the administration group of prednisolone, a representative steroid, in the case of long term administration.
Table 3. Effect on experimental allergic encephalomyelitis mice
[Table 3]

| Test Substance | Dose (mg/kg) | n | Onset Rate | Death Rate |
|---|---|---|---|---|
| Control Group | | 9 | 9/9 | 4/ 9 |

(continued)

| Test Substance | Dose (mg/kg) | n | Onset Rate | Death Rate |
|---|---|---|---|---|
| Normal Group | | 10 | 0/10 | 0/10 |
| Prednisolone | 3 | 10 | 2/10 | 0/10 |
| Compound (I) | 20 | 10 | 8/10 | 0/10 |
| | 40 | 10 | 7/10 | 0/10 |
| | 80 | 10 | 8/10 | 1/10 |

Example 12

Example 12 Expression of CaMKII in arthritis affected part in collagen-induced arthritis (CIA) in mice

Tissue slices of arthritic joint of CIA mice were prepared and expression of CaMKII protein in the inflammation part was examined by immunostaining procedure.
CIA was caused by an ordinary method. That is, 0.1 ml of emulsion was injected to a mouse (DBA/1J, male, Charles River Laboratories Japan) at the base of the tail under etherization as primary immunization. The emulsion was prepared by mixing the same volume of a bovine type II collagen solution (2 mg/ml, Collagen Kenshukai) and a Freund complete adjuvant (Difco). 0.1 ml of emulsion of the same composition was injected at the base of the tail as booster immunization after three weeks, the mouse was bred for further four weeks, and the hind paw of the mouse which developed arthritis was removed and fixed in 4% paraformaldehyde. Pathologic preparations were prepared from the fixed hind paw and subjected to immunostaining. As for immunostaining, Vectastain$^{(R)}$ ABC-PO (rabbit IgG) kit (Vector, Inc.) and rabbit anti-CaMKII polyclonal antibody (M -176; Santa Cruz Biotechnology) at 50-fold dilution as a primary antibody were used and reacted in a moist chamber overnight. The other procedure was carried out in accordance with the instructions attached to the kit.
As shown in Fig. 15, only few CaMKII expression cells are observed in a normal synovial membrane, while a number of CaMKII expression cells are observed at the sites affected by synovial inflammation. As shown in Fig. 16, a number of CaMKII expression cells were observed in pannus (granulation tissue) where the inflammation aggravated. The above-mentioned findings suggest that CaMKII participates in the formation of rheumatic condition. Example 13

Example 13 Examination of the effect on the degradation of IKBα

Effects of Compound (I) and KN-93 on the degradation of IκBα when T-lymphocytes were stimulated with Phorbol 12-myristate 13-acetate (PMA) and Calcium Ionophore A23187 were evaluated.
EL-4 cells (mouse T-lymphocyte cell line) were cultured in Dulbecco's Modified Eagle Medium (DMEM: GIBCO, Inc.) containing 10% fetal bovine serum and subjected to the experiments. Cells subconfluently multiplied were collected by centrifugation and dispensed into Eppendorf tubes to be contained at $5\times10^6$ cells/tube. After Compound (I) (final 30 μM) or KN-93 (final 10 μM) was added and preincubated at 37°C in a water bath, stimulation by PMA (final 125 ng/ml) and A23187 (final 1 μg/ml) was performed (total volume 500 μl). After incubated for 15 minutes or 30 minutes after the stimulation, 1 ml of an ice cold stop solution (1xPBS, 1 mM $Na_3VO_4$, 5 mM EDTA) was added to terminate the reaction. After centrifugation and rinsing, it was dissolved in TNE buffer (50 mMTris-HCl, 1% NP-40, 20 mM EDTA, 100 μg/ml PMSF, 30 μl/ml Aprotinin, 1 mM $Na_3VO_4$, 5 mM NaF, 12 mM β-glycerolphosphate). After performing 12% SDS-PAGE, proteins were transferred onto a PVDF membrane and western blotting was performed with anti-IκBα antibody (C-21, Santa Cruz, Inc.).
As shown in Fig. 17, Compound (I) and KN-93 suppressed the degradation of IκBa.

Example 14

Example 14 Examination of the effect on the expression of cyclin D1 and the phosphorylation of Akt

Effects of Compound (I) and KN-93 on effect on the expression of cyclin D1 and the phosphorylation of Akt when fibroblasts were stimulated with basic fibroblast growth factor (bFGF) were evaluated.
L929 cells (mouse fibroblast cell line) were cultured in MEM-Earl medium (E-MEM: GIBCO, Inc.) containing 10% fetal bovine serum and subjected to the experiment. The cells were suspended in a serum-free E-MEM and cultured for 72 hours. An E-MEM medium containing the test substance was added and incubated for 1 hour, and an E-MEM medium containing recombinant human FGF basic (bFGF) as a stimulant was added and further incubated. The cells were lysed in a RIPA buffer and a total cell lysate was obtained. After performing SDS-PAGE, proteins were transferred onto a

PVDF membrane and western blotting was performed with anti-cyclin antibody or anti-Akt antibody (both available from Santa Cruz, Inc.).
As shown in Figs. 18 and 19, Compound (I) and KN-93 suppressed the enhanced expression of cyclin D1 and also suppressed the phosphorylation of Akt.

Example 15

Example 15 Changes in the CaMKII expression by inflammatory stimulation

THP-1 (purchased from Dainippon Pharmaceutical), human monocyte cell line, was and subjected to the experiment. THP-1 cells were adjusted to a concentration of $5\times10^5$ cells/ml using a RPMI1640 culture medium (GIBCO) containing 10% FCS (SIGMA), and it is 0.5 ml was added to each well of a 24-well plate. 0.25 μl each of 4 μg/ml of polysaccharide (LPS:Difto) and 400 U/ml IFN-γ (Nacalai Tesque) prepared with RPMI1640 culture medium (GIBCO) containing 10% FCS (SIGMA): was further added and incubation was started. Only RPMI1640 culture medium (GIBCO) containing 10% FCS (SIGMA) was added in unstimulated group. Then, the cells were collected over time, and after cell lysate was prepared with RIPA Buffer, protein content was quantified with a protein content measuring kit (Bio-Rad Dc Protein Assay: BIORAD). 50 μg of cell lysate was added to 8-16% gradient gel (Asahi Techno Glass) to perform SDS-PAGE. After transferred onto a PVDF membrane, it was reacted with a blocking agent (ECL Blocking agent: Amersham) for one hour and then with anti-CaMKII γ antibody (Santa cruz) or anti-CaMKII δ antibody (Santa Cruz) which was a primary antibody and diluted with the blocking agent to 500-fold at room temperature for one hour. After the membrane was rinsed, it was reacted with horseradish iperoxidase labeled antigoat IgG antibody (Amersham) which was a second antibody and diluted with the blocking agent to 2000-fold for 40 minutes. Then the membrane was rinsed and proteins plotted on the membrane were detected with ECLPlus (Amersham).
The results are shown in Fig. 20. It has been revealed that the expression of CaMKII was induced by inflammatory stimulation in THP-1 cells.

Example 16

Example 16 Action of siRNA on the proliferation of the human synovial cells

In order to make the connection of CaMKII and autoimmune diseases more evident, siRNA which was specific to the CaMKII sequence was designed and prepared, and the influence of CaMK II sequence specific siRNA was examined using SW982 cell which was human synovial cell line.

1. Designing and preparation of CaMKII sequence specific siRNA (small interfering RNA)

As a CaMKII sequence specific siRNA, three kinds of specific siRNA sequences shown in Table 4 were designed based on the base sequence information of CaMKII γ gene (GenBank Accession No. L07044), and the siRNAs in which a double strand was formed by a sense strand and an antisense strand were purchased from Japan Bioservice Corporation (hereinbelow, three kinds of siRNAs were referred to as CaMKII γ siRNA(A), (B) and (C)).
Table 4 Sequence of CaMKII γ siRNAs

[Table 4]

| siRNA | Sequence | SEQ ID NO | Position |
|---|---|---|---|
| (A) Sense Strand | 5'-CGU GAG GCU CGG AUA UGU CdTdT-3' | 6 | 199-217 |
| (A) Antisense Strand | 5'-GAC AUA UCC GAG CCU CAC GdTdT-3' | 7 | |
| (b) Sense Strand | 5'-CAU CCA AAC AUC GUG CGC CdTdT-3' | 8 | 229-247 |
| (B) Antisense Strand | 5'-GGC GCA CGA UGU UUG GAU GdTdT-3' | 9 | |
| (C) Sense Strand | 5'-GCA GAU GCC AGC CAC UGU AdTdT-3' | 10 | 352-370 |
| (C) Antisense Strand | 5'-UAC AGU GGC UGG CAU CUG CdTdT-3' | 11 | |

(The "position" means the position numbers in the CaMKII γ sequence registered in GenBank Accession No. L07044).
Scramble II Duplex (DHARMACON) which was an siRNA made of a sequence not occurring in mammals was used as a control.

2. Effect on the cell proliferation by the suppression of CaMKII γ expression

SW982 cells (obtained from ATCC) were cultured in an FCS (SIGMA) containing DMEM culture medium (GIBCO) and subjected to the experiment. The cells were added in a 24-well plate at $1\times10^5$ cells/well, and after 24 hours, 3 μl (60 pmol) of CaMKII γ siRNA and 10.5 μl of Gene Silencer (Gene Therapy Systems, Inc) were added to each well.
Fluorescein Labeled luciferase GL2 Duplex (DHARMACON) which was an siRNA labeled with fluorescence was added instead of the CaMKII γ siRNA to examine the introduction efficiency of siRNA. After culturing for 24 hours, the introduction efficiency of the SiRNA was determined by measuring Fluorescein Labeled luciferase GL2 Duplex uptake in by the cells with a flow cytometer. In addition, as for the amount of CaMKII in the cells, the cells were collected on the 3 days after the introduction of siRNA, and after cell lysate was prepared with RIPA Buffer, protein content was quantified with a protein content measuring kit (Bio-Rad Dc Protein Assay: BIORAD), and 50 μg thereof was added to 8-16% gradient gel (Asahi Techno Glass) to perform SDS-PAGE. After transferred onto a PVDF membrane, it was reacted with a blocking agent (ECL Blocking agent: Amersham) for one hour and then with anti-CaMKII γ antibody (Santa cruz) which was a primary antibody and diluted with the blocking agent to 500-fold at room temperature for one hour. After the membrane was rinsed, it was reacted with horseradish peroxidase labeled antigoat IgG antibody (Amersham) which was a second antibody and diluted with the blocking agent to 2000-fold for 40 minutes. Then the membrane was rinsed and proteins plotted on the membrane were detected with ECLPlus (Amersham).
The proliferation of the cells was measured using uptake of Akmar Blue(R) as the index. That is, the cells were cultured for three days after the introduction of siRNA and cultured for further 24 hours after the medium was changed to serum-free DMEM culture medium. Then, AlamarBlue(R) was added in each well and the fluorescence intensity was measured in one hour with a fluorometer (excited at 544 nm, wavelength: 590 nm).
As a result, it was confirmed that siRNA was introduced into about 80% cells under the condition of this experiment (Fig. 21 and Fig. 22). The effect of the three kinds of siRNAs was examined based on the expression amount of CaMKII γ and the results were shown in Fig. 23. Expression of CaMKII γ was recognized in SW982 cells, but the suppression of the expression of CaMKII γ was observed by introducing siRNA of (A) sequence into the cells. Meanwhile, suppression of the proliferation of the cells was recognized only in the cells to which only siRNA of (A) sequence was introduced (Fig. 24).

3. Discussion

As described above, it has been made definite that the expression of CaMKII γ was deeply involved in the proliferation of SW982 cells because the cell proliferation of SW982, which was a human synovial membrane derived cell, was almost completely suppressed when Compound (I) or KN-93 which was a CaMKII inhibitor was added (Example 6), and because the cell proliferation was suppressed in SW982 to which CaMKII γ siRNA(A), an siRNA which suppressed expression of CaMKII γ, was introduced (this Example). This shows that aberrant proliferation of synovial cells is suppressed and the condition of joints in diseases such as rheumatoid arthritis (suppression of chronic inflammation, suppression of joint destruction, suppression of progress to joint deformation) is achieved by suppressing the expression of CaMKII γ or inhibiting the activity thereof, and I get together,.

Example 17

Example 17 Combination effect on adjuvant-induced arthritis

The adjuvant-induced arthritis model is a representative chronic inflammation model caused in rats, and it is widely used for pharmacological evaluation of anti-inflammatory drugs and antirheumatic drugs. Accordingly, combination effect of 3-[(1S)-1-(2-fluorobiphenyl-4-yl)ethyl]-5-{[amino(morpholin-4-yl)methylene]amino}isoxazole (Compound (I)) which is a CaMKII inhibitor and DMARDs (leflunomide and salazosulfapyridine) was examined using this model.

(Method)

A liquid paraffin suspension (1 mg/200 μl) of Mycobacterium butyricum (adjuvant) was subcutaneously injected at the tarsal part of the right hind paw of Lewis rats (male, six-week old) to cause arthritis. 0.5% methylcellulose suspension of Compound (I) (10 mg/kg) and DMARDs (leflunomide (5 mg/kg) or salazosulfapyridine(400 mg/kg)) which were prepared so that the dose was 0.5 ml per 100g body weight was orally administered singly or in combination once a day for consecutive five days from the 17th day after the injection of the adjuvant. The volume of the left hind paw was measured by water replacement method just before the administration and in five hours after the last administration and the difference was calculated and assumed as the changes in the edema volume. 0.5% methylcellulose alone which

did not contain the test compound was also administered as a control in the same way as above.

(Results)

Arthritis was developed in the left hind paw (non-injected paw) at about 10 days after adjuvant injection, and the paw volume increased to reach the maximum at about 17 days after adjuvant injection. Therefore, changes in the edema volume when Compound (I) and leflunomide or salazosulfapyridine as a representative example of DMARDs were orally administered in combination was shown in Figs. 25 and 26. The results of the group in which only Compound (I) was administered, the groups in which only DMARDs were administered and the control group were also shown in each of the drawings for the purpose of comparison.
As shown in Figs. 25 and 26, remarkable edema suppressing effect was observed in the group in which Compound (I) and DMARDs were administered in combination as compared with the group in which only Compound (I) was administered and the groups in which only DMARDs were administered in the test in which the administration was started after the edema in the left hind paw caused by adjuvant injection reached the maximum.

Example 18

Example 18 Combination effect on the adjuvant-induced arthritis

Combination effect with Compound (I) and DMARDs (methotrexate) was examined in the same way as in Example 17.

(Method)

Administration of a DMARD (methotrexate (0.060 mg/kg or 0.045 mg/kg; 0.5% methylcellulose suspension)) to the rat in which adjuvant-induced arthritis was caused in the same way as in Example 17 was started on the day when the adjuvant was injected and continued once a day for consecutive 21 days. Compound (I) (10 mg/kg) was orally administered singly or in combination once a day for consecutive 12 days from the 10 days after the sensitization. The volume of the left hind paw was measured by water replacement method every other day. 0.5% methylcellulose alone which did not contain the test compound was also administered as a control in the same way as above.

(Results)

The volume of the left hind paw when methotrexate (0.060 mg/kg, 0.045 mg/kg) as a representative example of DMARDs was administered from the day when the adjuvant was injected and Compound (I) (10 mg/kg) was administered together from the 10 days are shown in Fig. 27 and Fig. 29, and the onset ratio of the left hind paw are shown in Fig. 28 and Fig. 30 respectively. The results of the group in which only Compound (I) was administered, the groups in which only DMARDs were administered and the control group were also shown in each of the drawings for the purpose of comparison.
As shown in Fig. 27 and Fig. 28, the group in which Compound (I) and DMARDs were administered in combination showed remarkable onset suppressing effect and edema suppressing effect as compared with onset suppressing effect and edema suppressing effect of the groups in which only DMARDs were administered even in the case where the group in which only Compound (I) was administered showed very little effect in the test in which the administration of DMARDs was started before the edema in the left hind paw emerged. Furthermore, as shown in Fig. 29 and Fig. 30, the combined administration group showed remarkable edema suppressing effect even when a dose of DMARDs which showed little effect in the groups in which only DMARDs were administered and Compound (I) was administered in combination.

Example 19

Example 19 Combination effect on the adjuvant-induced arthritis

Combination effect for rat adjuvant-induced arthritis was examined in the same manner as in Example 17.

(Method)

Arthritis was caused by the same method as in Example 17, and 0.5% methylcellulose suspension of Compound (I) and methotrexate as a representative example of DMARDs which were prepared so that the dose was 0.5 ml per

100g body weight was orally administered singly or in combination once a day for consecutive 17 days from the day when the adjuvant was injected. The volume of the left hind paw was measured by water replacement method just before the administration and after the last administration, and AUC (the area under the curve formed by plotting the difference of the value at the time of grouping from the value of each time point during the administration period against the time) of the increased volume of the left hind paw was determined. Joint destruction of the right hind paw (injected paw) and the left hind paw (non-injection paw) can be measured by X-ray and this can be also used as the index to evaluate the combination effect.

(Results)

The changes in the edema volume (increased volume of the left hind paw, shown by the difference between the volume at the time point and the volume at the time of grouping) and the onset ratio of the left hind paw when Compound (I) (20 mg/kg) and methotrexate (0. 060 mg/kg) were administered in combination were shown in Fig. 31 and Fig. 32. Likewise, the results of the case where Compound (I) (40 mg/kg) and methotrexate (0.060 mg/kg) were administered in combination were shown in Fig. 33 and Fig. 34, and the results of the case where Compound (I) (80 mg/kg) and methotrexate (0.060 mg/kg) were administered in combination were shown in Fig. 35 and Fig. 36. The results of the group in which only Compound (I) was administered and the group in which only methotrexate was administered and results of the control group were also shown in each of the drawings for the purpose of comparison. Furthermore, each dose and AUC (area under the curve formed by plotting the increased volume at each time point during the administration period against the time) of the increased volume of the left hind paw for the single administration and combined administration were summarized and shown in Fig. 37.
As shown in Fig. 31 and Fig. 32, the group in which only Compound (I) (20 mg/kg) was administered showed edema suppressing effect, and the group in which only methotrexate (0.060 mg/kg) was administered showed onset suppressing effect and edema suppressing effect, whereas the group in which Compound (I) (20 mg/kg) and methotrexate were administered in combination showed remarkable edema suppressing effect as compared with each of the single substance administered groups in the test in which the administration was started on the day when the adjuvant was injected.
As shown in Fig. 33 to Fig. 36, each of the group in which only Compound (I) (40 mg/kg and 80 mg/kg) was administered and the group in which only methotrexate (0.060 mg/kg) was administered showed onset suppressing effect and edema suppressing effect, but in this case, the group in which Compound (I) (40 mg/kg and 80 mg/kg) and methotrexate (0.060 mg/kg) were administered in combination showed remarkable onset suppressing effect and edema suppressing effect as compared with any of the group in which only Compound (I) was administered at each dose and the group in which only methotrexate was administered. Furthermore, as shown in Fig. 37, it has been revealed that when AUC of the increased volume of the left hind paw was used as the index, the group in which Compound (I) (20, 40 and 80 mg/kg) and methotrexate (0.060 mg/kg) were administered in combination showed remarkable edema suppressing effect as compared with single substance administered groups at each dose.

Example 20

Example 20 Combination effect on the adjuvant-induced arthritis (Suppressing effect on joint destruction)

Combination effect for adjuvant-induced arthritic rats was examined in the same manner as in Example 17.

(Method)

Arthritis was caused by the same method as in Example 17, and 0.5% methylcellulose suspension of Compound (I) and methotrexate as a representative example of DMARDs which were prepared so that the dose was 0.5 ml per 100 g body weight was orally administered singly or in combination once a day for consecutive 17 days from the day when the adjuvant was injected. Euthanasia was carried out for all the rats by aspiration of high concentration carbon dioxide after the last administration. Both the hind paws were cut off at the middle point of cervical vertebra. Both the hind paws were fixed with 10% neutral buffered formalin liquid. Soft X-ray photographs were taken and the structure of the joint part was observed with a microscope. The degree of joint destruction was recorded with a score. Observed sites were three points of the tibia, calcaneum and tarsal bone, and the possible maximum score was 12.

Joint destruction score

0: Normal

1: Minimum destruction of joints and bones and new formation of cartilages
2: Mild destruction of joints and bones and new formation and light ossification of cartilages
3: Moderate destruction of joints and bones and ossification
4: Severe destruction and deformation of joints and bones and intense ossification

(Results)

Destruction scores of the right hind paw joints in the single and combined administrations at each dose (20, 40 and 80 mg/kg for Compound (I), 0.06 mg/kg for methotrexate per day) were summarized and shown in Fig. 38. In addition, soft X-ray photographs of the right hind paws of arthritic rats for the four groups of the control group (Fig. 39), the group in which only methotrexate (0.06 mg/kg, Fig. 40) was administered, the group in which only Compound (I) (40 mg/kg, Fig. 41) was administered and the group in which Compound (I) (40 mg/kg) + methotrexate (0.06 mg/kg) were administered in combination (Fig. 42) were shown by representative examples of the individual which showed average score in each group.
Likewise, destruction scores of the left hind paw joints in the single and combined administrations at each dose were summarized and shown in Fig. 43. In addition, soft X-ray photographs of the left hind paws of arthritic rats for the four groups of the control group (Fig. 44), the group in which only methotrexate (0.06 mg/kg, Fig. 45) was administered, the group in which only Compound (I) (40 mg/kg, Fig. 46) was administered and the group in which Compound (I) (40 mg/kg) + methotrexate (0.06 mg/kg) were administered in combination (Fig. 47) were shown by representative examples of the individual which showed average score in each group.
As shown in Fig. 38 to Fig. 47, the group in which only Compound (I) was administered dose-dependently showed joint destruction suppressing effect for both of the right and left hind paws in the test in which the administration was started on the day when the adjuvant was injected. Furthermore, Compound (I) dose-dependently and conspicuously enhanced joint destruction suppressing effect of methotrexate for the right hind paw and the left hind paw by administering methotrexate (0.06 mg/kg) in combination. Among all, the group in which Compound (I) (80 mg/kg) + methotrexate (0.06 mg/kg) were administered in combination showed an extremely high combination effect completely suppressing joint destruction of the left hind paw.
As demonstrated above, it has been revealed that the combined administration of Compound (I) and methotrexate resulted in conspicuously excellent joint destruction suppressing effect as compared with each of the single substance administration and that the combined use showed remarkable utility in the prophylaxis and/or treatment of rheumatoid arthritis.
The combination effect of Compound (I) and DMARDs other than methotrexate can be confirmed by performing combined administration tests in the same manner as in the above described Examples.

Example 21

<u>Example 21 Combination effect on T cells</u>

Combination effect of Compound (I) and methotrexate on IL-2 production when T-lymphocytes were stimulated with Phorbol 12-myristate 13-acetate (PMA) and Calcium Ionophore A23187 were evaluated.

(Method)

EL-4 cells (mouse T-lymphocyte cell line) were cultured in Dulbecco's Modified Eagle Medium (DMEM: GIBCO, Inc.) containing 10% fetal bovine serum and subjected to the experiment. The cells were disseminated in 96-well plate in a ratio of $5\times10^4$ cells/well and a DMEM medium containing the test substance and a DMEM medium containing PMA and A23187 as a stimulant were added and incubated for three days. The test compound was each single agent of Compound (I) and methotrexate and combination thereof, and they were stepwisely diluted and adjusted so that the addition ratio at the time of combination was Compound (1) to methotrexate was 10:1. The concentration of the stimulants was adjusted so that the final concentration was 25 nM for PMA and 250 ng/ml for A23187. The plate was centrifuged after incubation and the supernatant was collected with micropipette. The concentration of IL-2 in the supernatant was quantified by Enzyme-linked Immunosorbent Assay (ELISA) method. The quantitative method was carried out in accordance with the instructions attached to the kit (MOUSE IL-2 Immunoassay, R & D Systems).

(Calculation method of Combination Index)

Combination Index (CI) was calculated by the following method.

$$fa/f = (D/Dm)m \cdots \text{Formula (F1)}$$

$$fa: \text{Inhibition rate by a drug } (fa = 1-f) \cdots \text{Formula (F2)}$$

D: Drug concentration
Dm: $IC_{50}$
m: Parameter of drug concentration - curve

From Formula (F1) and Formula (F2),

$$(1-f)/f = (D/Dm)m \cdots \text{Formula (F3)}$$

From Formula (F3),

$$D = Dm \times ((1-f)/f)\ 1/m$$
$$= Dm \times (fa/(1-fa))\ 1/m \cdots \text{Formula (F4)}$$

D for each was determined using Formula (F4) from the results of the experiments at the time of treatment by single agent of Compound (I) and methotrexate (MTX) and combined treatment thereof.

$$CI = Dc1/Df1 + Dc2/Df2 + (Dc1 \times Dc2)/(Df1 \times Df2) \cdots \text{Formula (F5)}$$

D1: Concentration of Compound (I)
D2: Concentration of methotrexate
Subscript f: Single agent
Subscript c: Combination

CI was determined by Formula (F5) and evaluated with CI at fa =0.8.
CI <1 shows synergetic effect, CI =1 shows additive effect and CI >1 shows antagonism.

(Results)

As shown in Fig. 48, CI value at fa = 0.8 (80% inhibition concentration) was 0.368 (95 % confidence interval: 0.144 to 0.619) determined by using The SAS system Release 8.2 (SAS Institute Inc.). Thus, it has been shown that the suppression effect on the IL-2 production from T cells when Compound (I) and methotrexate were used in combination was significantly synergistic (critical rate 5%).

Example 22

Example 22 Combination effect on rheumatic patients

In the treatment by methotrexate, Compound (I) in addition to methotrexate is orally administered once a day after a meal to a rheumatoid arthritis patient for whom methotrexate is ineffective or the effect thereof is considered to be insufficient. The dose of methotrexate may be the same as previous treatment or selected to be in the range of 6 mg to 25 mg per week according to the condition of the patient. The dose of Compound (I) is placebo, 80 mg/day and 240 mg/day (about 100 cases for each group). The administration period is 48 weeks but the persons who do not achieve

the predetermined effective criteria in the 24th week (the predetermined effective criteria are considered to be satisfied when 20% or more improvement is recognized in any of tender joint count, swollen joint count or erythrocyte sedimentation) are excluded. The persons who satisfy the criteria continue to be dosed until 48th week while the blind test is maintained. The effectiveness (enhancing effect by Compound (I) on the therapeutic effect of rheumatoid arthritis by methotrexate) by the combined administration of Compound (1) and methotrexate can be evaluated by comparing the rate of patients satisfying "20% improvement according to the criteria by American College of Rheumatology (ACR criteria)" among the groups of placebo, 80 mg/day and 240 mg/day in 16 weeks or 24 weeks after the administration was started. In the Examples of this specification, 3) Global assessment of disease activity by the patient, 4) Global assessment of disease activity by medical doctor in charge and 5) Assessment of pain by the patient with ACR criteria can be evaluated by visual analog scale (VAS). "VAS (visual analog scale), 100 mm" is an index evaluated by the evaluator who indicates the evaluation on a line from 0 to 100 mm, and it is expressed by an integer from 0 to 100. The length of the line may be other than 100 mm (for example, 200 mm), but VAS (visual analog scale) in this specification refers to one scored in the range from 0 to 100.

Example 23

Example 23 Combination effect on rheumatic patients

In the treatment by methotrexate, Compound (I) in addition to methotrexate is orally administered once a day after a meal to a rheumatoid arthritis patient for whom methotrexate is ineffective or the effect thereof is considered to be insufficient. The dose of methotrexate may be the same as previous treatment or selected to be in the range of 10 mg to 25 mg per week according to the condition of the patient. The administration period is 48 weeks and Compound (I) is administered in a dose of placebo, 120 mg/day and 240 mg/day (about 100 cases for each group, about 300 cases in total) for the first 24 weeks under double blind condition. For the next 24 weeks, 240 mg/day is administered to all the subjects under non-blind condition (open test). The effectiveness (enhancing effect by Compound (I) on the therapeutic effect of rheumatoid arthritis by methotrexate) by the combined administration of Compound (1) and methotrexate can be evaluated by comparing the rate of patients satisfying "20% improvement according to the criteria by American College of Rheumatology (ACR criteria)" among the groups of placebo, 120 mg/day and 240 mg/day in 24 weeks after the administration was started.

Example 24

Example 24 Combination effect on rheumatic patients (Suppressing effect on joint destruction)

Compound (I) in addition to methotrexate is orally administered once a day after a meal to a rheumatoid arthritis patient for whom methotrexate is ineffective or the effect thereof is considered to be insufficient in the treatment history by methotrexate. The dose of methotrexate may be the same as previous treatment or selected to be in the range of 6 mg to 25 mg per week according to the condition of the patient. The dose of Compound (I) is placebo, 80 mg/day and 240 mg/day (about 100 cases for each group). The administration period is 48 weeks but the persons who do not achieve the predetermined effective criteria in the 24th week (the predetermined effective criteria are considered to be satisfied when 20% or more improvement is recognized in any of tender joint count, swollen joint count or erythrocyte sedimentation) are excluded. The persons who satisfy the criteria continue to be dosed until 48th week while the blind test is maintained. Joint destruction suppressing effect is evaluated by evaluating the progress of joint destruction at hands and feet by X-ray image in 48 weeks after the administration was started. The effectiveness (enhancing effect by Compound (I) on the therapeutic effect of rheumatoid arthritis by methotrexate) by the combined administration of Compound (1) and methotrexate can be evaluated by comparing the effect among the groups of placebo, 80 mg/day and 240 mg/day.

Example 25

Example 25 Combination effect on rheumatic patients (Suppressing effect on joint destruction)

Compound (I) in addition to methotrexate is orally administered once a day after a meal to a rheumatoid arthritis patient for whom methotrexate is ineffective or the effect thereof is considered to be insufficient in the treatment history by methotrexate. The dose of methotrexate may be the same as previous treatment or selected to be in the range of 10 mg to 25 mg per week according to the condition of the patient. The administration period is 48 weeks and Compound (I) is administered in a dose of placebo, 120 mg/day and 240 mg/day (about 100 cases for each group, about 300 cases in total) for the first 24 weeks under double blind condition. For the next 24 weeks, 240 mg/day is administered to all

the subjects under non-blind condition (open test). Joint destruction suppressing effect is evaluated by evaluating the progress of joint destruction at hands and feet by X-ray image in 24 weeks and 48 weeks after the administration was started. The effectiveness (enhancing effect by Compound (I) on the therapeutic effect of rheumatoid arthritis by methotrexate) by the combined administration of Compound (1) and methotrexate can be evaluated by comparing the effect among the groups of placebo, 120 mg/day and 240 mg/day.

It has been revealed from Examples (Examples 17 to 25) described above that conspicuously excellent prophylactic effect and/or therapeutic effect on autoimmune diseases by combined use of a CaMKII inhibitor (for example, Compound (I), etc.) and a DMARDS (for example, methotrexate, salazosulfapyridine, leflunomide, etc.), which cannot be anticipated from independent effect of each agent, can be achieved, that is, combined use of a CaMKII inhibitor and a DMARD has remarkable usefulness in the prophylaxis and/or treatment of autoimmune diseases.
According to the results of repeated administration test separately conducted on rat and human, it has been found that the dose of 5 mg/kg (once a day, oral administration) for rat corresponds to the dose of 14.2 to 18.8 mg/person (once a day, oral administration) for human in order that the blood concentration (AUC) of Compound (I) may be equivalent. Therefore, it is considered that the dose of Compound (I) per day to a rat in Examples above, 10 to 80 mg/kg, corresponds to about 28 to about 300 mg/person as a dose per day to a human.

INDUSTRIAL APPLICABILITY

According to the present invention, an excellent combination drug for preventing and/or treating autoimmune diseases can be provided. Furthermore, according to the present invention, a screening process useful in searching for and obtaining candidate substances which can be an active ingredient of the combination drug by the present invention can be provided.
Free text for sequence listing
SEQ ID NO 1: Inhibitor peptide
SEQ ID NO 2: Inhibitor peptide
SEQ ID NO 3: Inhibitor peptide
SEQ ID NO 4: Primer
SEQ ID NO 5: Primer
SEQ ID NO 6: siRNA
SEQ ID NO 7: siRNA
SEQ ID NO 8: siRNA
SEQ ID NO 9: siRNA
SEQ ID NO 10: siRNA
SEQ ID NO 11: siRNA
SEQ ID NO 12: PLP(139-151)

## Claims

1. A prophylactic agent and/or therapeutic agent for an autoimmune disease comprising a CaMKII inhibitor and a disease modifying antirheumatic drug.

2. A prophylactic agent and/or therapeutic agent for an autoimmune disease comprising a CaMKII inhibitor for use in combination with a disease modifying antirheumatic drug.

3. The prophylactic agent and/or therapeutic agent according to claim 2, wherein the prophylactic agent and/or therapeutic agent is administered simultaneously with, separately from, or subsequent to administration of the disease modifying antirheumatic drug.

4. A prophylactic agent and/or therapeutic agent for an autoimmune disease comprising a CaMKII inhibitor, which is intended to be administered simultaneously with, separately from, or subsequent to administration of a disease modifying antirheumatic drug to a mammal with an autoimmune disease to which the disease modifying antirheumatic drug in an amount giving a sufficient prophylactic and/or therapeutic effect on the autoimmune disease cannot be administered due to a side effect.

5. The prophylactic agent and/or therapeutic agent according to claim 4, wherein the side effect is one or several side effects selected from hepatic disorder, renal disorder, and gastrointestinal disorder.

6. The prophylactic agent and/or therapeutic agent according to any of claims 1 to 5, wherein the CaMKII inhibitor is

an isoxazole derivative represented by Formula 1 or a pharmaceutically acceptable salt thereof:
Formula 1:

[Formula 1]

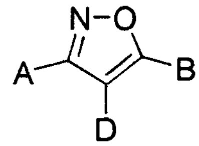

[wherein D represents a hydrogen atom, a halogen atom, a hydroxy group, a mercapto group, a nitro group, a cyano group, a carboxy group, an amino group which may be substituted, a hydroxylamino group which may be substituted, a carbamoyl group which may be substituted, a sulfamoyl group which may be substituted, a sulfo group, $-R^5$, $-OR^5$, $-CO_2R^6$, $-SR^7$, $-(CO)SR^7$, $-(CS)OR^7$, or $-CS_2R^7$ (wherein $R^5$ represents an alkyl group which may be substituted, an alkenyl group which may be substituted, an alkynyl group which may be substituted, a cycloalkyl group which may be substituted, a cycloalkenyl group which may be substituted, an aryl group which may be substituted, a heterocyclic group which may be substituted, or an acyl group; $R^6$ represents an alkyl group which may be substituted, an alkenyl group which may be substituted, an alkynyl group which may be substituted, an aryl group which may be substituted, or a heterocyclic group which may be substituted; $R^7$ represents an alkyl group which may be substituted or an aryl group which may be substituted);
one of A and B represents a group represented by Formula:

[Formula 2]

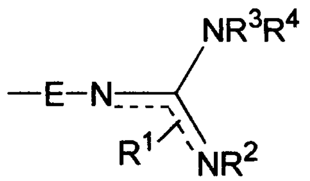

(E represents a single bond or an alkylene group;
one of two broken lines denotes a double bond together with a solid line, and the other denotes a single bond together with a solid line; $R^1$ binds to a nitrogen atom binding to the bond in which the broken line denotes the single bond together with the solid line;
$R^1$, $R^2$, $R^3$ and $R^4$ each independently represent a hydrogen atom, a halogen atom, a hydroxy group, a mercapto group, a nitro group, a cyano group, a carboxy group, an amino group which may be substituted, a hydroxylamino group which may be substituted, a carbamoyl group which may be substituted, a sulfamoyl group which may be substituted, a sulfo group, a protecting group for an NH group, $-R^5$, $-OR^5$, $-CO_2R^6$, $-SR^7$, $-(CO)SR^7$, $-(CS)OR^7$, or $-CS_2R^7$ (wherein $R^5$, $R^6$, and $R^7$ represent the same as above); or any two of $R^1$, $R^2$, $R^3$, and $R^4$ may bind together and form, together with a nitrogen atom, a heterocyclic ring which may be substituted; or Formula: $-NR^3R^4$ may represent a group represented by Formula: $-N=C(NH_2)NR^{43}R^{44}$;
$R^{43}$ and $R^{44}$ represent the following (1) or (2):

(1) $R^{43}$ and $R^{44}$ each independently represent a hydrogen atom, an alkyl group having 1 to 4 carbon atoms, $-(CH_2)_n-COCH_3$ (n represents an integer of 1 to 3) , $-(CH_2)_n-CO_2R^{32}$ (n represents the same as above; $R^{32}$ represents an alkyl group having 1 to 3 carbon atoms), $-(CH_2)_n-CONR^{33}R^{14}$ (n represents the same as above; $R^{33}$ and $R^{34}$ each independently represent a hydrogen atom or an alkyl group having 1 to 3 carbon atoms), $-(CH_2)_m-OR^{35}$ (m represents 2 or 3; $R^{35}$ represents a hydrogen atom, an alkyl group having 1 to 3 carbon atoms, or $-(CH_2)_m-OR^{36}$ (m represents the same as above; $R^{36}$ represents a hydrogen atom or an alkyl group having 1 to 3 carbon atoms)), $-(CH_2)_m-NR^{37}R^{38}$ (m represents the same as above; $R^{37}$ and $R^{38}$ each independently represent a hydrogen atom or an alkyl group having 1 to 3 carbon atoms, or together represent pyrrolidine, piperidine, azepane, morpholine, or N-methylpiperazine (wherein the pyrrolidine, piperidine,

azepane, morpholine, and N-methylpiperazine may be substituted by one or two methyl groups) together with a nitrogen atom), phenyl, pyridyl, pyrimidinyl, pyridazinyl, pyrazinyl, tetrazolyl, benzyl, pyridylmethyl, pyrimidinylmethyl, pyridazinylmethyl, pyrazinylmethyl, tetrazolylmethyl, a hydroxy group, an alkoxy group having 1 to 3 carbon atoms, or $-NR^{39}R^{40}$ ($R^{39}$ and $R^{40}$ each independently represent a hydrogen atom, an alkyl group having 1 to 3 carbon atoms, phenyl, or pyridyl); and

(2) $R^{43}$ and $R^{44}$ together represent a 5- to 7-membered saturated nitrogen-containing heterocyclic group (the 5- to 7-membered saturated nitrogen-containing heterocyclic group may be substituted by one or two substituents optionally selected from the group consisting of an alkyl group, an amino group, a hydroxy group, an alkoxy group, and an oxo group) together with a nitrogen atom);

the other of A and B represents a group represents by Formula: -J-G (wherein G represents an aryl group which may be substituted or a heterocyclic group which may be substituted; J represents $-C(R^8R^9)$ - or $-C(=CR^8R^9)-$; $R^8$ and $R^9$ each independently represent a hydrogen atom, a lower alkoxy group which may be substituted, or a lower alkyl group which may be substituted; or $R^8$ and $R^9$ may bind together and form, together with a carbon atom, a hydrocarbon ring which may be substituted, a 1,3-dioxane ring which may be substituted, or a 1,3-dioxolane ring which may be substituted)].

**7.** The prophylactic agent and/or therapeutic agent according to claim 6, wherein E is a single bond or a lower alkylene group.

**8.** The prophylactic agent and/or therapeutic agent according to claim 6 or 7, wherein D is a hydrogen atom, a nitro group, a cyano group, a carboxy group, an amino group which may be substituted, a hydroxylamino group which may be substituted, a carbamoyl group which may be substituted, $-R^5$, or $-CO_2R^6$ (wherein $R^5$ and $R^6$ represent the same as in claim 6).

**9.** The prophylactic agent and/or therapeutic agent according to claim 8, wherein D is a hydrogen atom, a carboxy group, $-R^5$, or $-CO_2R^6$ (wherein $R^5$ and $R^6$ represent the same as in claim 6).

**10.** The prophylactic agent and/or therapeutic agent according to any of claims 6 to 9, wherein $R^1$, $R^2$, $R^3$, and $R^4$ each independently represent a hydrogen atom, a hydroxy group, an amino group which may be substituted, a hydroxylamino group which may be substituted, an alkoxy group which may be substituted, an alkyl group which may be substituted, an alkenyl group which may be substituted, an alkynyl group which may be substituted, a cycloalkyl group which may be substituted, a cycloalkenyl group which may be substituted, an aryl group which may be substituted, a heterocyclic group which may be substituted, or an acyl group; or Formula: $-NR^3R^4$ represents a group represented by Formula: $-N=C(NH_2)NR^{43}R^{44}$ ($R^{43}$ and $R^{44}$ represent the same as in claim 6); or any two of $R^1$, $R^2$, $R^3$, and $R^4$ bind together and form, together with a nitrogen atom, a heterocyclic ring which may be substituted.

**11.** The prophylactic agent and/or therapeutic agent according to claim 10, wherein $R^1$, $R^2$, $R^3$, and $R^4$ each independently represent a hydrogen atom, a hydroxy group, an amino group which may be substituted, a hydroxylamino group which may be substituted, an alkoxy group which may be substituted, an alkyl group which may be substituted, an alkenyl group which may be substituted, an alkynyl group which may be substituted, a cycloalkyl group which may be substituted, an aryl group which may be substituted, a heterocyclic group which may be substituted, or an acyl group; or Formula: $-NR^3R^4$ represents a group represented by Formula: $- N=C (NH_2) NR^{43}R^{44}$ ($R^{43}$ and $R^{44}$ represent the same as in claim 6); or any two of $R^1$, $R^2$, $R^3$. and $R^4$ bind together and form, together with a nitrogen atom, a heterocyclic ring which may be substituted.

**12.** The prophylactic agent and/or therapeutic agent according to any of claims 6 to 11, wherein G is phenyl which may be substituted, naphthyl which may be substituted, furyl which may be substituted, thienyl which may be substituted, indolyl which may be substituted, isothiazolyl which may be substituted, benzothienyl which may be substituted, isobenzofuranyl which may be substituted, pyrrolyl which may be substituted, benzofuryl which may be substituted, imidazolyl which may be substituted, pyrazolyl which may be substituted, isoxazolyl which may be substituted, isothiazolyl which may be substituted, thiazolyl which may be substituted, oxazolyl which may be substituted, benzimidazolyl which may be substituted, benzothiazolyl which may be substituted, benzoxazolyl which may be substituted, pyridyl which may be substituted, pyrazinyl which may be substituted, pyrimidinyl which may be substituted, pyridazinyl which may be substituted, triazinyl which may be substituted, quinolyl which may be substituted, isoquinolyl which may be substituted, quinazolinyl which may be substituted, quinoxalinyl which may be substituted, 2,3-dihydro-benzo[1,4]dioxinyl which may be substituted, or carbazolyl which may be substituted.

**13.** The prophylactic agent and/or therapeutic agent according to claim 6, wherein the CaMKII inhibitor is an isoxazole derivative represented by Formula A1 or A2 or a pharmaceutically acceptable salt thereof:
Formula A1:

[Formula 3]

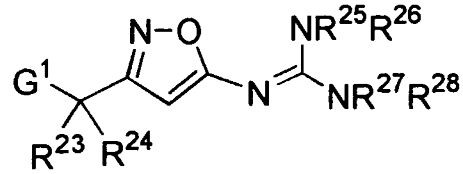

[wherein $G^1$ represents phenyl, biphenyl-4-yl, 3-benzoylphenyl, 4-benzoylphenyl, 1H-indol-2-yl, 1H-indol-3-yl, 1-methyl-1H-indol-2-yl, 1-methyl-1H-indol-3-yl, 2,3-dihydro-benzo[1,4]dioxin-6-yl, 1-benzofuran-5-yl, 1-benzofuran-6-yl, quinolyl, isoquinolyl, phenylpyridyl, phenylpyrimidinyl, phenylpyridazinyl, or phenylpyrazinyl (wherein the phenyl, biphenyl-4-yl, 3-benzoylphenyl, 4-benzoylphenyl, 1H-indol-2-yl, 1H-indol-3-yl, 1-methyl-1H-indol-2-yl, 1-methyl-1H-indol-3-yl, 2, 3-dihydro-benzo[1,4]dioxin-6-yl, 1-benzofuran-5-yl, 1-benzofuran-6-yl, quinolyl, isoquinolyl, phenylpyridyl, phenylpyrimidinyl, phenylpyridazinyl, and phenylpyrazinyl may be substituted by one or two groups optionally selected from the group consisting of a fluorine atom, a chlorine atom, a bromine atom, acetyl, cyano, $-CO_2R^{29}$ ($R^{29}$ represents an alkyl group having 1 to 3 carbon atoms), and $-CONR^{30}R^{31}$ ($R^{30}$ and $R^{31}$ each independently represent a hydrogen atom or an alkyl group having 1 to 3 carbon atoms));
$R^{23}$ and $R^{24}$ each independently represent a hydrogen atom, an alkyl group having 1 to 4 carbon atoms, methoxy, or ethoxy, or together represent methylene;
Formula: $=C(NR^{25}R^{26})\ NR^{27}R^{28}$ represents the following (1), (2), or (3):

(1) $R^{25}$ and $R^{26}$ represent the following (a) or (b) and $R^{27}$ and $R^{28}$ represent the following (c) or (d):

(a) $R^{25}$ and $R^{26}$ each independently represent a hydrogen atom, an alkyl group having 1 to 4 carbon atoms, $-(CH_2)_n-COCH_3$ (n represents the same as in claim 6 ), $-(CH_2)_n-CO_2R^{32}$ (n and $R^{32}$ represent the same as in claim 6), $-(CH_2)_n-CONR^{33}R^{34}$ (n, $R^{33}$, and $R^{34}$ represent the same as in claim 6), $-(CH_2)_m-OR^{35}$ (m and $R^{35}$ represent the same as in claim 6), $-(CH_2)_m-NR^{37}R^{38}$ (m, $R^{37}$, and $R^{38}$ represent the same as in claim 6), phenyl, pyridyl, pyrimidinyl, pyridazinyl, pyrazinyl, tetrazolyl, benzyl, pyridylmethyl, pyrimidinylmethyl, pyridazinylmethyl, pyrazinylmethyl, tetrazolylmethyl, a hydroxy group, an alkoxy group having 1 to 3 carbon atoms, or $-NR^{39}R^{40}$ ($R^{39}$ and $R^{40}$ represent the same as in claim 6);
(b) $R^{25}$ and $R^{26}$ together represent a 5- to 7-membered saturated nitrogen-containing heterocyclic group (wherein the 5- to 7-membered saturated nitrogen-containing heterocyclic group may be substituted by one or two substituents optionally selected from the group consisting of an alkyl group, an amino group, a hydroxy group, an alkoxy group, and an oxo group) together with a nitrogen atom;
(c) $R^{27}$ and $R^{28}$ each independently represent a hydrogen atom, an alkyl group having 1 to 4 carbon atoms, $-(CH_2)_n-COCH_3$ (n represents the same as above), $-(CH_2)n-CO_2R^{32}$ (n and $R^{32}$ represent the same as above), $-(CH_2)_n-CONR^{33}R^{34}$ (n, $R^{33}$ , and $R^{34}$ represent the same as above), $-(CH_2)_m-OR^{35}$ (m and $R^{35}$ represent the same as above), $-(CH_2)_m-NR^{37}R^{38}$ (m, $R^{37}$, and $R^{38}$ represent the same as above), phenyl, pyridyl, pyrimidinyl, pyridazinyl, pyrazinyl, tetrazolyl, benzyl, pyridylmethyl, pyrimidinylmethyl, pyridazinylmethyl, pyrazinylmethyl, tetrazolylmethyl, a hydroxy group, an alkoxy group having 1 to 3 carbon atoms, or $-NR^{39}R^{40}$ ($R^{39}$ and $R^{40}$ represent the same as above); and
(d) $R^{27}$ and $R^{28}$ together represent a 5- to 7-membered saturated nitrogen-containing heterocyclic group (wherein the 5- to 7-membered saturated nitrogen-containing heterocyclic group may be substituted by one or two substituents optionally selected from the group consisting of an alkyl group, an amino group, a hydroxy group, an alkoxy group, and an oxo group) together with a nitrogen atom;

(2) $R^{26}$ and $R^{27}$ together represent a 5- to 7-membered saturated nitrogen-containing heterocyclic group (wherein the 5- to 7-membered saturated nitrogen-containing heterocyclic group may be substituted by one or two substituents optionally selected from the group consisting of an alkyl group, an amino group, a hydroxy group, an alkoxy group, and an oxo group) together with two nitrogen atoms and a carbon atom;

$R^{25}$ and $R^{28}$ each independently represent a hydrogen atom, an alkyl group having 1 to 3 carbon atoms, acetyl, or - $(CH_2)_m$ -$OR^{36}$ (m represents the same as above; $R^{36}$ represents the same as in claim 6); and
(3) Formula: =C ($NR^{25}R^{26}$) $NR^{27}R^{28}$ represents Formula: =C ($NR^{41}R^{42}$) N=C ($NH_2$) $NR^{43}R^{44}$;
$R^{41}$ and $R^{42}$ represent the following ($a^1$) or ($b^1$) and $R^{43}$ and $R^{44}$ represent the following ($C^1$) or ($d^1$):

($a^1$) $R^{41}$ and $R^{42}$ each independently represent a hydrogen atom, an alkyl group having 1 to 4 carbon atoms, $-(CH_2)_n-COCH_3$ (n represents the same as above), $-(CH_2)_n-CO_2R^{32}$ (n and $R^{32}$ represent the same as above),- $(CH2)_n-CONR^{33}R^{34}$ (n, $R^{33}$, and $R^{34}$ represent the same as above), $-(CH_2)_m-OR^{35}$ (m and $R^{35}$ represent the same as above), - $(CH_2)_m-NR^{37}R^{38}$ (m, $R^{37}$, and $R^{38}$ represent the same as above), phenyl, pyridyl, pyrimidinyl, pyridazinyl, pyrazinyl, tetrazolyl, benzyl, pyridylmethyl, pyrimidinylmethyl, pyridazinylmethyl, pyrazinylmethyl, tetrazolylmethyl, a hydroxy group, an alkoxy group having 1 to 3 carbon atoms, or $-NR^{39}R^{40}$ ($R^{39}$ and $R^{40}$ represent the same as above);
($b^1$) $R^{41}$ and $R^{42}$ together represent a 5- to 7-membered saturated nitrogen-containing heterocyclic group (wherein the 5- to 7-membered saturated nitrogen-containing heterocyclic group may be substituted by one or two substituents optionally selected from the group consisting of an alkyl group, an amino group, a hydroxy group, an alkoxy group, and an oxo group) together with a nitrogen atom;
($C^1$) $R^{43}$ and $R^{44}$ each independently represent a hydrogen atom, an alkyl group having 1 to 4 carbon atoms, $-(CH_2)_n-COCH_3$ (n represents the same as above),$-(CH_2)_n-CO_2R^{32}$ (n and $R^{32}$ represent the same as above), $-(CH_2)_n-CONR^{33}R^{34}$ (n, $R^{33}$, and $R^{34}$ represent the same as above), - $(CH_2)_m-OR^{35}$ (m and $R^{35}$ represent the same as above), $-(CH_2)_m-NR^{37}R^{38}$ (m, $R^{37}$, and $R^{38}$ represent the same as above), phenyl, pyridyl, pyrimidinyl, pyridazinyl, pyrazinyl, tetrazolyl, benzyl, pyridylmethyl, pyrimidinylmethyl, pyridazinylmethyl, pyrazinylmethyl, tetrazolylmethyl, a hydroxy group, an alkoxy group having 1 to 3 carbon atoms, or $-NR^{39}R^{40}$ ($R^{39}$ and $R^{40}$ represent the same as above); and
($d^1$) $R^{43}$ and $R^{44}$ together represent a 5- to 7-membered saturated nitrogen-containing heterocyclic group (wherein the 5- to 7-membered saturated nitrogen-containing heterocyclic group may be substituted by one or two substituents optionally selected from the group consisting of an alkyl group, an amino group, a hydroxy group, an alkoxy group, and an oxo group) together with a nitrogen atom];
Formula A2:

[Formula 4]

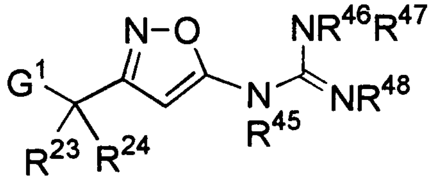

[wherein $G^1$, $R^{23}$, and $R^{24}$ represent the same as above;
Formula: $-N(R^{45})$ C($NR^{46}R^{47}$) =$NR^{48}$ represents the following ($1^1$) or ($2^1$) :

($1^1$) $R^{45}$ represents an alkyl group having 1 to 3 carbon atoms or acetyl. $R^{48}$ represents a hydrogen atom, an alkyl group having 1 to 3 carbon atoms, or acetyl;
$R^{46}$ and $R^{47}$ represent the following ($a^2$) or ($b^2$) :

($a^2$) $R^{46}$ and $R^{47}$ each independently represent a hydrogen atom, an alkyl group having 1 to 4 carbon atoms, $-(CH_2)_n-COCH_3$ (n represents the same as above), $-(CH_2)_n- CO_2R^{32}$ (n and $R^{32}$ represent the same as above),- $(CH_2)_n-CONR^{33}R^{34}$ (n, $R^{33}$, and $R^{34}$ represent the same as above), $-(CH_2)_m-OR^{35}$ (m and $R^{35}$ represent the same as above), $-(CH_2)_m-NR^{37}R^{38}$ (m, $R^{37}$, and $R^{38}$ represent the same as above), phenyl, pyridyl, pyrimidinyl, pyridazinyl, pyrazinyl, tetrazolyl, benzyl, pyridylmethyl, pyrimidinylmethyl, pyridazinylmethyl, pyrazinylmethyl, tetrazolylmethyl, a hydroxy group, an alkoxy group having 1 to 3 carbon atoms, or $-NR^{39}R^{40}$ ($R^{39}$ and $R^{40}$ represent the same as above); and
($b^2$) $R^{46}$ and $R^{47}$ together represent a 5- to 7-membered saturated nitrogen-containing heterocyclic group (wherein the 5- to 7-membered saturated nitrogen-containing heterocyclic group may be substituted by one or two substituents optionally selected from the group consisting of an alkyl

group, an amino group, a hydroxy group, an alkoxy group, and an oxo group) together with a nitrogen atom; and

($2^1$) $R^{45}$ and $R^{46}$ together represent a 5- to 7-membered saturated nitrogen-containing heterocyclic group (wherein the 5- to 7-membered saturated nitrogen-containing heterocyclic group may be substituted by one or two substituents optionally selected from the group consisting of an alkyl group, an amino group, a hydroxy group, an alkoxy group, and an oxo group) together with a nitrogen atom; $R^{47}$ and $R^{48}$ each independently represent a hydrogen atom, an alkyl group having 1 to 3 carbon atoms, acetyl, or $-(CH_2)_m-OR^{36}$ (m and $R^{36}$ represent the same as above)].

**14.** The prophylactic agent and/or therapeutic agent according to claim 6, wherein the CaMKII inhibitor is an isoxazole derivative represented by Formula A3 or a pharmaceutically acceptable salt thereof:
Formula A3:

[Formula 5]

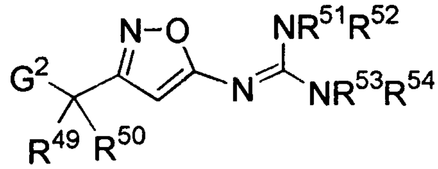

[wherein $G^2$ represents 2-fluoro-biphenyl-4-yl, 2'-fluoro-biphenyl-4-yl, or 3-benzoylphenyl; $R^{49}$ represents methyl; $R^{50}$ represents a hydrogen atom, methyl, methoxy, or ethoxy;
Formula: $=C(NR^{51}R^{52})NR^{53}R^{54}$ represents the following ($1^2$) ($2^2$), or ($3^2$) :

($1^2$) $R^{51}$ and $R^{52}$ represent the following ($a^3$), ($b^3$), or ($C^3$) and $R^{53}$ and $R^{54}$ represent the following ($d^3$), ($e^3$) or ($f^3$) :

($a^3$) $R^{51}$ and $R^{52}$ each independently represent a hydrogen atom or an alkyl group having 1 to 4 carbon atoms;
($b^3$) one of $R^{51}$ and $R^{52}$ represents a hydrogen atom, and the other represents $-(CH_2)_n-COCH_3$ (n represents the same as in claim 6), $-(CH_2)_n-CO_2R^{32}$ (n and $R^{32}$ represent the same as in claim 6), $-(CH_2)_n-CONR^{33}R^{34}$ (n, $R^{33}$, and $R^{34}$ represent the same as in claim 6), $-(CH_2)_m-OR^{35}$ (m and $R^{35}$ represent the same as in claim 6), or $-(CH_2)_m-NR^{37}R^{38}$ (m, $R^{37}$, and $R^{38}$ represent the same as in claim 6);
($c^3$) $R^{51}$ and $R^{52}$ together represent pyrrolidine, azepane, morpholine, thiazoline, piperidin-2-one, piperidin-4-one, thiamorpholine, piperadine which may be substituted at the 4th position by an alkyl group having 1 to 3 carbon atoms, piperidine which may be substituted at the 4th position by an alkoxy group having 1 to 3 carbon atoms, 4-hydroxypiperidine, or piperidine substituted at the 4th position by an amino group which may be substituted by one or two alkyl groups having 1 to 3 carbon atoms (wherein the pyrrolidine, azepane, morpholine, thiazoline, piperidin-2-one, piperidin-4-one, thiamorpholine, piperadine which may be substituted at the 4th position by an alkyl group having 1 to 3 carbon atoms, piperidine which may be substituted at the 4th position by an alkoxy group having 1 to 3 carbon atoms, 4-hydroxypiperidine, and piperidine substituted at the 4th position by an amino group which may be substituted by one or two alkyl groups having 1 to 3 carbon atoms may be substituted by one or two methyl groups) together with a nitrogen atom;
($d^3$) $R^{53}$ and $R^{54}$ each independently represent a hydrogen atom or an alkyl group having 1 to 4 carbon atoms;
($e^3$) one of $R^{53}$ and $R^{54}$ represents a hydrogen atom, and the other represents $-(CH_2)_n-COCH_3$ (n represents the same as above), $-(CH_2)_n-CO_2R^{32}$ (n and $R^{32}$ represent the same as above), $-(CH_2)_n-CONR^{33}R^{34}$ (n, $R^{33}$, and $R^{34}$ represent the same as above), $-(CH_2)_m-OR^{35}$ (m and $R^{35}$ represent the same as above), or $-(CH_2)_m-NR^{37}R^{38}$ (m, $R^{37}$, and $R^{38}$ represent the same as above); and
($f^3$) $R^{53}$ and $R^{54}$ together represent pyrrolidine, azepane, morpholine, thiazoline, piperidin-2-one, piperidin-4-one, thiamorpholine, piperadine which may be substituted at the 4th position by an alkyl group having 1 to 3 carbon atoms, piperidine which may be substituted at the 4th position by an alkoxy group having 1 to 3 carbon atoms, 4-hydroxypiperidine, or piperidine substituted at the 4th position by an amino group which may be substituted by one or two alkyl groups having 1 to 3 carbon atoms (wherein the pyrrolidine, azepane, morpholine, thiazoline, piperidin-2-one, piperidin-4-one, thiamorpholine, piperadine which may be substituted at the 4th position by an alkyl group having 1 to 3 carbon atoms, piperidine which may be substituted

at the 4th position by an alkoxy group having 1 to 3 carbon atoms, 4-hydroxypiperidine, and piperidine substituted at the 4th position by an amino group which may be substituted by one or two alkyl groups having 1 to 3 carbon atoms may be substituted by one or two methyl groups) together with a nitrogen atom;

($2^2$) Formula: = $C(NR^{51}R^{52})NR^{53}R^{54}$ represents a group represented by Formula:

[Formula 6]

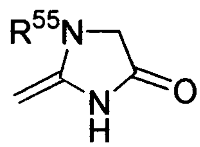

(wherein $R^{55}$ represents an alkyl group having 1 to 3 carbon atoms, acetyl, or - $(CH_2)_m$-$OR^{56}$ (m represents the same as above; $R^{56}$ represents a hydrogen atom or an alkyl group having 1 to 3 carbon atoms)); and ($3^2$) Formula: =$C(NR^{51}R^{52})NR^{53}R^{54}$ represents Formula: =C $(NR^{57}R^{58})$ N=C $(NH_2)$ $NR^{59}R^{60}$;
$R^{57}$ and $R^{58}$ represent the following ($a^4$), ($b^4$), or ($c^4$), and $R^{59}$ and $R^{60}$ represent the following ($d^4$), ($e^4$), or ($f^4$) :

($a^4$) $R^{57}$ and $R^{58}$ each independently represent a hydrogen atom or an alkyl group having 1 to 4 carbon atoms;
($b^4$) one of $R^{57}$ and $R^{58}$ represents a hydrogen atom, and the other represents -$(CH_2)$n-$COCH_3$ (n represents the same as above), - $(CH_2)_n$-$CO_2R^{32}$ (n and $R^{32}$ represent the same as above), $(CH_2)_n$-$CONR^{33}R^{34}$ (n, $R^{33}$, and $R^{34}$ represent the same as above), - $(CH_2)_m$-$OR^{35}$ (m and $R^{35}$ represent the same as above), or $(CH_2)_m$-$NR^{37}R^{38}$ (m, $R^{37}$, and $R^{38}$ represent the same as above);
($c^4$) $R^{57}$ and $R^{58}$ together represent pyrrolidine, azepane, morpholine, thiazoline, piperidin-2-one, piperidin-4-one, thiamorpholine, piperadine which may be substituted at the 4th position by an alkyl group having 1 to 3 carbon atoms, piperidine which may be substituted at the 4th position by an alkoxy group having 1 to 3 carbon atoms, 4-hydroxypiperidine, or piperidine substituted at the 4th position by an amino group which may be substituted by one or two alkyl groups having 1 to 3 carbon atoms (wherein the pyrrolidine, azepane, morpholine, thiazoline, piperidin-2-one, piperidin-4-one, thiamorpholine, piperadine which may be substituted at the 4th position by an alkyl group having 1 to 3 carbon atoms, piperidine which may be substituted at the 4th position by an alkoxy group having 1 to 3 carbon atoms, 4-hydroxypiperidine, and piperidine substituted at the 4th position by an amino group which may be substituted by one or two alkyl groups having 1 to 3 carbon atoms may be substituted by one or two methyl groups) together with a nitrogen atom;
($d^4$) $R^{59}$ and $R^{60}$ each independently represent a hydrogen atom or an alkyl group having 1 to 4 carbon atoms;
($e^4$) one of $R^{59}$ and $R^{60}$ represents a hydrogen atom, and the other represents -$(CH_2)_n$-$COCH_3$ (n represents the same as above), -$(CH_2)_n$-$CO_2R^{32}$ (n and $R^{32}$ represent the same as above), -$(CH_2)_n$-$CONR^{33}R^{34}$ (n, $R^{33}$, and $R^{34}$ represent the same as above), -$(CH_2)_m$-$OR^{35}$ (m and $R^{35}$ represent the same as above), or -$(CH_2)_m$-$NR^{37}R^{38}$ (m, $R^{37}$, and $R^{38}$ represent the same as above); and
($f^4$) $R^{59}$ and $R^{60}$ together represent pyrrolidine, azepane, morpholine, thiazoline, piperidin-2-one, piperidin-4-one, thiamorpholine, piperadine which may be substituted at the 4th position by an alkyl group having 1 to 3 carbon atoms, piperidine which may be substituted at the 4th position by an alkoxy group having 1 to 3 carbon atoms, 4-hydroxypiperidine, or piperidine substituted at the 4th position by an amino group which may be substituted by one or two alkyl groups having 1 to 3 carbon atoms (wherein the pyrrolidine, azepane, morpholine, thiazoline, piperidin-2-one, piperidin-4-one, thiamorpholine, piperadine which may be substituted at the 4th position by an alkyl group having 1 to 3 carbon atoms, piperidine which may be substituted at the 4th position by an alkoxy group having 1 to 3 carbon atoms, 4-hydroxypiperidine, and piperidine substituted at the 4th position by an amino group which may be substituted by one or two alkyl groups having 1 to 3 carbon atoms may be substituted by one or two methyl groups) together with a nitrogen atom].

**15.** The prophylactic agent and/or therapeutic agent according to claim 6, wherein the CaMKII inhibitor is an isoxazole derivative represented by Formula A4 or a pharmaceutically acceptable salt thereof:
Formula A4:

[Formula 7]

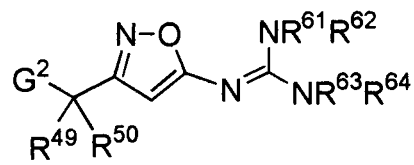

[wherein $G^2$, $R^{49}$, and $R^{50}$ represent the same as in claim 14;
Formula: =C ($NR^{61}R^{62}$) $NR^{63}R^{64}$ represents the following ($1^3$) ($2^3$), or ($3^3$) :

($1^3$) $R^{63}$ and $R^{64}$ both represent a hydrogen atom;
$R^{61}$ and $R^{62}$ represent the following ($a^5$), ($b^5$), or ($c^5$) :

($a^5$) $R^{61}$ and $R^{62}$ each independently represent a hydrogen atom or an alkyl group having 1 to 3 carbon atoms;
($b^5$) one of $R^{61}$ and $R^{62}$ represents a hydrogen atom, and the other represents $-(CH_2)_n-CO_2R^{32}$ (n and $R^{32}$ represent the same as in claim 6), $-(CH_2)_m-OR^{65}$ (m represents 2 or 3. $R^{65}$ represents a hydrogen atom, an alkyl group having 1 to 3 carbon atoms, 2-hydroxyethyl, or 3-hydroxypropyl), or $-(CH_2)_m-NR^{66}R^{67}$ (m represents the same as above; $R^{66}$ and $R^{67}$ each independently represent a hydrogen atom or an alkyl group having 1 to 3 carbon atoms, or together represent pyrrolidine, piperidine, morpholine, or N-methylpiperazine (wherein the pyrrolidine, piperidine, morpholine, and N-methylpiperazine may be substituted by one or two methyl groups) together with a nitrogen atom); and
($c^5$) $R^{61}$ and $R^{62}$ together represent pyrrolidine, piperidine, morpholine, or N-methylpiperazine (wherein the pyrrolidine, piperidine, morpholine, and N-methylpiperazine may be substituted by one or two methyl groups) together with a nitrogen atom;

($2^3$) Formula: =C ($NR^{61}R^{62}$) $NR^{63}R^{64}$ represents a group represented by Formula:

[Formula 8]

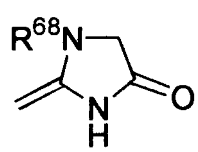

(wherein $R^{68}$ represents an alkyl group having 1 to 3 carbon atoms, 2-hydroxyethyl, or 3-hydroxypropyl); and
($3^3$) $R^{61}$ and $R^{62}$ together represent morpholine together with a nitrogen atom, and $R^{63}$ and $R^{64}$ together represent amino-morpholin-4-yl-methylene].

**16.** The prophylactic agent and/or therapeutic agent according to claim 15, wherein $G^2$ is 2-fluoro-biphenyl-4-yl or 2'-fluoro-biphenyl-4-yl, $R^{50}$ represents a hydrogen atom or methyl, $R^{63}$ and $R^{64}$ both are a hydrogen atom, and $R^{61}$ and $R^{62}$ represent the following ($a^6$) or ($b^6$) :

($a^6$) $R^{61}$ and $R^{62}$ each independently represent a hydrogen atom or an alkyl group having 1 to 3 carbon atoms; and
($b^6$) $R^{61}$ and $R^{62}$ together represent morpholine (the morpholine may be substituted by one or two methyl groups) together with a nitrogen atom.

**17.** The prophylactic agent and/or therapeutic agent according to any of claims 1 to 5, wherein the CaMKII inhibitor is 3-[(1S)-1-(2-fluorobiphenyl-4-yl)ethyl]-5-{[amino(morpholin-4-yl)methylene]amino}isoxazole or a pharmaceutically acceptable salt thereof.

**18.** The prophylactic agent and/or therapeutic agent according to any of claims 1 to 17, wherein the disease modifying antirheumatic drug is one or several agents selected from the followings:

leflunomide, methotrexate, D-penicillamine, bucillamine, salazosulfapyridine, actarit, lobenzarit, cyclophosphamide, chlorambucil, mizoribine, azathioprine, auranofin, gold sodium thiomaleate, aurothiosulfate, aurothioglucose, cyclosporine, hydroxychloroquine, chlorouquine, tacrolimus, etanercept, infliximab, anakinra, keliximab, adalimumab, rituximab, and a humanized anti-IL-6R antibody.

**19.** The prophylactic agent and/or therapeutic agent according to claim 18, wherein the disease modifying antirheumatic drug is methotrexate, salazosulfapyridine, or leflunomide.

**20.** The prophylactic agent and/or therapeutic agent according to any of claims 1 to 19, wherein the autoimmune disease is any disease selected from the followings:

rheumatoid arthritis, systemic lupus erythematosus, discoid lupus erythematosus, polymyositis, scleroderma, mixed connective tissue disease, Hashimoto's disease, primary myxedema, thyrotoxicosis, pernicious anemia, Good-pasture's syndrome, rapidly progressive glomerulonephritis, myasthenia gravis, pemphigus vulgaris, bullous pemphigoid, insulin resistant diabetes, juvenile diabetes, type I diabetes mellitus, Addison's disease, atrophic gastritis, male sterility, climacterium precox, lens-induced uveitis, multiple sclerosis, ulcerative colitis, primary biliary cirrhosis, chronic active hepatitis, autoimmune blood disease, autoimmune hemolytic anemia, idiopathic thrombocytopenia, paroxysmal hemoglobinuria, idiopathic thrombocytopenic purpura, interstitial pulmonary fibrosis, and Sjogren's syndrome.

**21.** The prophylactic agent and/or therapeutic agent according to claim 20, wherein the autoimmune disease is rheumatoid arthritis.

**22.** An enhancer for a prophylactic and/or therapeutic effect of a disease modifying antirheumatic drug on an autoimmune disease, comprising a CaMKII inhibitor as an active ingredient.

**23.** The enhancer according to claim 22, wherein the CaMKII inhibitor is a substance selected by a screening method comprising the following steps (a), (b), and (c):

(a) bringing a test substance into contact with a CaMKII enzyme and a substrate;
(b) measuring a phosphorylation level of the substrate by the CaMKII enzyme brought into contact with the test substance, and comparing the phosphorylation level with a phosphorylation level of the substrate by a control enzyme kept from contact with the test substance; and
(c) selecting, on the basis of the comparison result of the step (b), a test substance that inhibits CaMKII activity.

**24.** The enhancer according to claim 23, wherein the CaMKII inhibitor is a substance having a CaMKII inhibition rate of 30% or higher at a concentration of 10 $\mu$M.

**25.** The enhancer according to claim 22, wherein the CaMKII inhibitor is 3-[(1S)-1-(2-fluorobiphenyl-4-yl)ethyl]-5-{[amino (morpholin-4-yl)methylene]amino}isoxazole or a pharmaceutically acceptable salt thereof.

**26.** The enhancer according to any of claims 22 to 25, wherein the disease modifying antirheumatic drug is methotrexate, salazosulfapyridine, or leflunomide.

**27.** The enhancer according to any of claims 22 to 26, wherein the autoimmune disease is rheumatoid arthritis.

**28.** A first pharmaceutical composition for use in combination with a second pharmaceutical composition to achieve a prophylactic and/or therapeutic effect on an autoimmune disease for a mammal with the autoimmune disease, wherein the effect exceeds total preventive and/or therapeutic effects on the autoimmune disease achieved by separately administering the first and second pharmaceutical compositions, and the second pharmaceutical composition and the first pharmaceutical composition comprise a disease modifying antirheumatic drug and a CaMKII inhibitor, respectively.

**29.** A first pharmaceutical composition for use in combination with a second pharmaceutical composition to achieve a prophylactic and/or therapeutic effect on an autoimmune disease for a mammal with the autoimmune disease,

wherein the effect exceeds each of a prophylactic and/or therapeutic effect on the autoimmune disease achieved by separately administering the first and second pharmaceutical compositions, and the second pharmaceutical composition and the first pharmaceutical composition comprise a disease modifying antirheumatic drug and a CaMKII inhibitor, respectively.

**30.** The composition according to claim 28 or 29, wherein the CaMKII inhibitor is 3-[(1S)-1-(2-fluorobiphenyl-4-yl)ethyl]-5-{[amino(morpholin-4-yl)methylene]amino}isoxazole or a pharmaceutically acceptable salt thereof.

**31.** The composition according to any of claims 28 to 30, wherein the disease modifying antirheumatic drug is methotrexate, salazosulfapyridine, or leflunomide.

**32.** The composition according to any of claims 28 to 31, wherein the autoimmune disease is rheumatoid arthritis.

**33.** A commercial package comprising the following

(1) and (2):
(1) a pharmaceutical composition comprising a CaMKII inhibitor; and
(2) a document regarding the pharmaceutical composition indicating that the pharmaceutical composition can be used or should be used in a prophylactic and/or therapeutic application for an autoimmune disease in combination with a disease modifying antirheumatic drug.

**34.** A commercial package comprising the following

(1) and (2) :
(1) a pharmaceutical composition comprising a CaMKII inhibitor; and
(2) a document regarding the pharmaceutical composition indicating that the pharmaceutical composition can be used or should be used in an application for enhancing a prophylactic and/or therapeutic effect of a disease modifying antirheumatic drug on an autoimmune disease.

**35.** The commercial package according to claim 33 or 34, wherein the CaMKII inhibitor is 3-[(1S)-1-(2-fluorobiphenyl-4-yl)ethyl]-5-{[amino(morpholin-4-yl)methylene]amino}isoxazole or a pharmaceutically acceptable salt thereof.

**36.** The commercial package according to any of claims 33 to 35, wherein the disease modifying antirheumatic drug is methotrexate, salazosulfapyridine, or leflunomide.

**37.** The commercial package according to any of claims 33 to 36, wherein the autoimmune disease is rheumatoid arthritis.

**38.** A kit for prophylaxis and/or treatment of an autoimmune disease comprising a first composition comprising a CaMKII inhibitor and a second composition comprising a disease modifying antirheumatic drug in a package.

**39.** Use of a CaMKII inhibitor and a disease modifying antirheumatic drug for manufacturing a pharmaceutical composition for prophylaxis and/or treatment of an autoimmune disease.

**40.** Use of a CaMKII inhibitor for manufacturing a prophylactic agent and/or therapeutic agent for an autoimmune disease **characterized by** being used in combination with a disease modifying antirheumatic drug.

**41.** Use of a CaMKII inhibitor for manufacturing a pharmaceutical composition for enhancing a prophylactic and/or therapeutic effect of a disease modifying antirheumatic drug on an autoimmune disease.

**42.** A method for preventing and/or treating an autoimmune disease, **characterized by** comprising administering a CaMKII inhibitor in combination with a disease modifying antirheumatic drug.

**43.** A method for enhancing a prophylactic and/or therapeutic effect of a disease modifying antirheumatic drug on an autoimmune disease, comprising administering a CaMKII inhibitor.

**44.** A method for preventing and/or treating an autoimmune disease, comprising administering a CaMKII inhibitor in an amount effective for enhancing a prophylactic and/or therapeutic effect of a disease modifying antirheumatic drug on the autoimmune disease to a mammal with the autoimmune disease on which the disease modifying antirheumatic

drug cannot exert a sufficient prophylactic and/or therapeutic effect.

**45.** A prophylactic agent and/or therapeutic agent for an autoimmune disease comprising a CaMKII inhibitor in combination with a disease modifying antirheumatic drug.

**46.** A drug comprising a CaMKII inhibitor in combination with a disease modifying antirheumatic drug.

**47.** An agent for inhibiting joint destruction comprising a CaMKII inhibitor and a disease modifying antirheumatic drug.

**48.** An agent for inhibiting joint destruction comprising a CaMKII inhibitor, which is intended to be used in combination with a disease modifying antirheumatic drug.

**49.** An enhancer for a joint destruction effect of a disease modifying antirheumatic drug, comprising a CaMKII inhibitor as an active ingredient.

# FIG.1

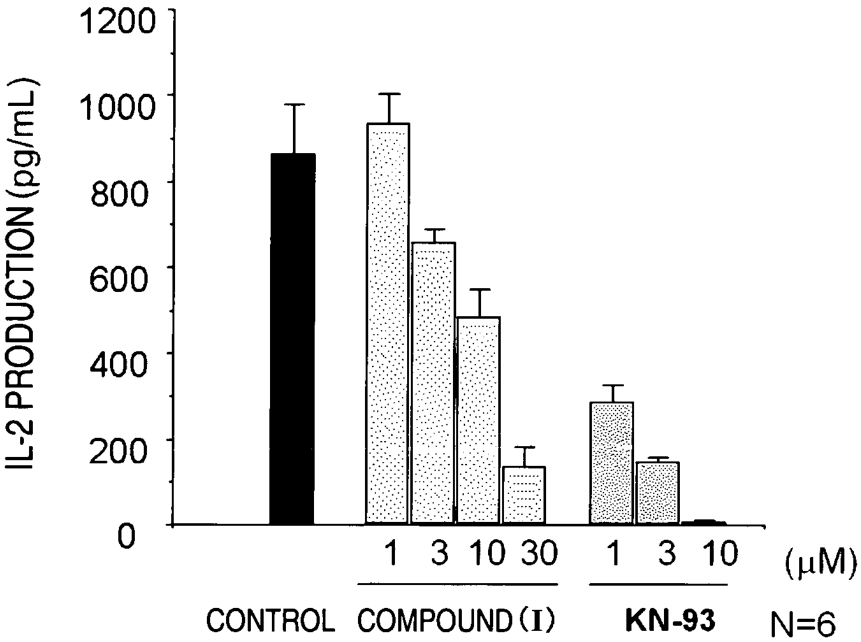
IL-2 PRODUCTION (pg/mL)
1200
1000
800
600
400
200
0
1 3 10 30 1 3 10 (μM)
CONTROL COMPOUND (I) KN-93 N=6

# FIG.2

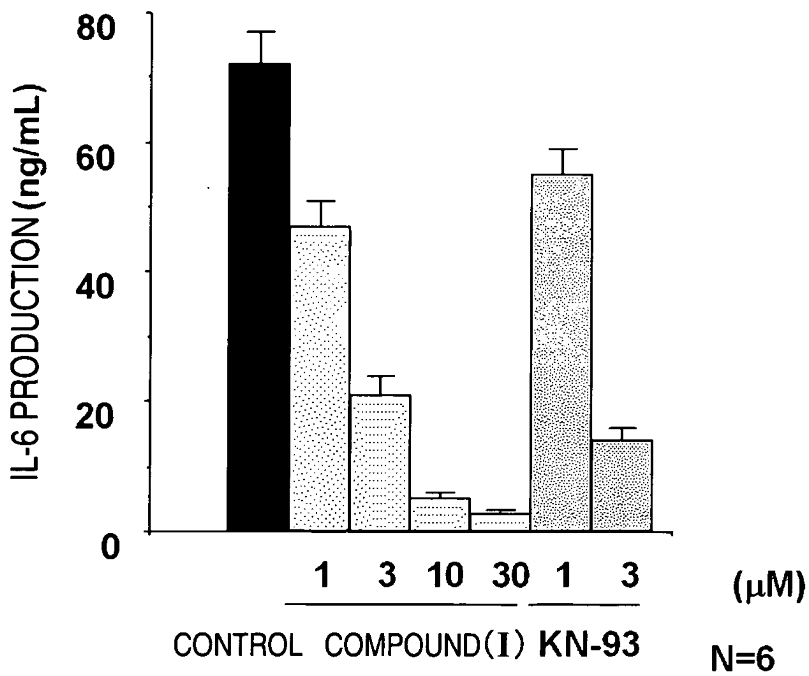
IL-6 PRODUCTION (ng/mL)
80
60
40
20
0
1 3 10 30 1 3 (μM)
CONTROL COMPOUND (I) KN-93 N=6

FIG.3

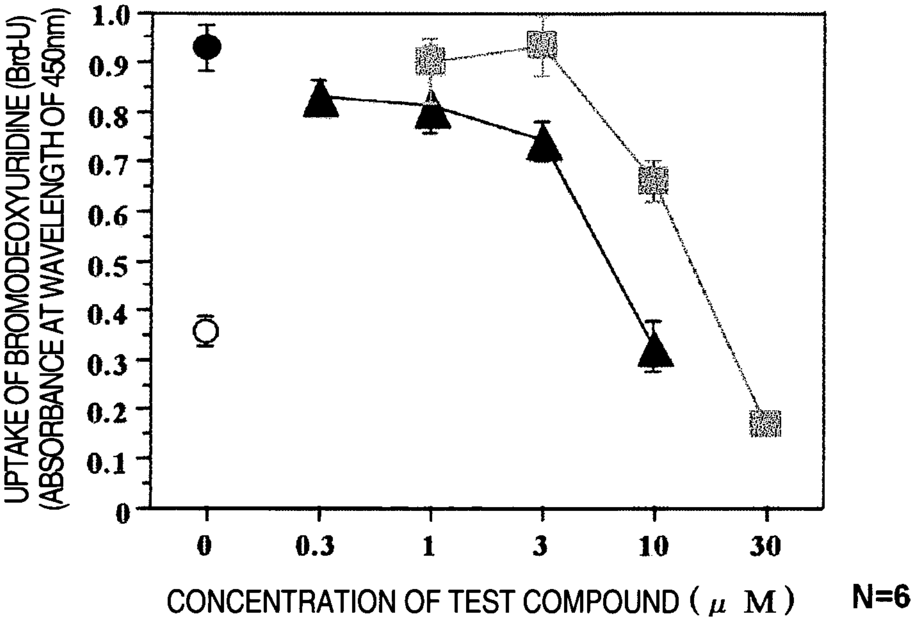
UPTAKE OF BROMODEOXYURIDINE (BrdU)
(ABSORBANCE AT WAVELENGTH OF 450nm)
1.0
0.9
0.8
0.7
0.6
0.5
0.4
0.3
0.2
0.1
0
0
0.3
1
3
10
30
CONCENTRATION OF TEST COMPOUND (μ M)
N=6

FIG.4

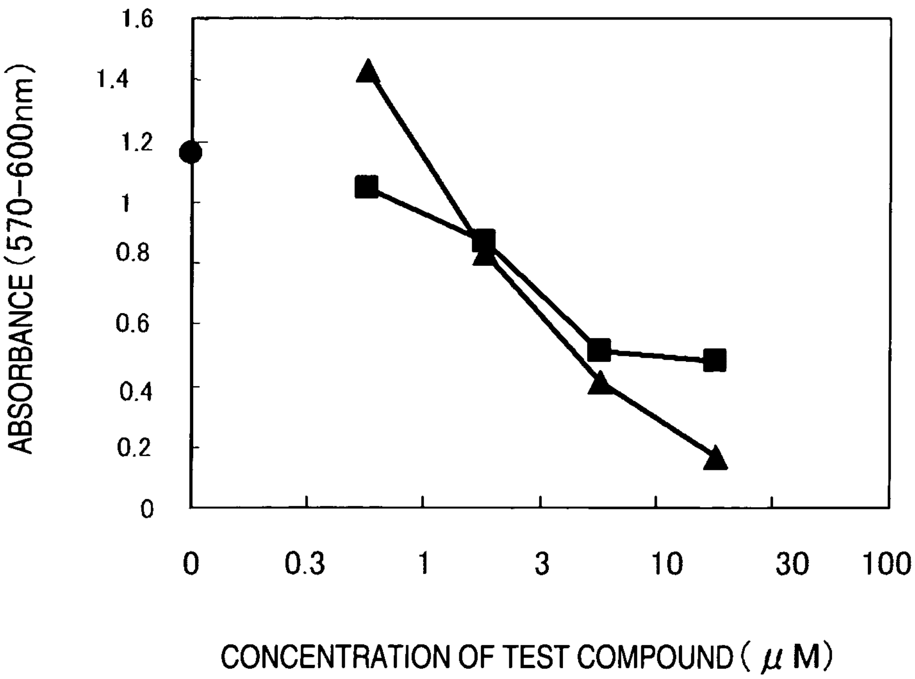
ABSORBANCE (570-600nm)
1.6
1.4
1.2
1
0.8
0.6
0.4
0.2
0
0
0.3
1
3
10
30
100
CONCENTRATION OF TEST COMPOUND (μ M)

# FIG.5

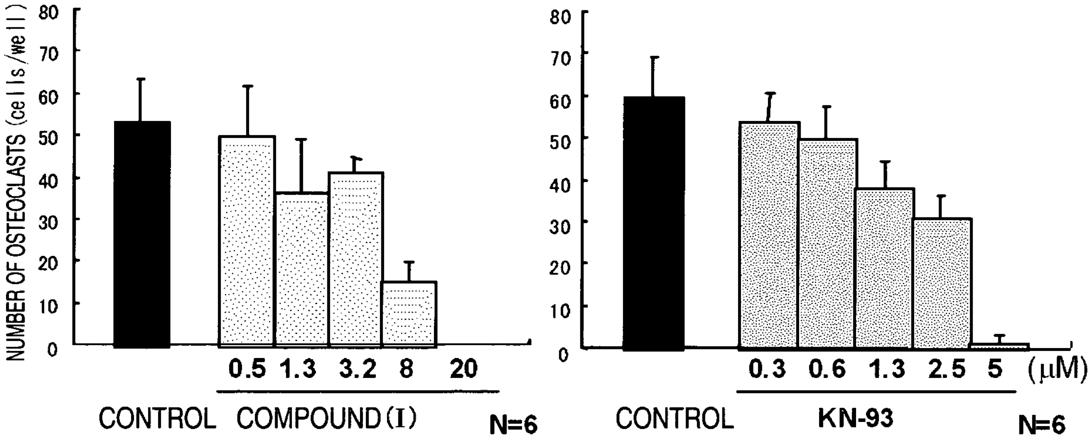


# FIG.6

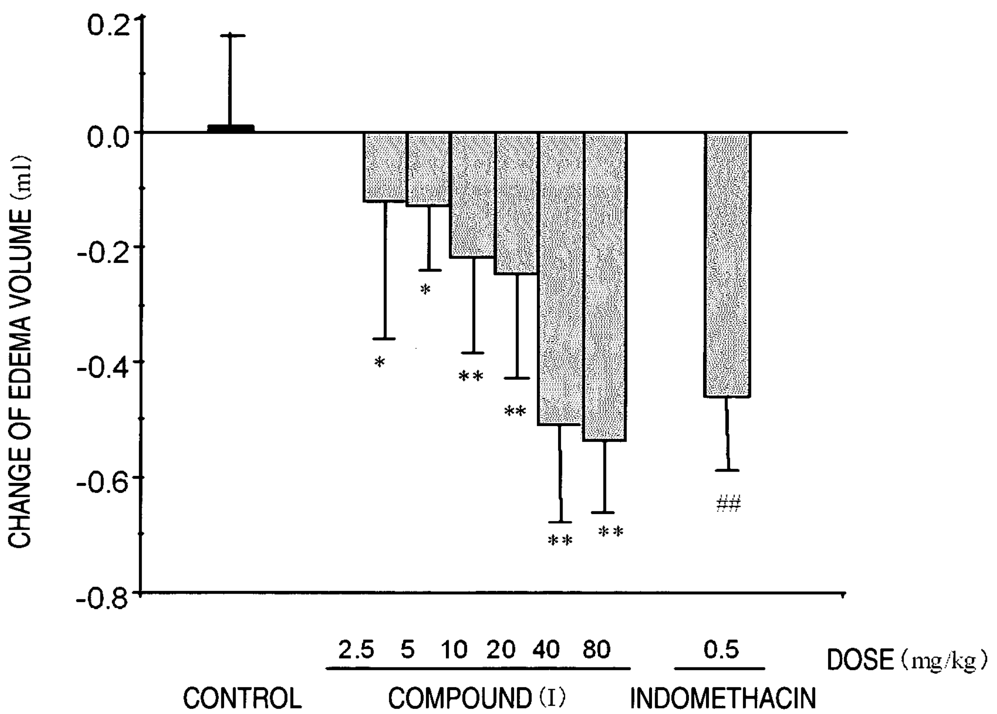


N=10.
*p<0.05, **p<0.01 (WILLIAM'S MULTIPLE COMPARISON, ONE-SIDED TEST)
##p<0.01 (STUDENT'S T TEST, TWO-SIDED TEST)

# FIG.7

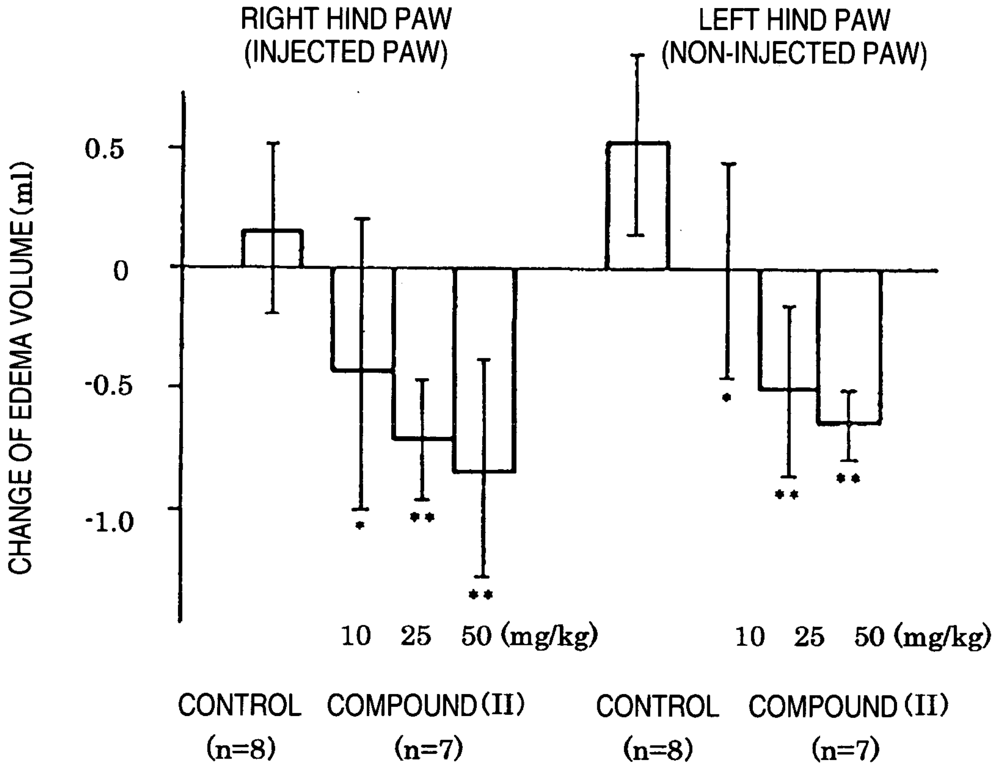


*<0.05,**<0.01 (DUNNETT'S MULTIPLE COMPARISON, TWO-SIDED TEST)

# FIG.8

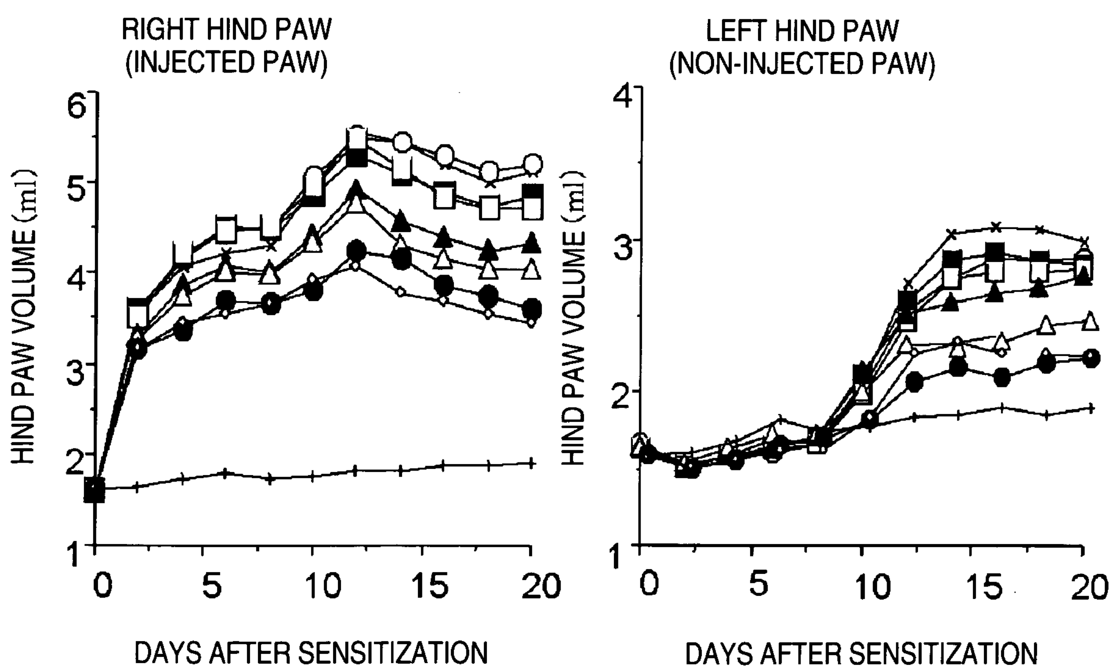

FIG.9

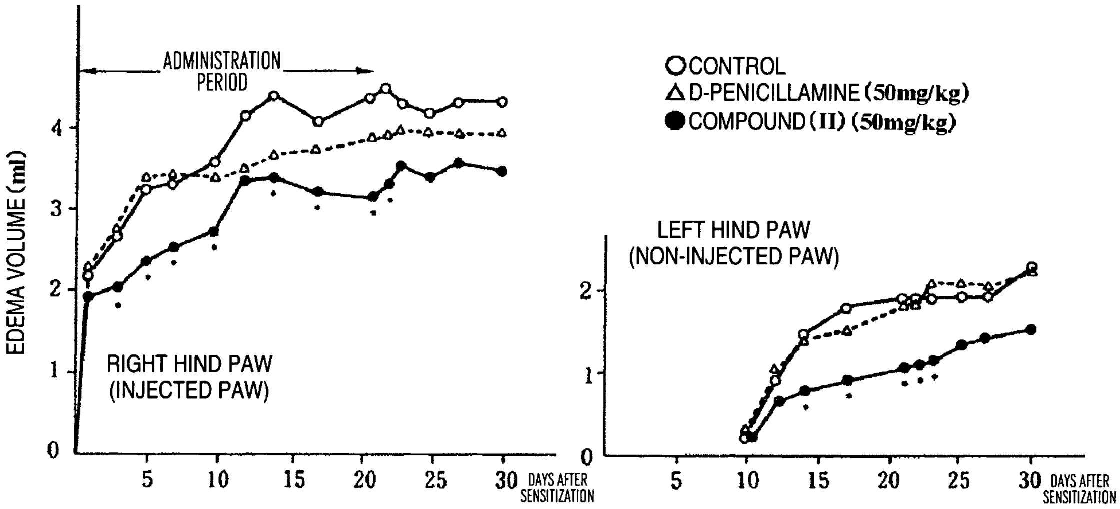


N=10-12
*$p<0.05$ (DUNNETT'S MULTIPLE COMPARISON, TWO-SIDED TEST)

FIG.10

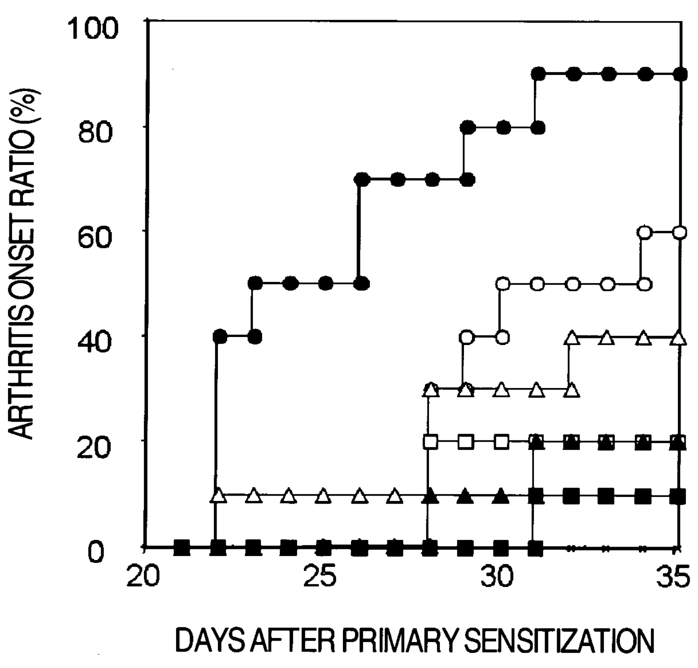

# FIG.11

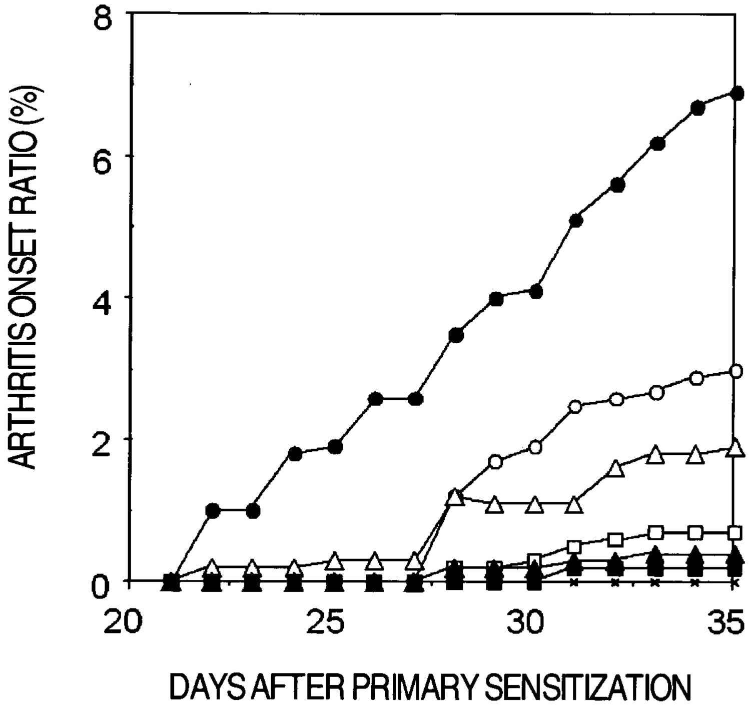
8
6
4
2
0
ARTHRITIS ONSET RATIO (%)
20
25
30
35
DAYS AFTER PRIMARY SENSITIZATION

# FIG.12

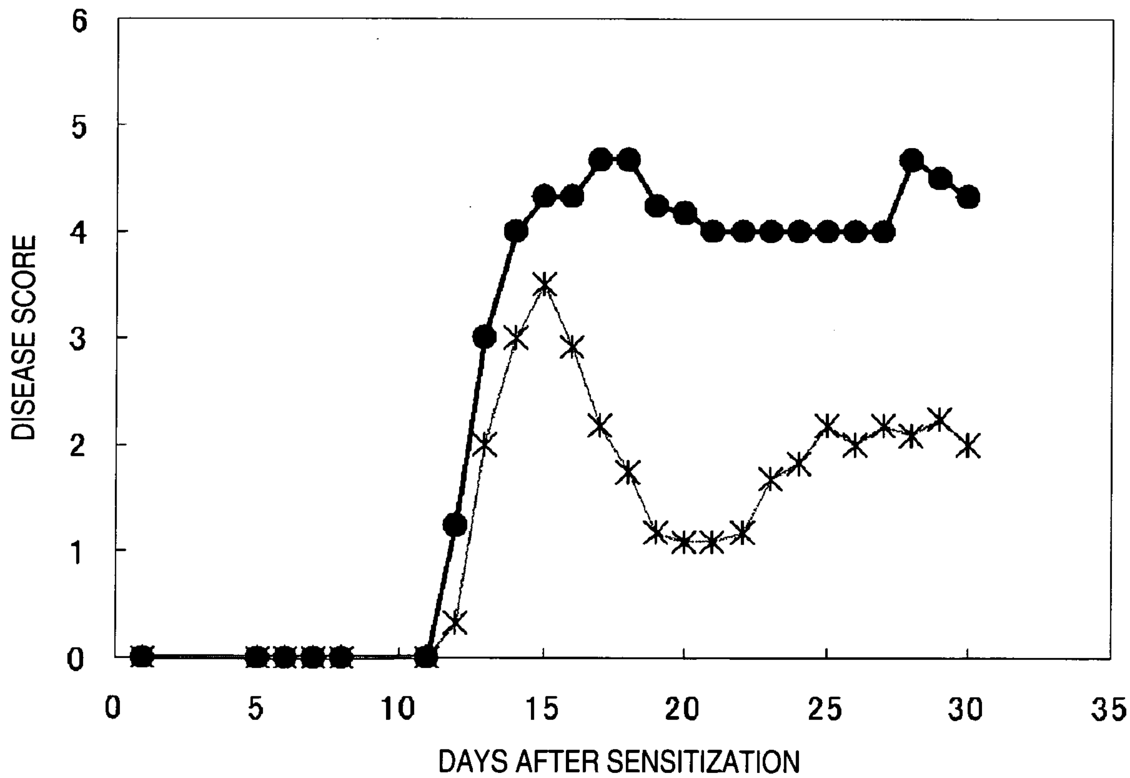
6
5
4
3
2
1
0
DISEASE SCORE
0
5
10
15
20
25
30
35
DAYS AFTER SENSITIZATION

FIG.13

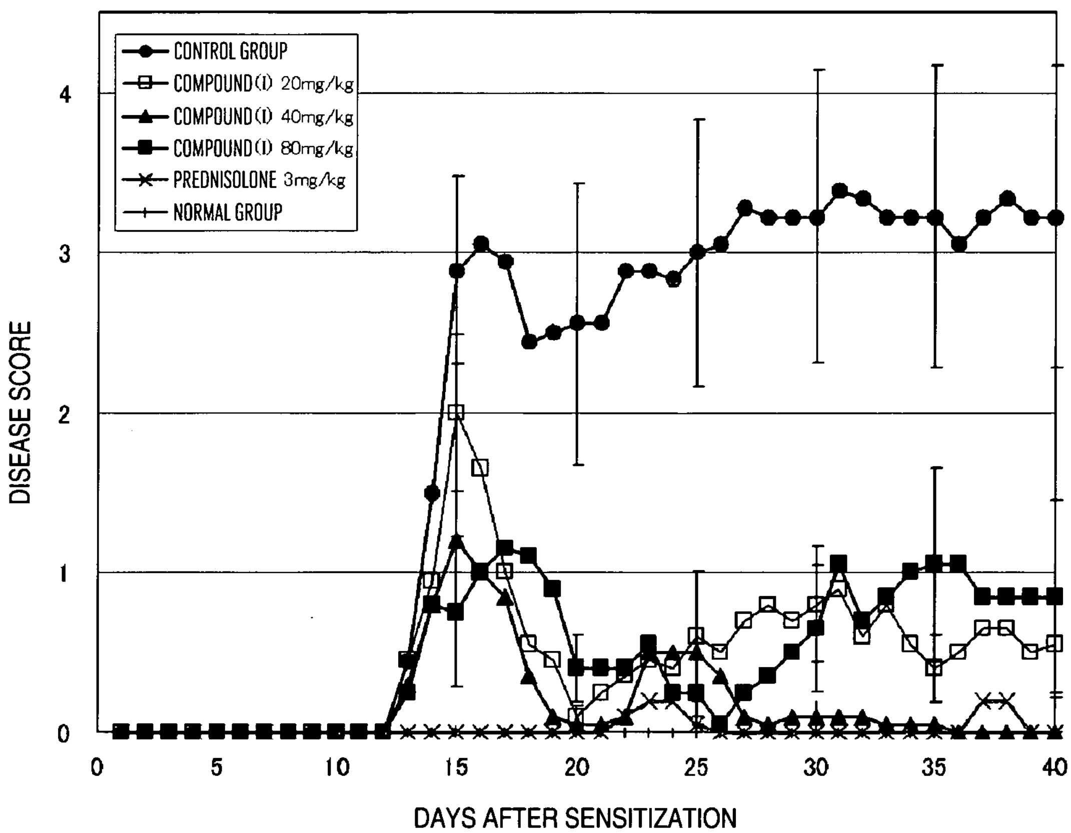
CONTROL GROUP
COMPOUND(I) 20mg/kg
COMPOUND(I) 40mg/kg
COMPOUND(I) 80mg/kg
PREDNISOLONE 3mg/kg
NORMAL GROUP
DISEASE SCORE
0
1
2
3
4
0
5
10
15
20
25
30
35
40
DAYS AFTER SENSITIZATION

FIG.14

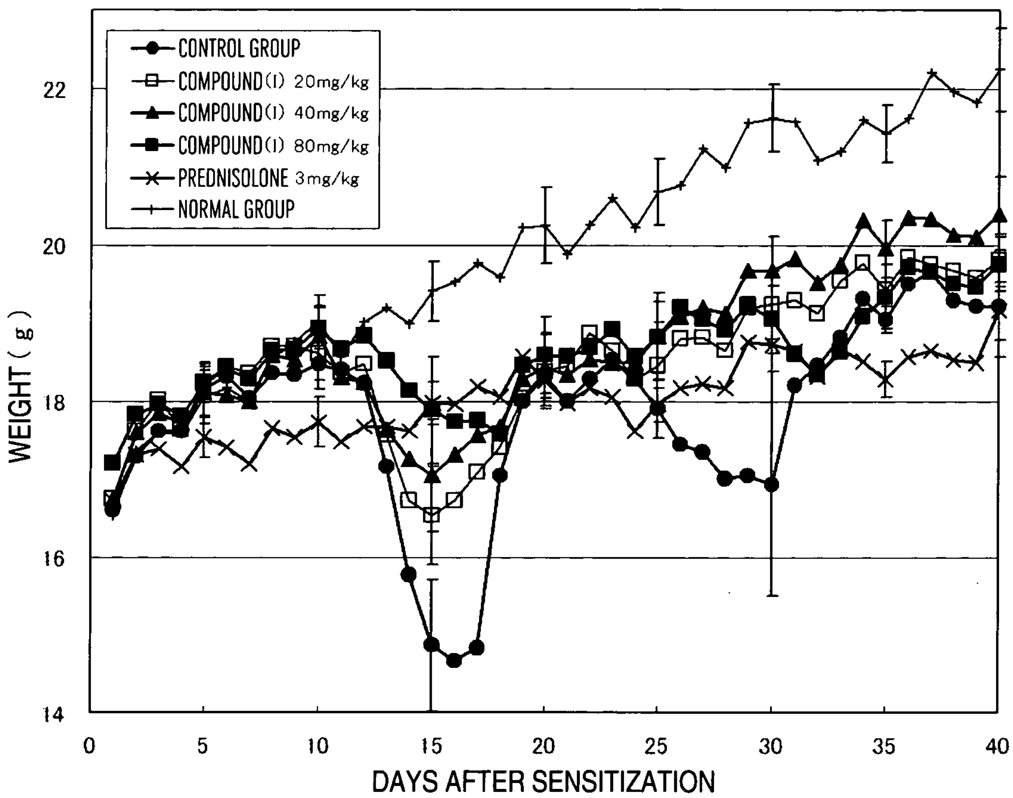
CONTROL GROUP
COMPOUND(I) 20mg/kg
COMPOUND(I) 40mg/kg
COMPOUND(I) 80mg/kg
PREDNISOLONE 3mg/kg
NORMAL GROUP
WEIGHT ( g )
22
20
18
16
14
0
5
10
15
20
25
30
35
40
DAYS AFTER SENSITIZATION

FIG.15

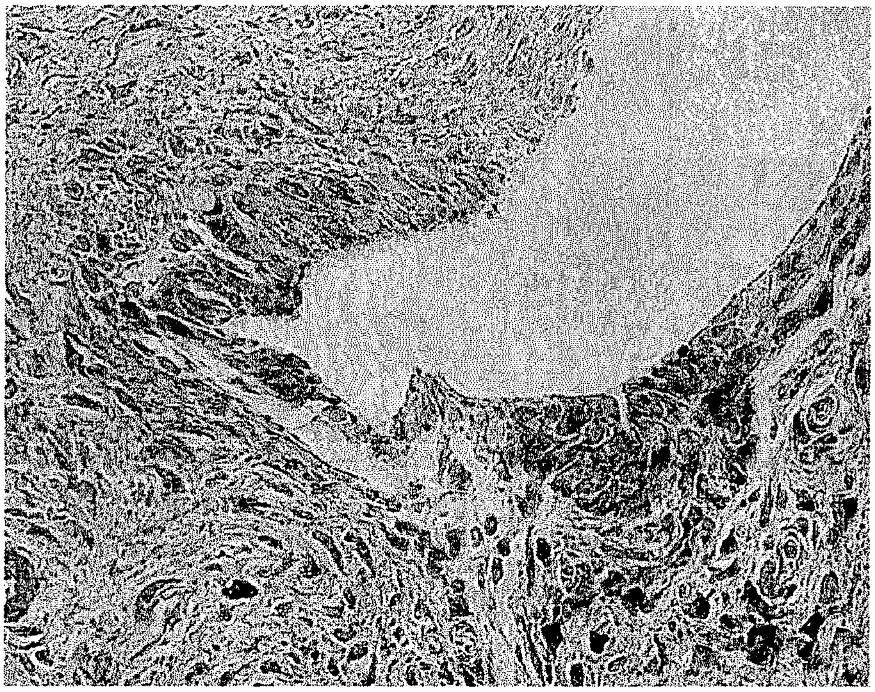

FIG.16

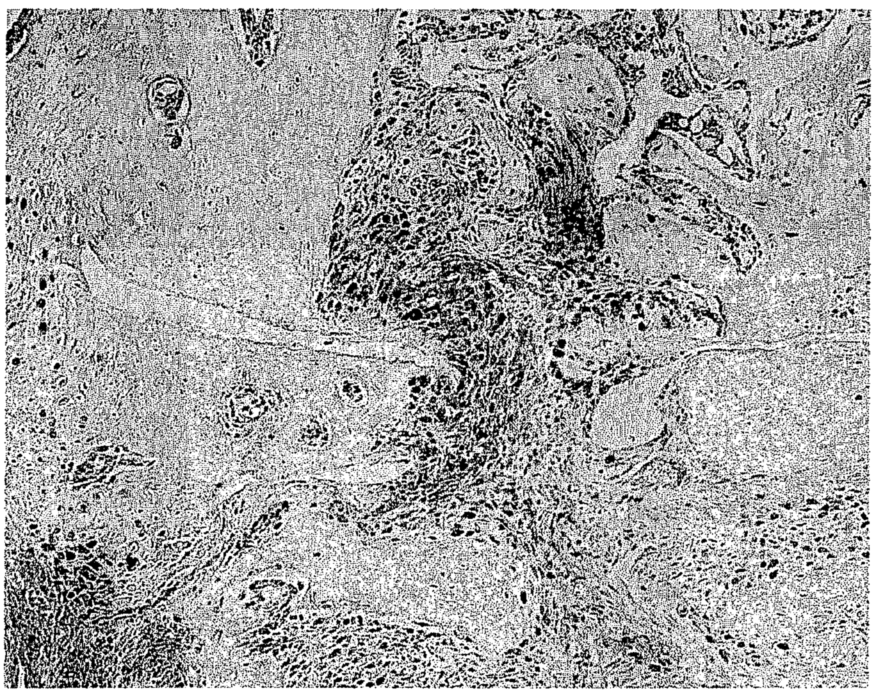

## FIG.17

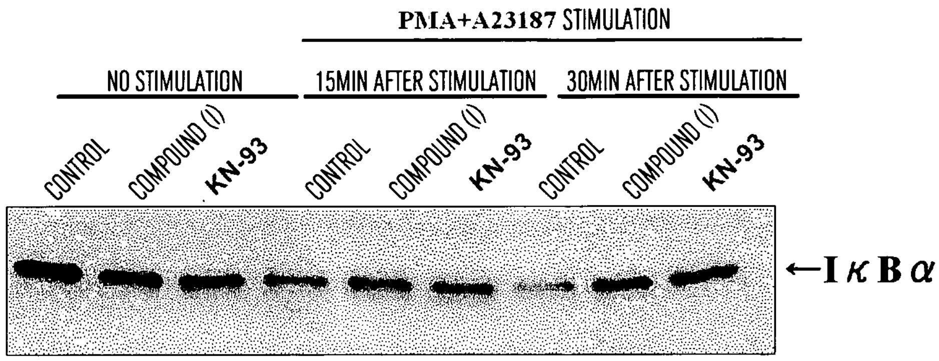


## FIG.18

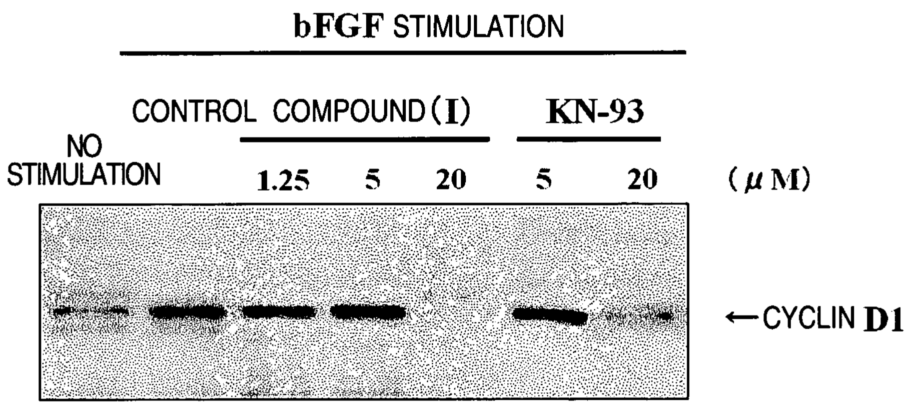


## FIG.19

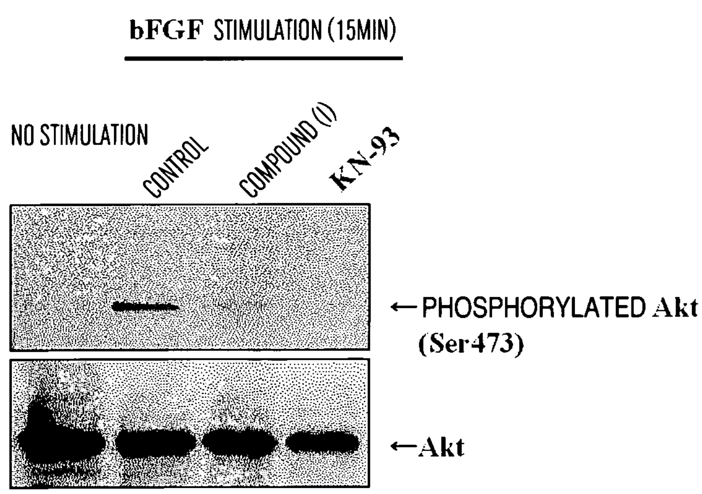

FIG.20

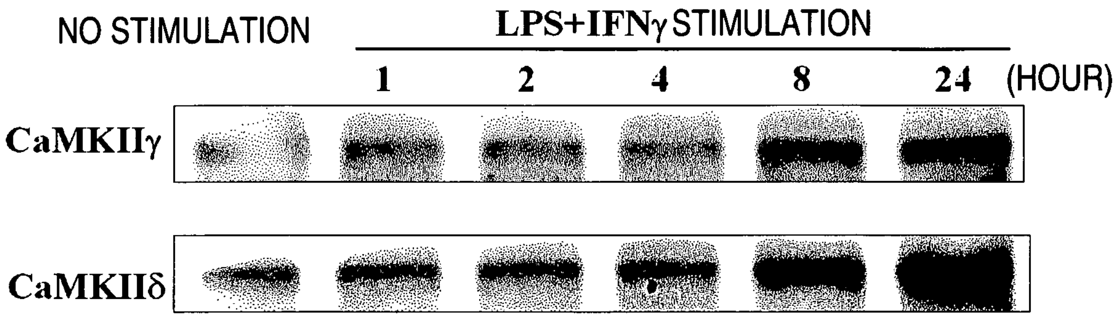
NO STIMULATION
LPS+IFNγ STIMULATION
1
2
4
8
24
(HOUR)
CaMKIIγ
CaMKIIδ

FIG.21

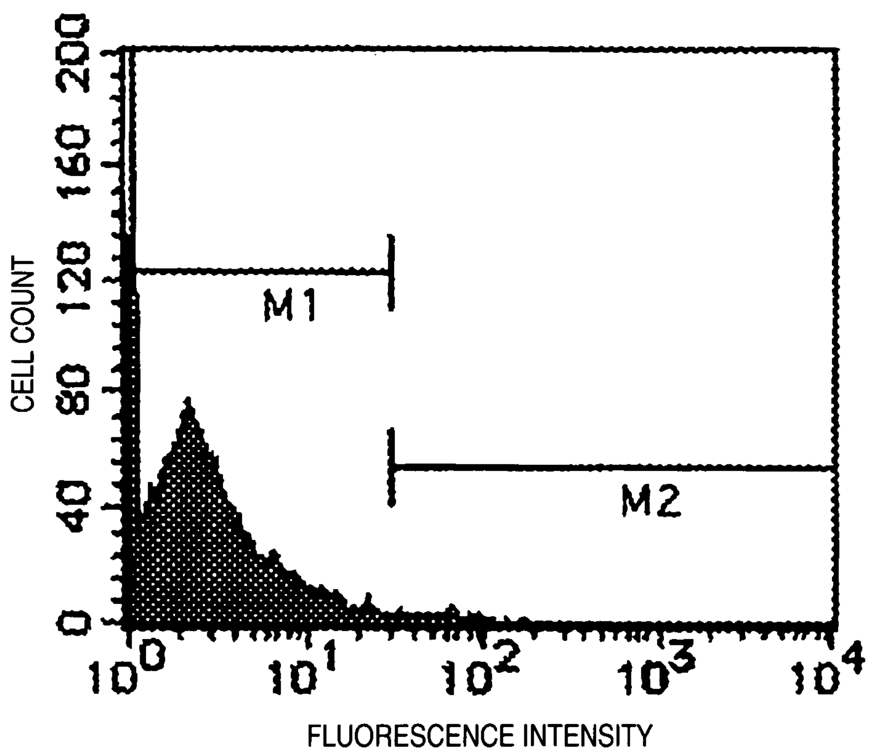
CELL COUNT
0
40
80
120
160
200
M1
M2
10^0
10^1
10^2
10^3
10^4
FLUORESCENCE INTENSITY

FIG.22

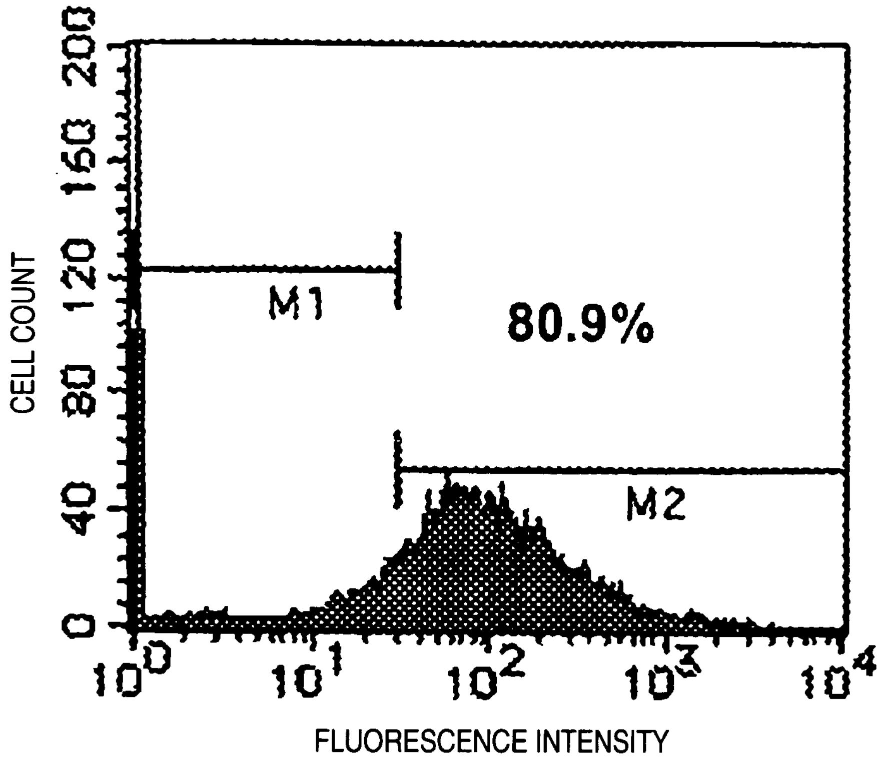
CELL COUNT
0
40
80
120
160
200
M1
80.9%
M2
10^0
10^1
10^2
10^3
10^4
FLUORESCENCE INTENSITY

FIG.23

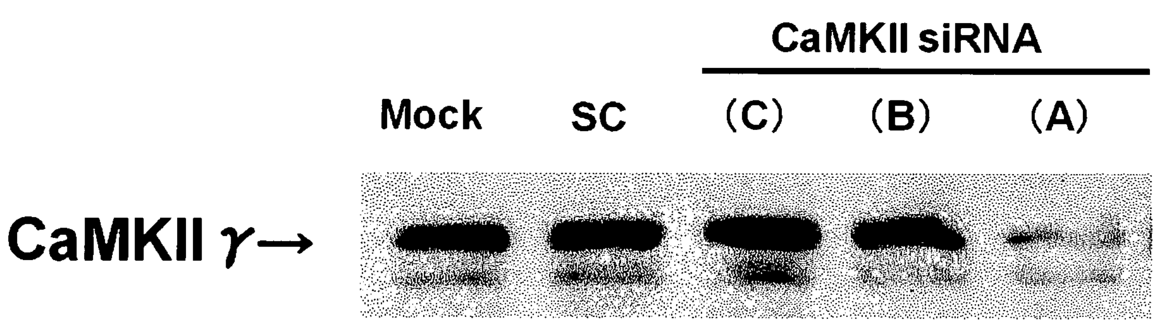
CaMKII siRNA
Mock
SC
(C)
(B)
(A)
CaMKII γ→

FIG.24

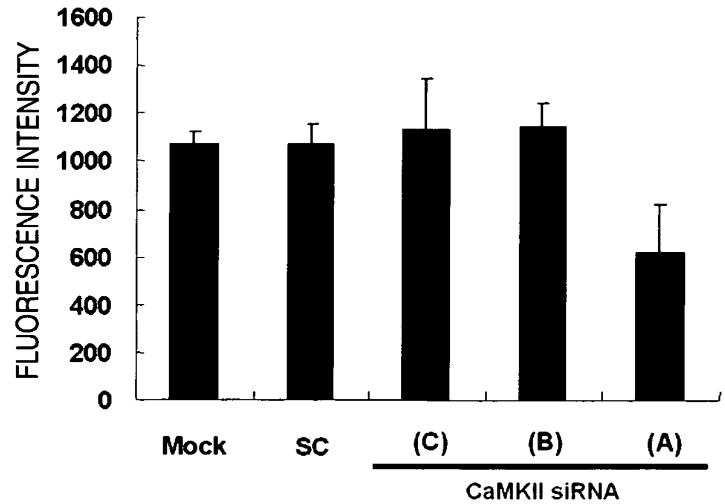


FIG.25

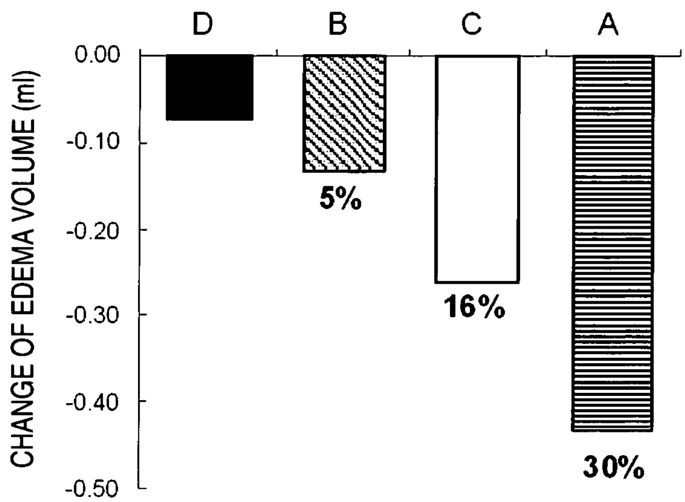


FIG.26

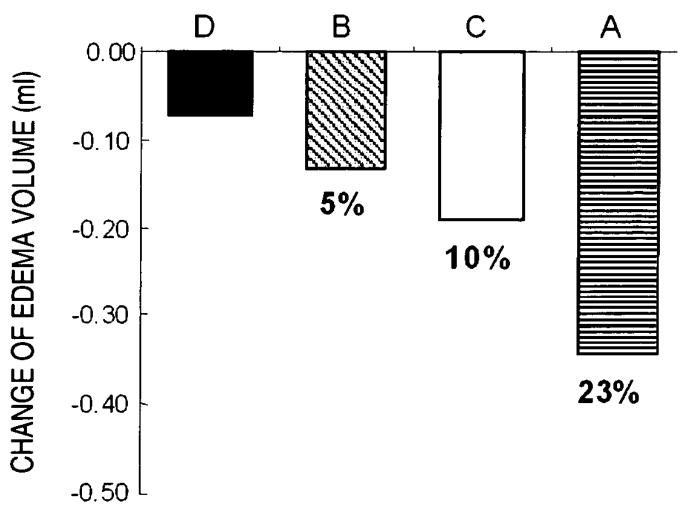

# FIG.27

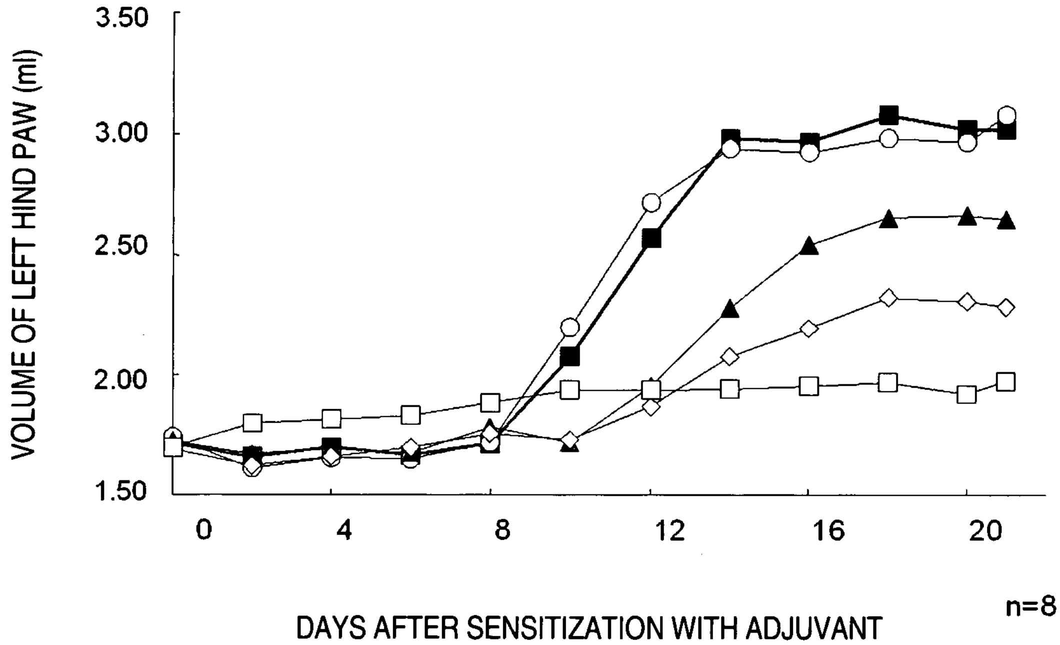
3.50
3.00
2.50
2.00
1.50
VOLUME OF LEFT HIND PAW (ml)
0
4
8
12
16
20
n=8
DAYS AFTER SENSITIZATION WITH ADJUVANT

# FIG.28

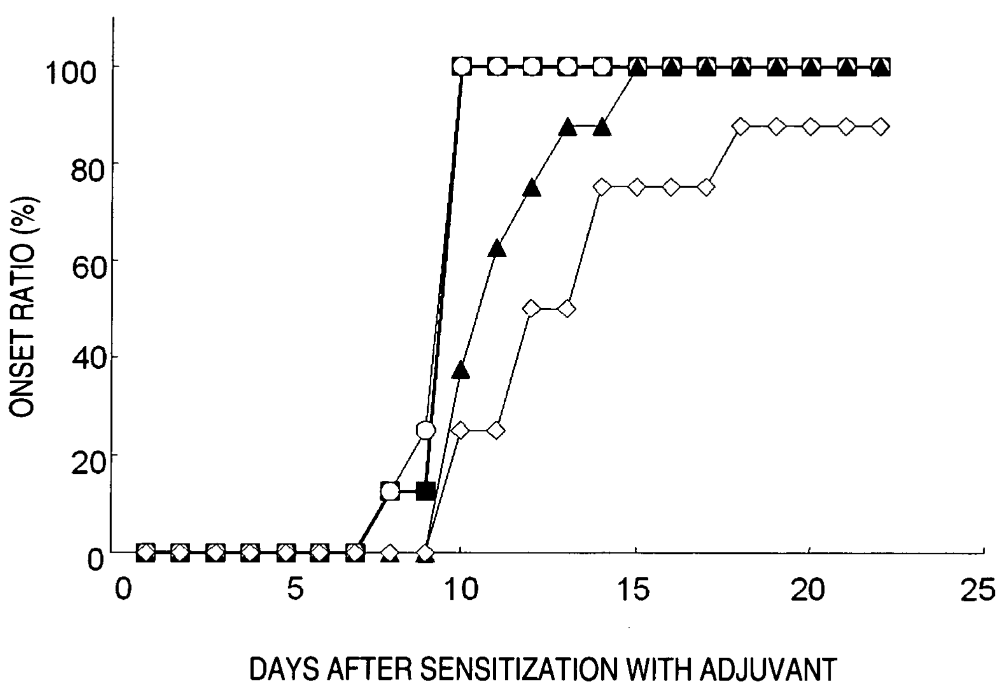
100
80
60
40
20
0
ONSET RATIO (%)
0
5
10
15
20
25
DAYS AFTER SENSITIZATION WITH ADJUVANT

FIG.29

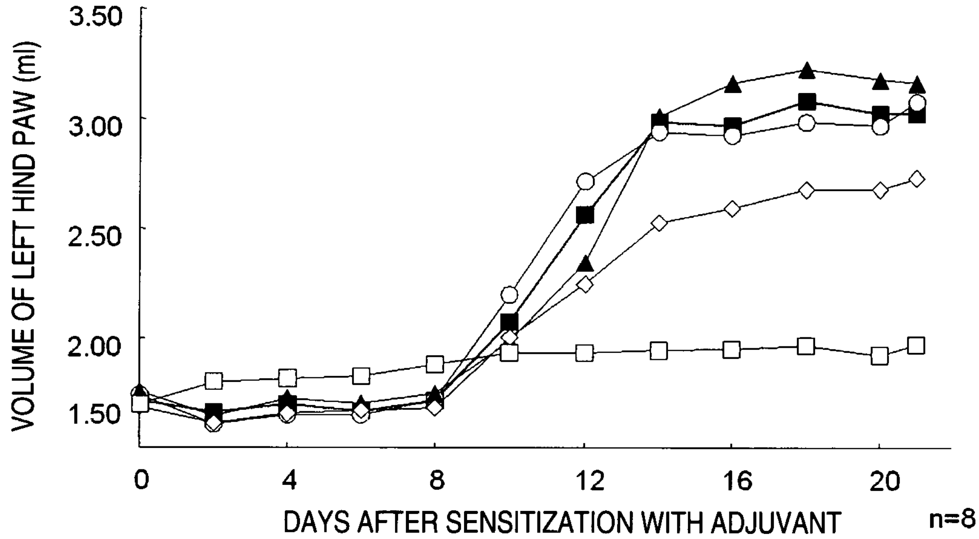
3.50
3.00
2.50
2.00
1.50
VOLUME OF LEFT HIND PAW (ml)
0
4
8
12
16
20
DAYS AFTER SENSITIZATION WITH ADJUVANT
n=8

FIG.30

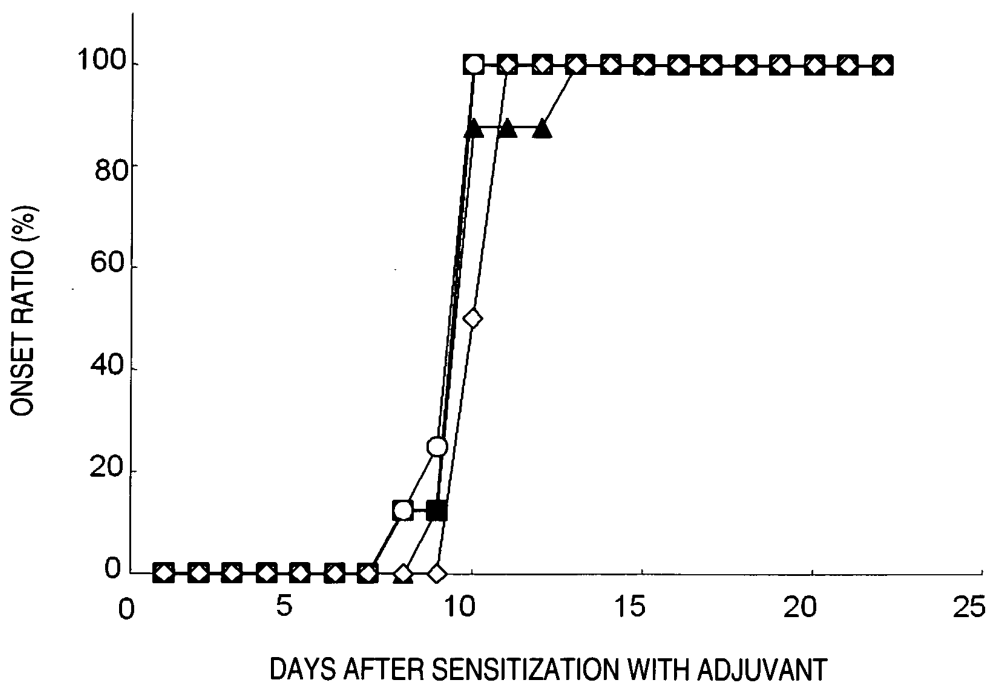
100
80
60
40
20
0
ONSET RATIO (%)
0
5
10
15
20
25
DAYS AFTER SENSITIZATION WITH ADJUVANT

# FIG.31

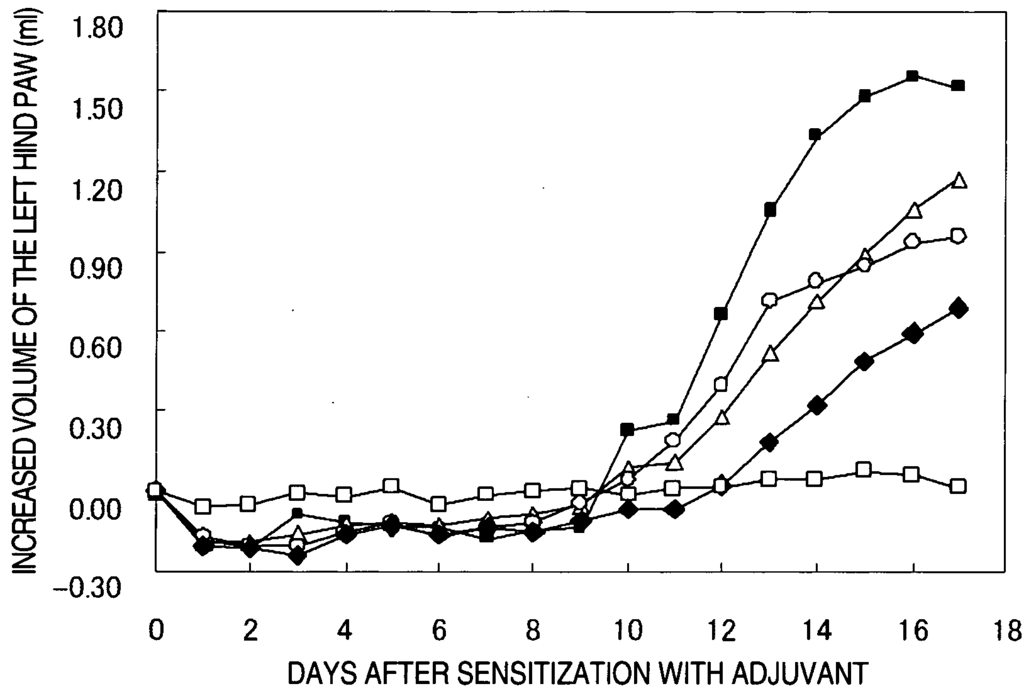
INCREASED VOLUME OF THE LEFT HIND PAW (ml)
1.80
1.50
1.20
0.90
0.60
0.30
0.00
−0.30
0
2
4
6
8
10
12
14
16
18
DAYS AFTER SENSITIZATION WITH ADJUVANT

# FIG.32

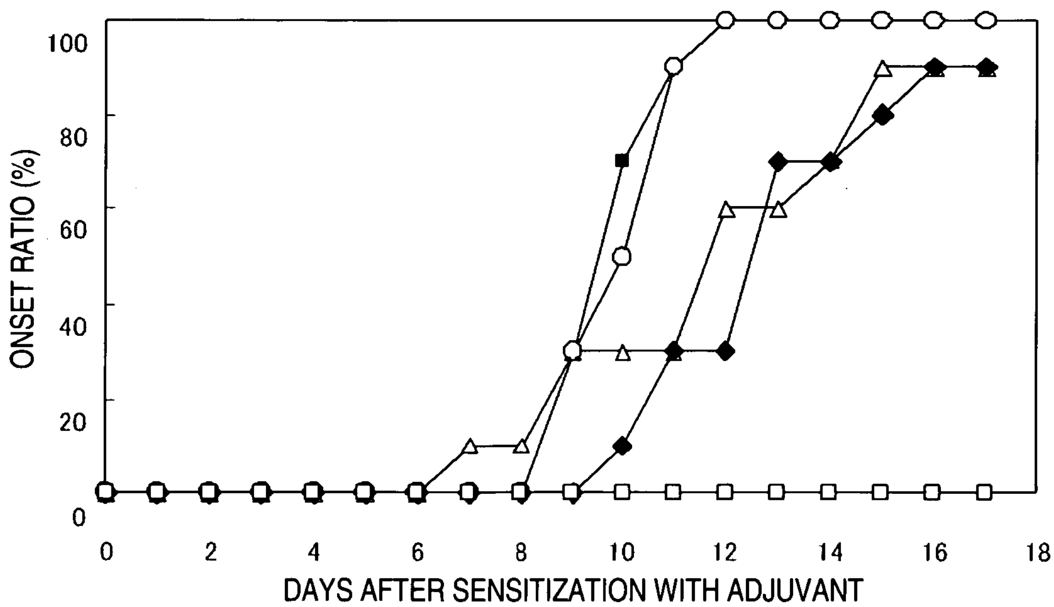
ONSET RATIO (%)
100
80
60
40
20
0
0
2
4
6
8
10
12
14
16
18
DAYS AFTER SENSITIZATION WITH ADJUVANT

FIG.33

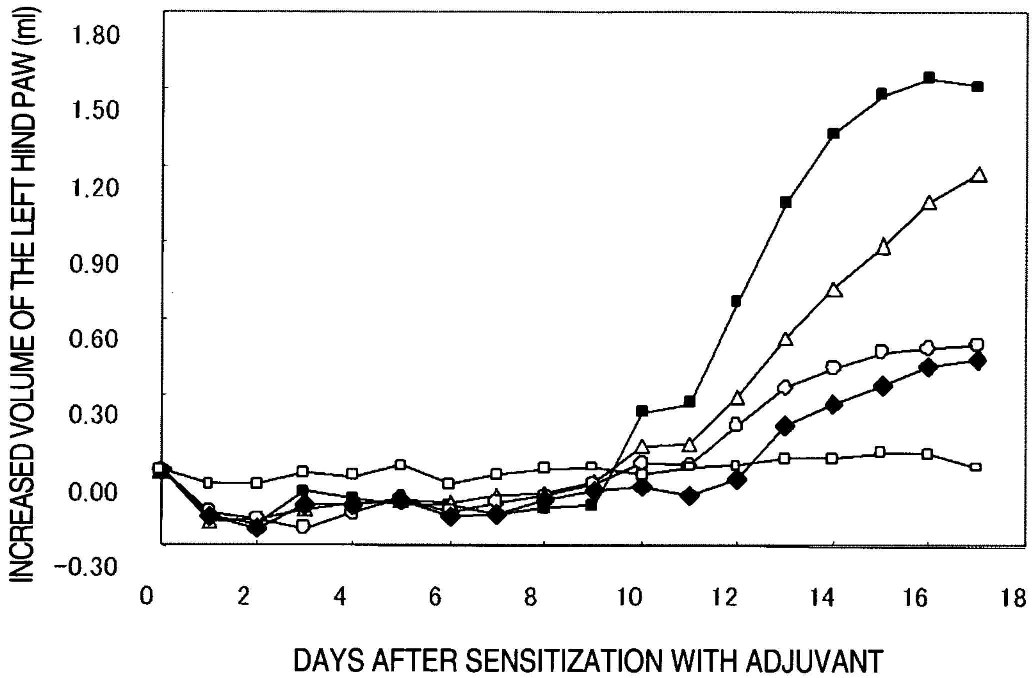
INCREASED VOLUME OF THE LEFT HIND PAW (ml)
1.80
1.50
1.20
0.90
0.60
0.30
0.00
-0.30
0
2
4
6
8
10
12
14
16
18
DAYS AFTER SENSITIZATION WITH ADJUVANT

FIG.34

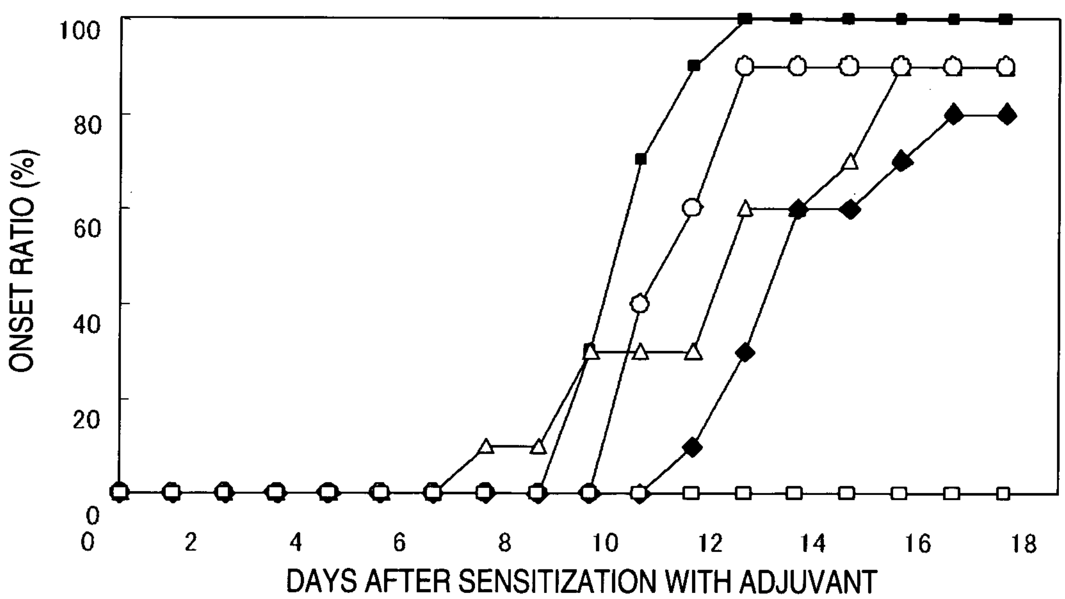
ONSET RATIO (%)
100
80
60
40
20
0
0
2
4
6
8
10
12
14
16
18
DAYS AFTER SENSITIZATION WITH ADJUVANT

FIG.35

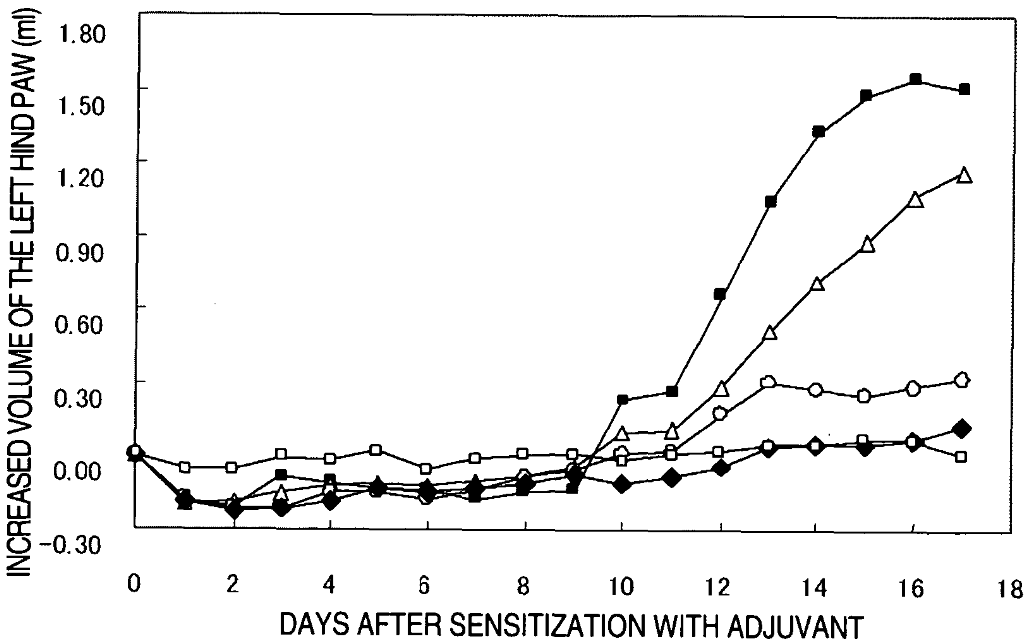
INCREASED VOLUME OF THE LEFT HIND PAW (ml)
1.80
1.50
1.20
0.90
0.60
0.30
0.00
-0.30
0
2
4
6
8
10
12
14
16
18
DAYS AFTER SENSITIZATION WITH ADJUVANT

FIG.36

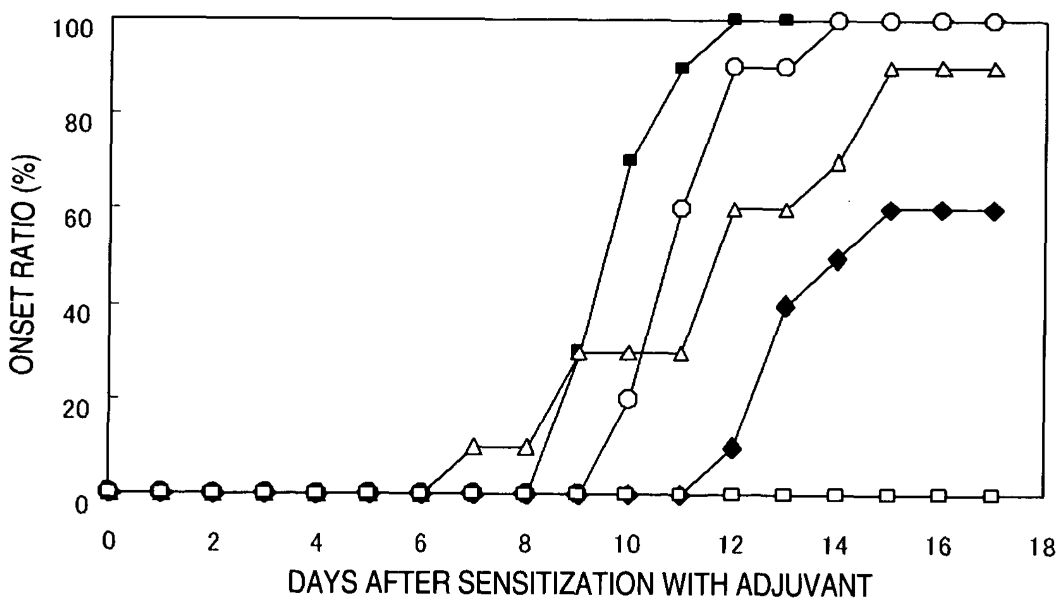
ONSET RATIO (%)
100
80
60
40
20
0
0
2
4
6
8
10
12
14
16
18
DAYS AFTER SENSITIZATION WITH ADJUVANT

FIG.37

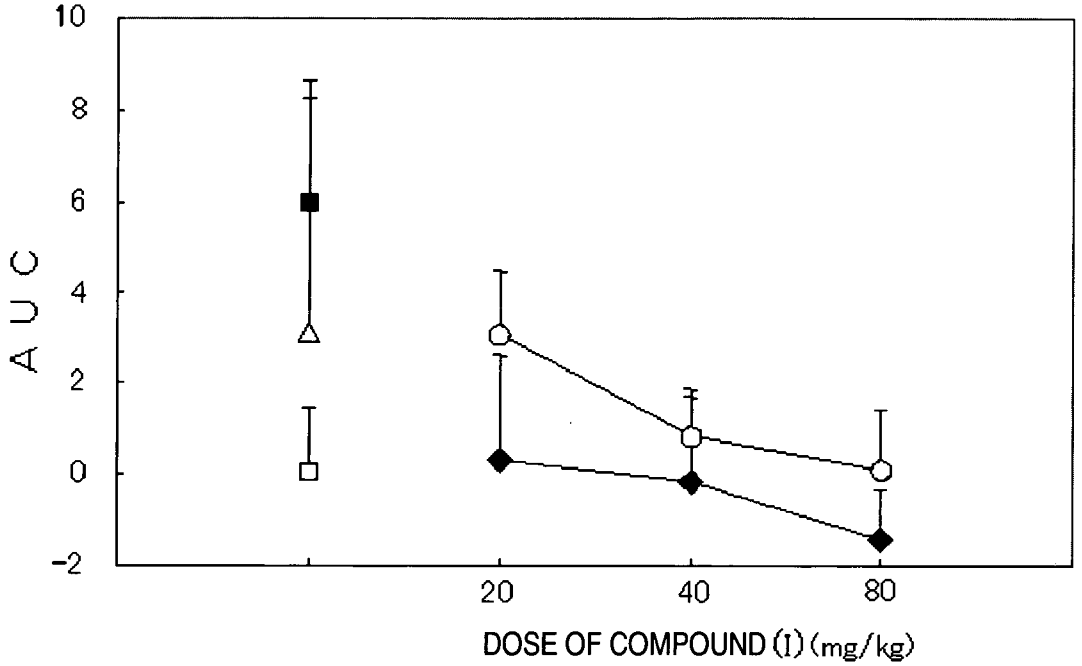

FIG.38

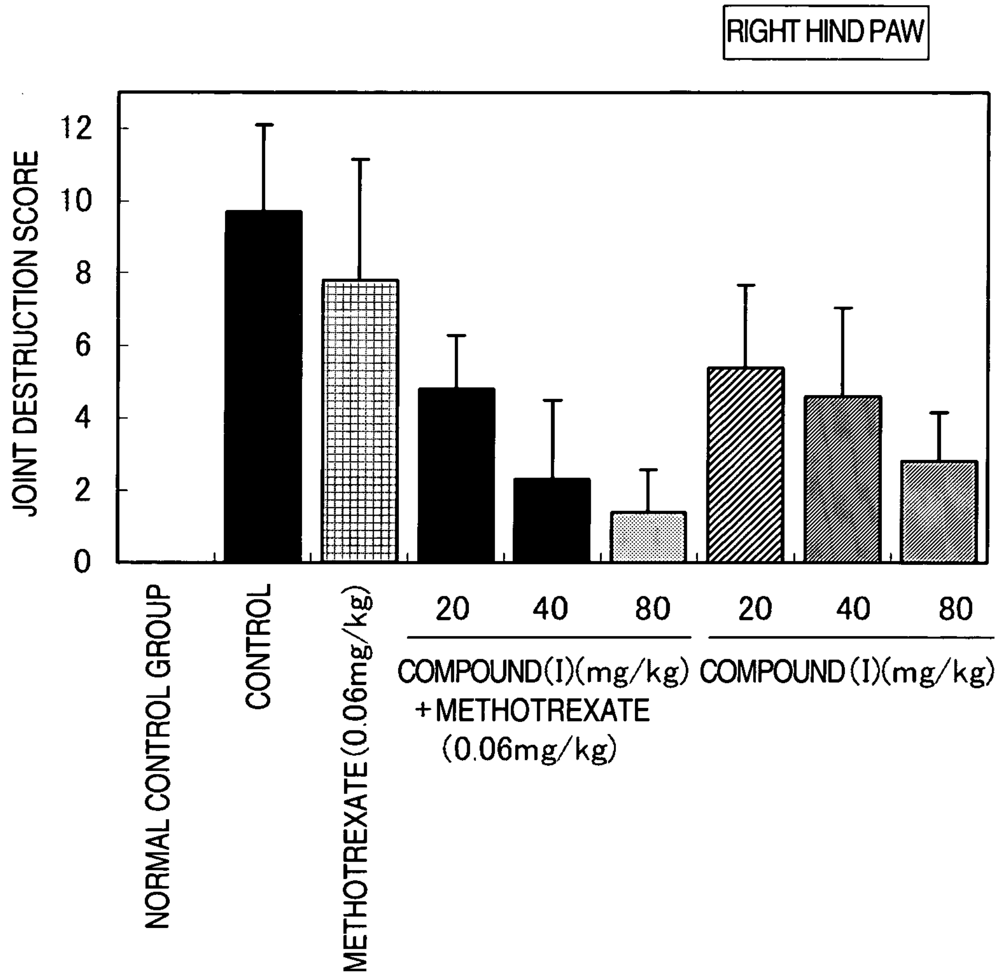
RIGHT HIND PAW
JOINT DESTRUCTION SCORE
0
2
4
6
8
10
12
NORMAL CONTROL GROUP
CONTROL
METHOTREXATE (0.06mg/kg)
20
40
80
COMPOUND(I)(mg/kg)
+METHOTREXATE
(0.06mg/kg)
20
40
80
COMPOUND (I)(mg/kg)

FIG.39

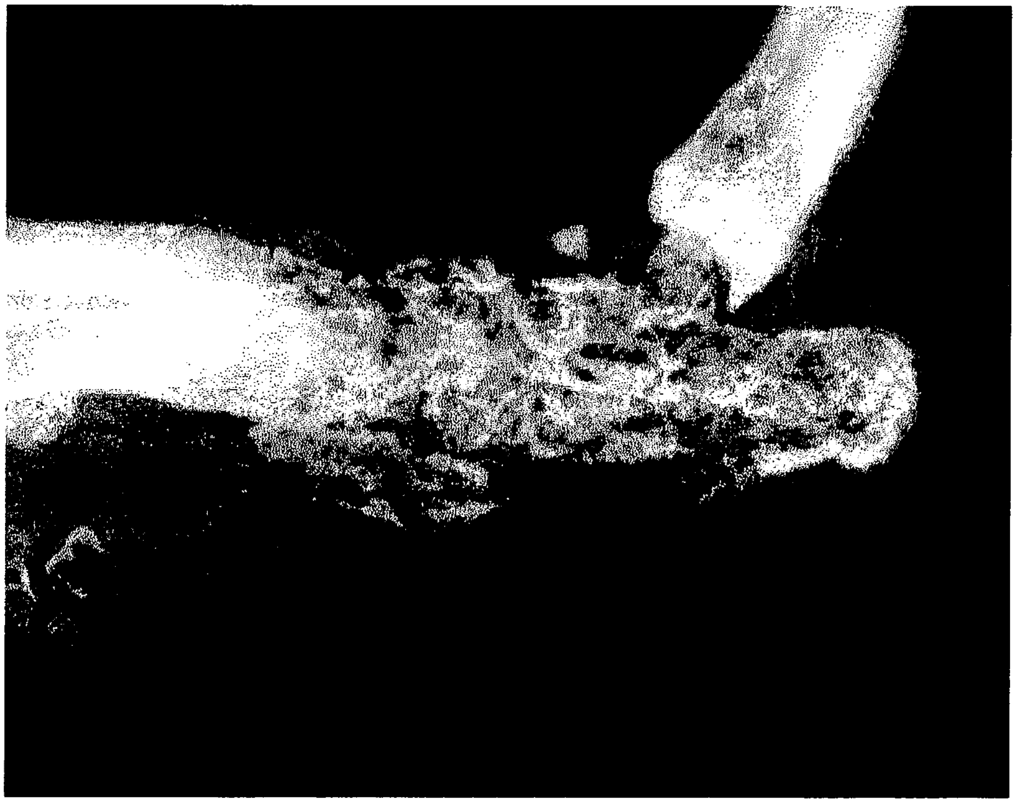

FIG.40

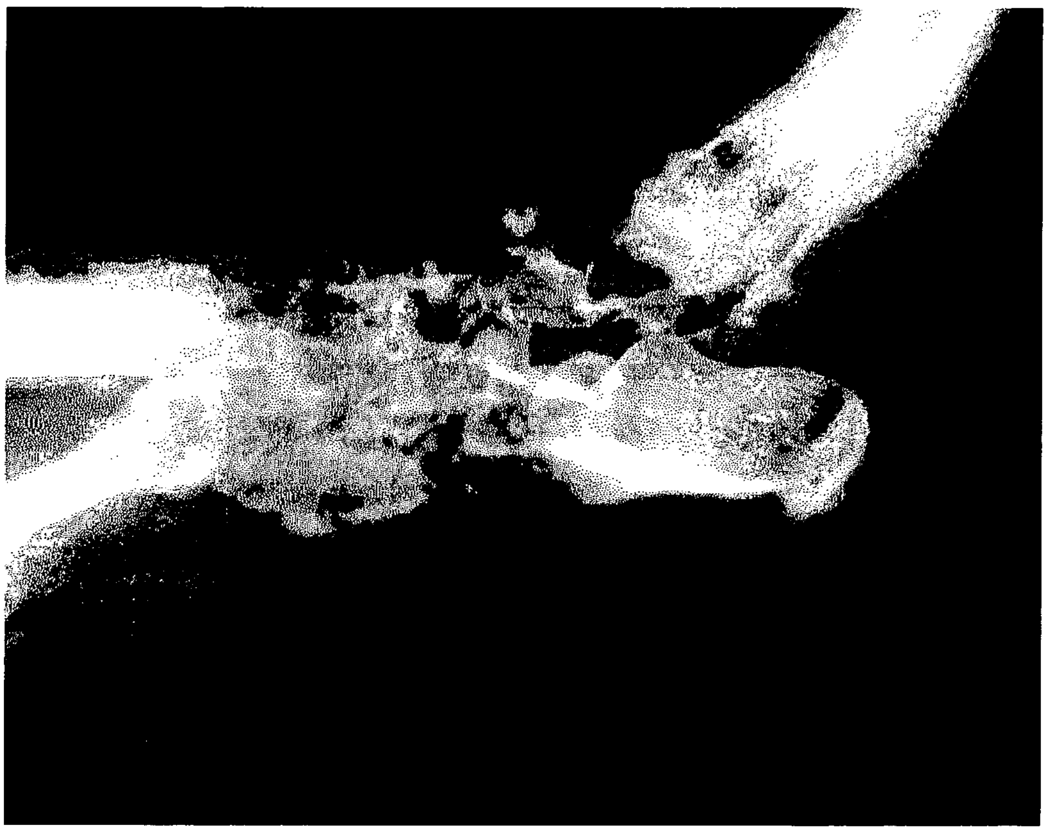

FIG.41

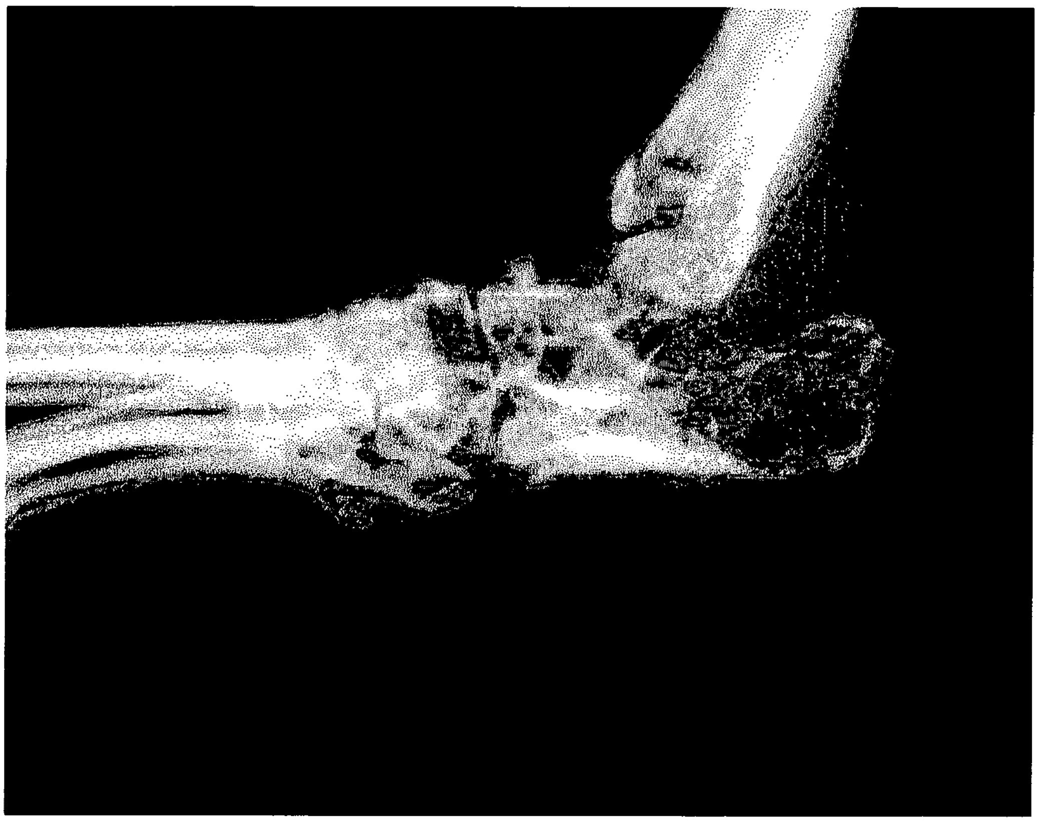

FIG.42

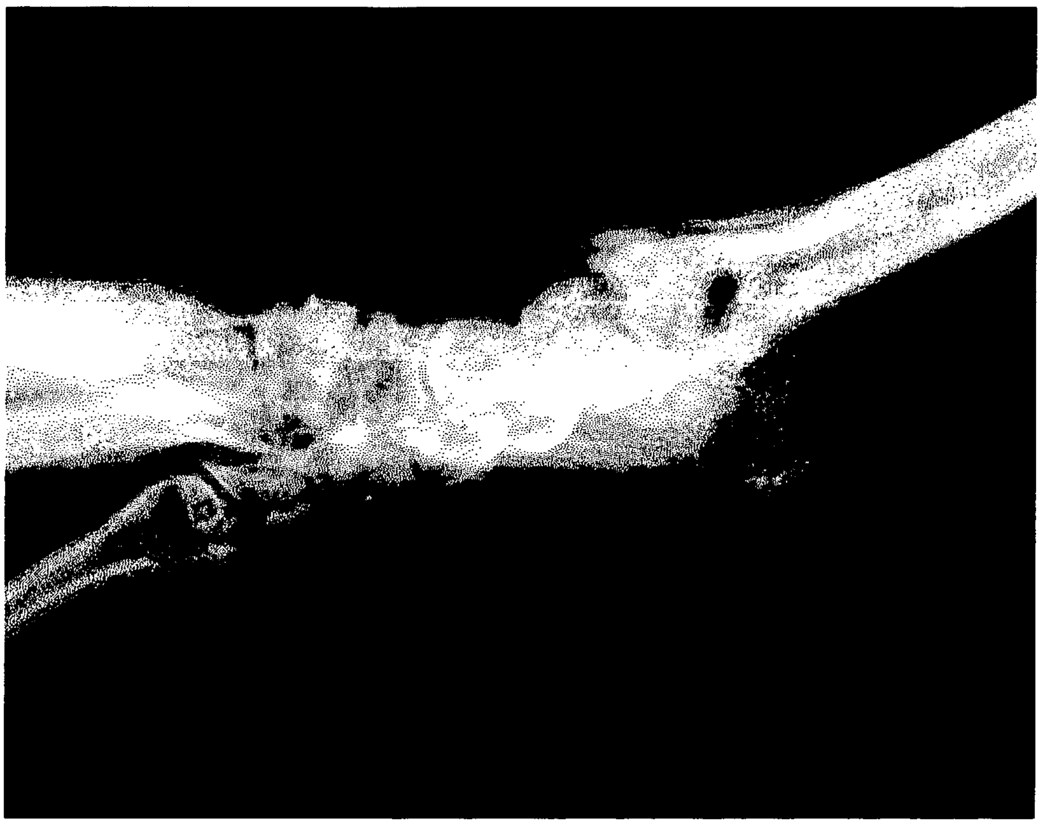

FIG.43

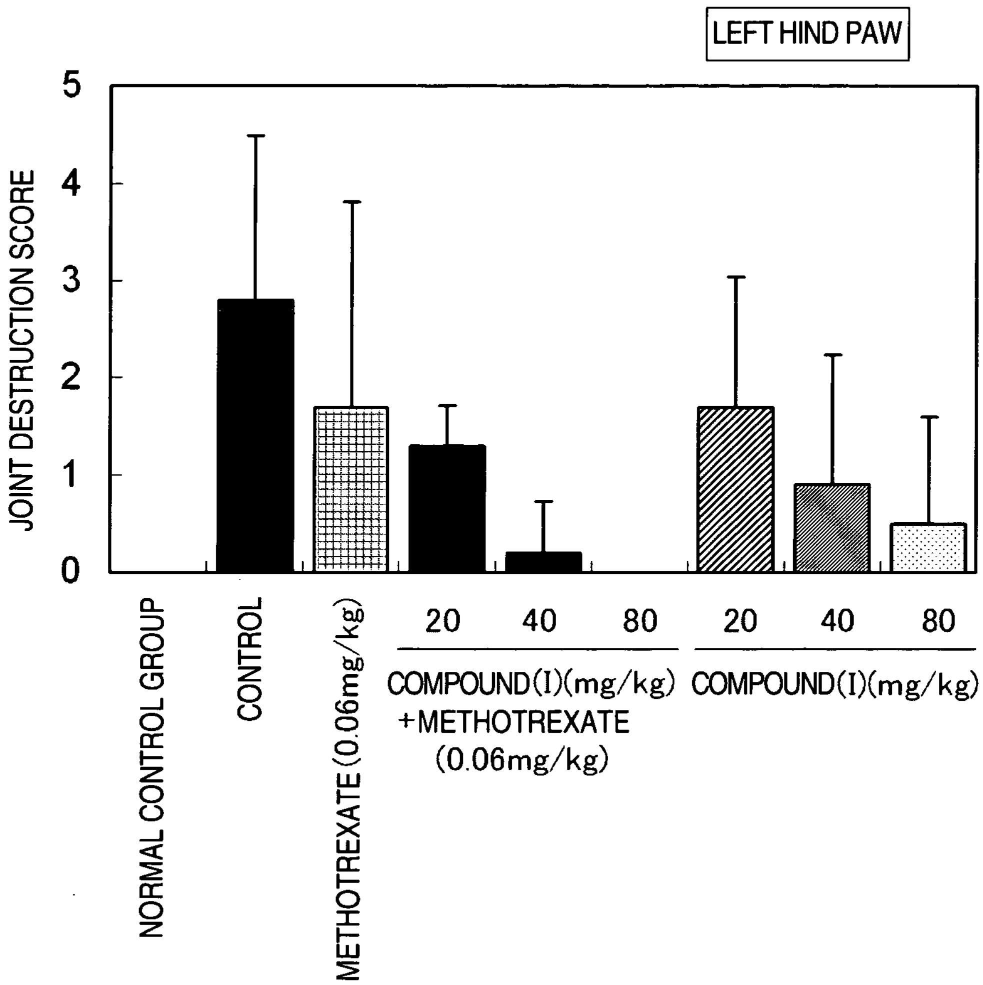
LEFT HIND PAW
JOINT DESTRUCTION SCORE
0
1
2
3
4
5
NORMAL CONTROL GROUP
CONTROL
METHOTREXATE (0.06mg/kg)
20
40
80
COMPOUND(I)(mg/kg)
+METHOTREXATE
(0.06mg/kg)
20
40
80
COMPOUND(I)(mg/kg)

FIG.44

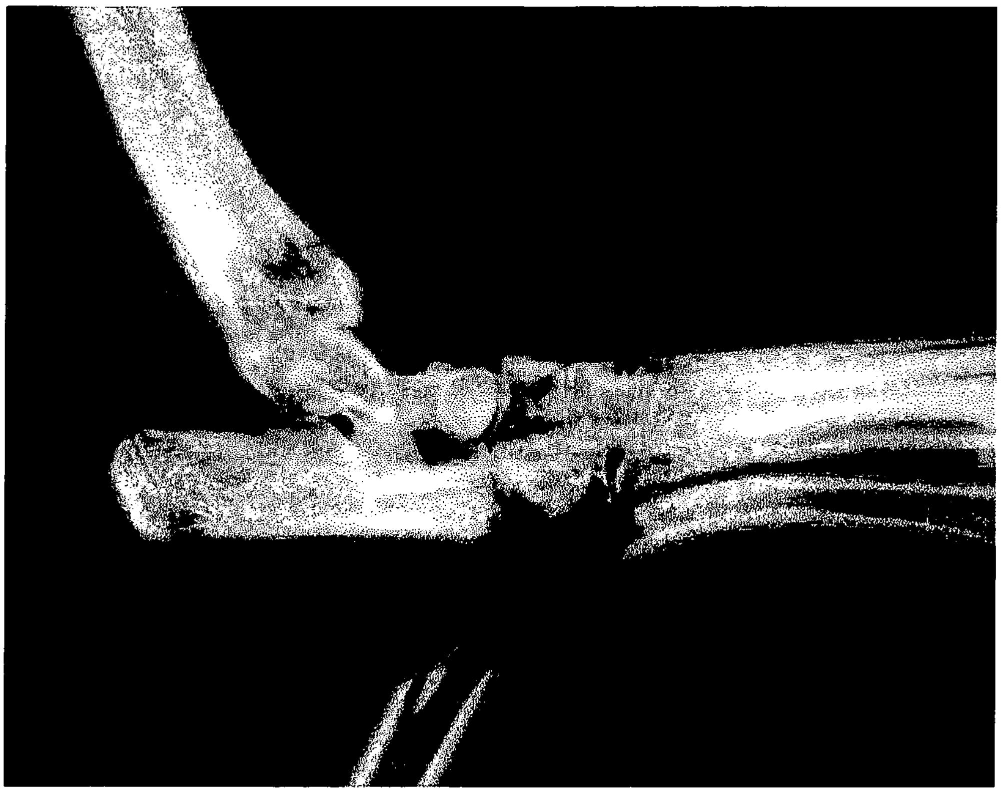

FIG.45

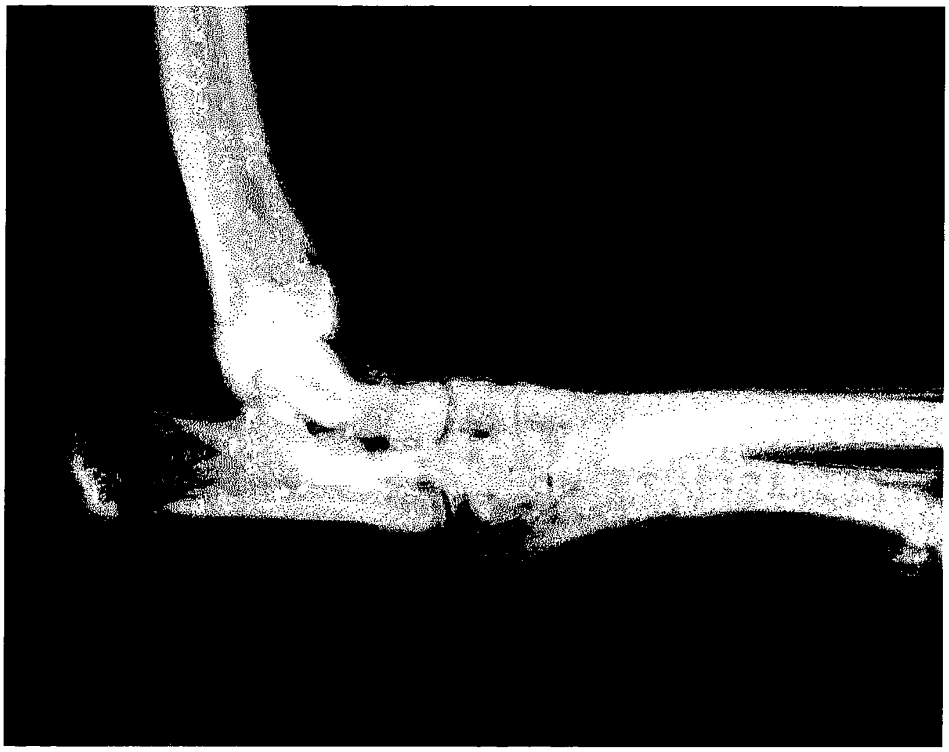

FIG.46

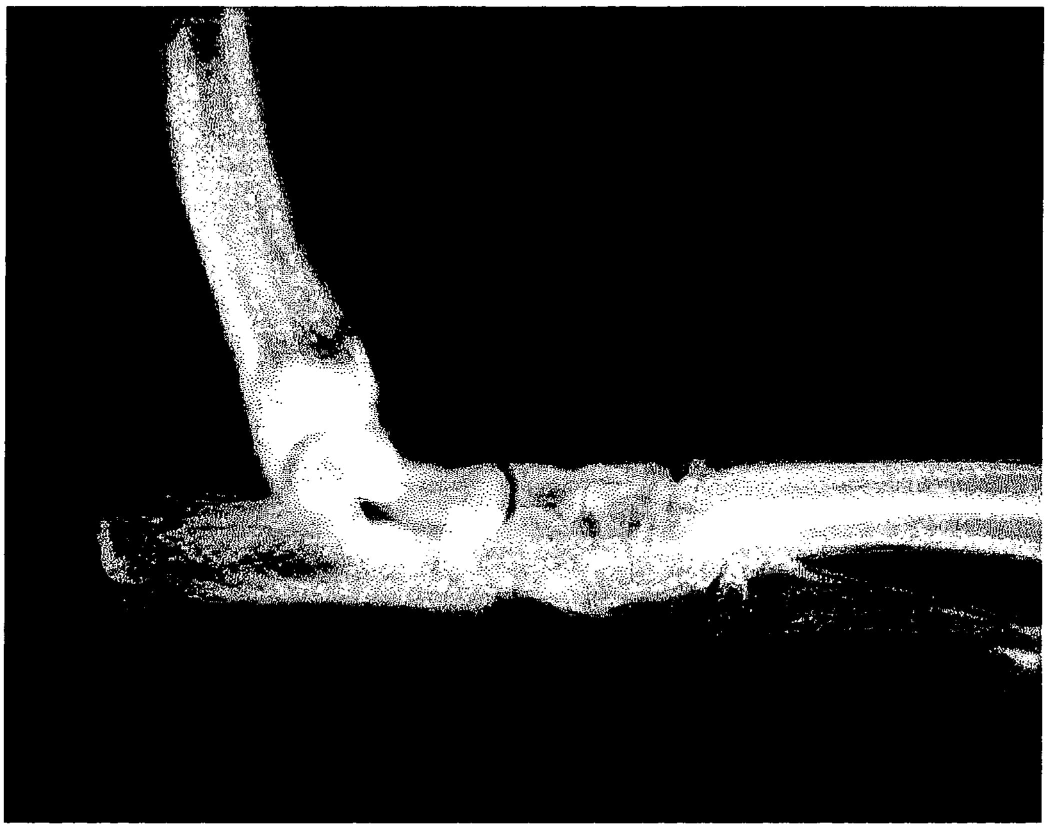

FIG.47

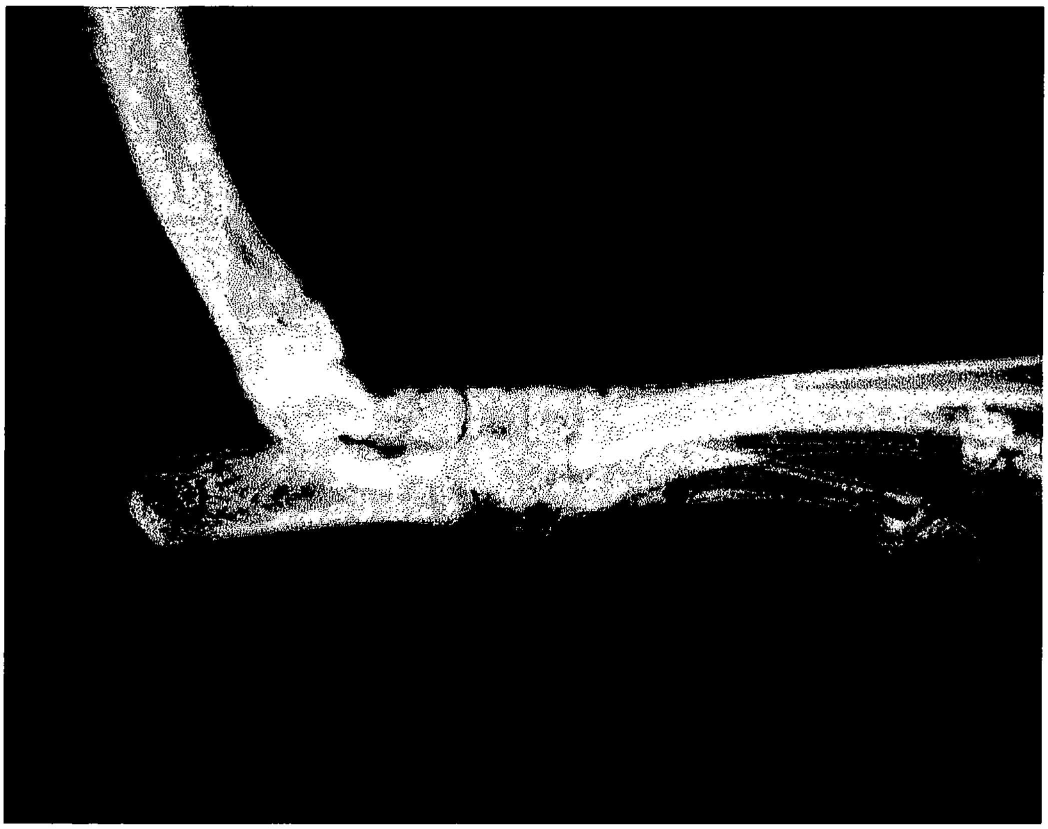

FIG.48

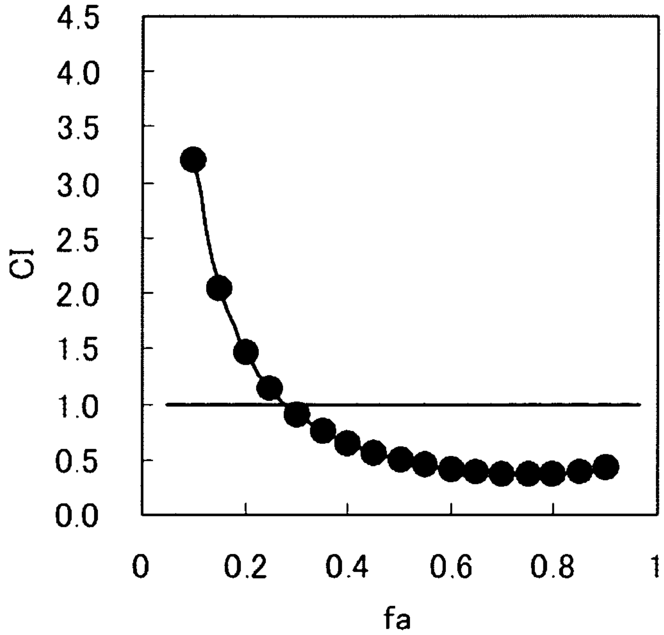

**INTERNATIONAL SEARCH REPORT**

International application No.
PCT/JP2005/020221

A. CLASSIFICATION OF SUBJECT MATTER
***A61K45/06***(2006.01), ***A61K31/18***(2006.01), ***A61K31/42***(2006.01), ***A61K31/426***(2006.01), ***A61K31/519***(2006.01), ***A61K31/5377***(2006.01), ***A61K31/635***(2006.01), ***A61P1/04***(2006.01), ***A61P1/16***(2006.01), ***A61P3/00***(2006.01),
According to International Patent Classification (IPC) or to both national classification and IPC

B. FIELDS SEARCHED

Minimum documentation searched (classification system followed by classification symbols)
***A61K45/06***(2006.01), ***A61K31/18***(2006.01), ***A61K31/42***(2006.01), ***A61K31/426***(2006.01), ***A61K31/519***(2006.01), ***A61K31/5377***(2006.01), ***A61K31/635***(2006.01), ***A61P1/04***(2006.01), ***A61P1/16***(2006.01), ***A61P3/00***(2006.01),

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
BIOSIS(STN), CAplus(STN), EMBASE(STN), MEDLINE(STN), JMEDPlus(JOIS), JSTPlus(JOIS)

C. DOCUMENTS CONSIDERED TO BE RELEVANT

| Category* | Citation of document, with indication, where appropriate, of the relevant passages | Relevant to claim No. |
|---|---|---|
| Y | WO 03/39552 A1 (ALTANA PHARMA AG.), 15 May, 2003 (15.05.03), Claims 1 to 20 & JP 2005-508983 A & EP 1448202 A | 1-41,45-49 |
| Y | WO 03/41650 A2 (IMMUNEX CORP.), 22 May, 2003 (22.05.03), Claim 9 & JP 2005-532256 A & EP 1453540 A | 1-41,45-49 |
| Y | JP 2000-72776 A (Takeda Chemical Industries, Ltd.), 07 March, 2000 (07.03.00), Par. No. [0034] & WO 99/65916 A1 & EP 1087977 A | 1-41,45-49 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 15 December, 2005 (15.12.05) | 27 December, 2005 (27.12.05) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

International application No.
PCT/JP2005/020221

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2003-146903 A (Sankyo Co., Ltd.), 21 May, 2003 (21.05.03), Claims 4 to 5 & WO 03/20768 A1 | 1-41,45-49 |
| Y | JP 11-240873 A (Sumitomo Pharmaceuticals Co., Ltd.), 07 September, 1999 (07.09.99), Claims 1 to 16 & WO 98/47880 A1 & EP 979226 A1 | 1-41,45-49 |
| Y | ROTHENBERG Ralph J., EFFECTS OF CALMODULIN INHIBITORS ON RABBIT SYNOVIOCYTE PHOSPHOLIPASE $A_2$, PROSTAGLANDINS LEUKOTRIENES AND MEDICINE, 1987, Vol.29, No.1, pages 61 to 69 | 1-41,45-49 |
| Y | JP 11-152298 A (Director General, Agency of Industrial Science and Technology), 08 June, 1999 (08.06.99), Claim 2; Par. No. [0017] (Family: none) | 1-41,45-49 |
| Y | WO 03/29428 A2 (VANDERBILT UNIVERSITY), 10 April, 2003 (10.04.03), Claims 1 to 8 & JP 2005-504829 A & EP 1439849 A2 | 1-41,45-49 |

Form PCT/ISA/210 (continuation of second sheet) (April 2005)

**INTERNATIONAL SEARCH REPORT**

International application No.
PCT/JP2005/020221

Continuation of A. CLASSIFICATION OF SUBJECT MATTER
(International Patent Classification (IPC))

***A61P3/08***(2006.01), ***A61P3/10***(2006.01), ***A61P5/14***(2006.01), ***A61P7/00***(2006.01), ***A61P7/04***(2006.01), ***A61P7/06***(2006.01), ***A61P11/00***(2006.01), ***A61P13/02***(2006.01), ***A61P15/08***(2006.01), ***A61P15/12***(2006.01), ***A61P17/00***(2006.01), ***A61P19/02***(2006.01), ***A61P19/04***(2006.01), ***A61P21/00***(2006.01), ***A61P21/04***(2006.01), ***A61P25/00***(2006.01), ***A61P27/02***(2006.01), ***A61P29/00***(2006.01), ***A61P31/04***(2006.01), ***A61P37/00***(2006.01), ***A61P43/00***(2006.01), ***C07D261/14***(2006.01), ***C07D277/20***(2006.01), ***C07D277/42***(2006.01)

According to International Patent Classification (IPC) or to both national classification and IPC)

Continuation of B. FIELDS SEARCHED
Minimum documentation searched (International Patent Classification (IPC))

***A61P3/08***(2006.01), ***A61P3/10***(2006.01), ***A61P5/14***(2006.01), ***A61P7/00***(2006.01), ***A61P7/04***(2006.01), ***A61P7/06***(2006.01), ***A61P11/00***(2006.01), ***A61P13/02***(2006.01), ***A61P15/08***(2006.01), ***A61P15/12***(2006.01), ***A61P17/00***(2006.01), ***A61P19/02***(2006.01), ***A61P19/04***(2006.01), ***A61P21/00***(2006.01), ***A61P21/04***(2006.01), ***A61P25/00***(2006.01), ***A61P27/02***(2006.01), ***A61P29/00***(2006.01), ***A61P31/04***(2006.01), ***A61P37/00***(2006.01), ***A61P43/00***(2006.01), ***C07D261/14***(2006.01), ***C07D277/20***(2006.01), ***C07D277/42***(2006.01)

Minimum documentation searched (classification system followed by classification symbols)

**INTERNATIONAL SEARCH REPORT**

International application No.
PCT/JP2005/020221

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 42-44
because they relate to subject matter not required to be searched by this Authority, namely:
Claims 42 to 44 are relevant to methods for treatment of the human body by surgery or therapy and diagnostic methods.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying an additional fee, this Authority did not invite payment of any additional fee.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the

☐ The additional search fees were accompanied by the applicant's protest and, where applicable, payment of a protest fee..

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

INTERNATIONAL SEARCH REPORT

International application No.
PCT/JP2005/020221

<Subject of search>

Claims 1 to 41 and 45 to 49 relate to a preventive and/or a remedy for an autoimmune disease which contains a compound defined by the desired property as "a CaMKII inhibitor" as the active ingredient. Although claims 1 to 5, 18 to 24, 26 to 29, 31 to 34, 36 to 41 and 45 to 49 involve any compounds having this property, only small part of the claimed compounds are disclosed in the meaning within PCT Article 5. Thus, it appears that these claims are not supported by the disclosure of the description in the meaning of PCT Article 6.

Although the common technical knowledge at the point of the application is taken into consideration, the scope of the compounds having the property as "a CaMKII inhibitor" cannot be specified. Thus, claims 1 to 5, 18 to 24, 26 to 29, 31 to 34, 36 to 41 and 45 to 49 do not comply with the requirement of clearness in the meaning within PCT Article 6 too.

Such being the case, the search was made on the relationship between "a CaMKII inhibitor" and an autoimmune disease and preventives and/or remedies containing, as the active ingredients, the compound that is particularly cited in the description and specified in claim 6 or KN-93 and a disease-modifying antirheumatic drug. On the other hand, complete search was made on claims 6 to 17, 25, 30 and 35.

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 6293730 A
- JP 2001509808 A
- JP 11152298 A
- WO 9833491 A
- JP 3237608 B
- JP 4080035 B
- JP 11240873 A
- WO 9847880 A
- JP 2000186077 A
- WO 02092094 A
- EP 1374871 A1
- US 2512572 A
- US 4305958 A
- US 4720506 A
- US 2944079 A
- US 3888843 A
- US 3056785 A
- US 3635945 A
- US 4117118 A
- US 2546658 A
- US 2233970 A
- EP 184162 A


### Non-patent literature cited in the description


- **G. S. FIRESTEIN et al.** RHEUMATOID ARTHRITIS. OXFORD UNIVERSITY PRESS, 2000
- *Biochem Biophys Res Commun,* 1991, vol. 181 (3), 968-75
- *J. Med. Chem.,* 2002, vol. 45, 563-566
- **S. TAGASHIRA.** *Inflammation and Regeneration,* 2001, vol. 21 (4), 472
- **F. NISHIKAKU.** *Annals of the Rheumatic Diseases,* 2001, vol. 60 (1), 159
- **F. NISHIKAKU.** *Annals of the Rheumatic Diseases,* 2002, vol. 61 (1), 194
- *J. Biol. Chem.,* vol. 276, 36008-36013
- *J. Biol. Chem.,* vol. 276, 39653-39660
- *Exp. Cell Res.,* vol. 240, 218-227
- *Biochem. J.,* vol. 357, 843-850
- *J. Biol. Chem.,* vol. 274, 16168-16173
- *Proc. Natl. Acad. Sci. USA,* vol. 99, 5971-5976
- **BERGER ; KIMMEL.** Guide to Molecular Clonirlg Techniques Methods in Enzymology. Academic Press, 1987, vol. 152
- **CHALK et al.** Improved and automated prediction of effective siRNA. *Biochem Biophys Res Commun.,* 2004, vol. 319 (1), 264-74
- **REYNOLDS et al.** Rational siRNA design for RNA interference. *Nature Biotechnology,* 2004, vol. 22, 326-330
- **TRENTHAM DE ; TOWNES AS ; KANG AH.** Autoimmunity to type II collagens: An experimental model of arthritis. *J Exp Med,* 1977, vol. 146, 857-868
- **PEARSON CM.** Development of arthritis, periarthritis and periotitis in rats given adjuvant. *Proc Soc Expe Biolo Med,* 1956, vol. 91, 95-101
- *Lancet,* 23 January 1999, vol. 353 (9149), 259-66
- *Nature,* 1943, vol. 151, 107
- *Br Med J.,* 16 February 1980, vol. 280 (6212), 442-4
- *J Rheumatol.,* October 1984, vol. 11 (5), 615-23
- *Naturwiss,* 1957, vol. 45, 64
- *Rheumatol Rehabil,* May 1975, vol. 14 (2), 101-14
- *Agents Actions,* February 1976, vol. 6 (1-3), 355-63
- *Ann Pharmacother,* November 1997, vol. 31 (11), 1335-8
- *Lancet,* 22 October 1994, vol. 344 (8930), 1105-10
- *Expert Opin Investig Drugs.,* January 2000, vol. 9 (1), 113-27
- *J Rheumatol,* February 2002, vol. 29 (2), 220-9
- *Ann Rheum Dis.,* November 1999, vol. 58 (1), 170-2
- *Blood,* January 1994, vol. 83 (2), 435-45
- *Blood,* September 1997, vol. 90 (6), 2188-95
- *Ann Rheum Dis.,* November 2000, vol. 59 (1), i21-7
- **FRIES JF et al.** *Arthritis Rheumatism,* 1980, vol. 23, 137-145
- **FELSON DT et al.** The American College of Rheumatology preliminary definition of improvement in rheumatoid arthritis. *Arthritis Rheum.,* 1995, vol. 38, 727-735
- *ARTHRITIS RHEUM.,* 1996, vol. 39, 34-40

- *Acta Radiologica Diagnosis,* 1977, vol. 18, 481-491
- *J Rheumatol,* 1995, vol. 22 (10), 1974-1975
- *Bailliere's Clinical Rheumatology,* 1996, vol. 10 (3), 435-453
- *Biochemical & Biophysical Research Communications,* 1990, vol. 173 (2), 578-84
- **BRADLEY et al.** *J. Clinical Investigation,* 2001, vol. 107, 995-1006